# EUROPÄISCHE PATENTSCHRIFT

(11) **EP 0 673 923 B1**
(45) Veröffentlichungstag und Bekanntmachung des Hinweises auf die Patenterteilung: **20.05.1998**
(21) Anmeldenummer: 95103942.9
(22) Anmeldetag: 17.03.1995
(51) Int. Cl.: C07C 255/64, C07C 259/02, C07C 259/10, C07C 259/18, A01N 37/50, A01N 37/52

(54) **Substituierte Phenoxymethylphenyl-Derivate, Verfahren zur ihrer Herstellung und ihre Verwendung zur Bekämpfung von Schädlingen und Pilzen**
Substituted phenoxymethyl-phenyl-derivatives, process for their preparation and their use as parasiticides and fungicides
Dérivés substitués de phénoxyméthyle-phényle, procédé pour leur préparation et leur usage comme parasiticides et fongicides

(30) Priorität: 25.03.1994 DE 4410424
(43) Veröffentlichungstag der Anmeldung: 27.09.1995
(73) Patentinhaber: BASF AKTIENGESELLSCHAFT, 67056 Ludwigshafen (DE)
(72) Erfinder: Kirstgen, Reinhard, Dr., D-67434 Neustadt (DE); Oberdorf, Klaus, Dr., D-69117 Heidelberg (DE); Sauter, Hubert, Dr., D-68167 Mannheim (DE); Bayer, Herbert, Dr., D-68159 Mannheim (DE); Grammenos, Wassilios, Dr., D-67063 Ludwigshafen (DE); Rang, Harald, Dr., D-67122 Altrip (DE); Harries, Volker, Dr., D-67227 Frankenthal (DE); Lorenz, Gisela, Dr., D-67434 Hambach (DE); Ammermann, Eberhard, Dr., D-64646 Heppenheim (DE)

(56) Entgegenhaltungen:
- EP-A- 0 386 561
- EP-A- 0 513 580
- EP-A- 0 579 124

## Beschreibung

Die vorliegende Erfindung betrifft neue substituierte Phenoxymethylphenyl-Derivate der Formel I 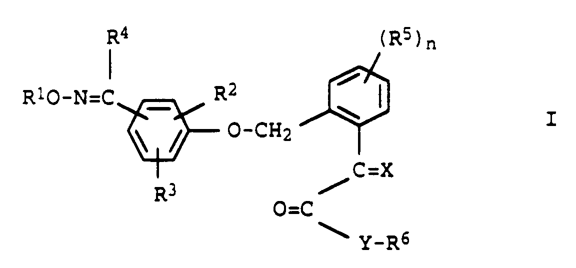 in der die Variablen die folgende Bedeutung haben:
- X: =CH-OCH₃, =CH-CH₃ oder =N-OCH₃;
- R¹: C₁-C₆-Alkyl, C₂-C₆-Alkenyl, C₃-C₆-Alkinyl, C₁-C₆-Halogenalkyl, C₃-C₆-Halogenalkenyl, C₁-C₄-Alkoxy-C₁-C₆-alkyl, Cyano-C₁-C₆-alkyl, C₁-C₆-Alkoxycarbonyl-C₁-C₆-alkyl, eine C₃-C₆-Cycloalkyl- oder C₃-C₆-Cycloalkyl-C₁-C₄-alkylgruppe, eine Aryl-C₁-C₆-alkyl- oder Aryl-C₃-C₆-alkenyl- oder Aryloxy-C₁-C₆-alkylgruppe, eine gesättigte oder ungesättigte 4- bis 6-gliedrige Heterocyclyl- oder Heterocyclyl-C₁-C₄-alkylgruppe oder eine Heteroaryl-C₁-C₆-alkylgruppe, wobei die heterocyclischen Ringe neben C-Atomen jeweils ein oder zwei Ringglieder enthalten, die ausgewählt sind aus der Gruppe bestehend aus einem Sauerstoff- oder Schwefelatom und ein oder zwei Stickstoffatomen und ein oder zwei Gruppen -N(CH₃)-,
wobei die carbocyclischen und heterocyclischen Ringe jeweils ihrerseits einen oder mehrere Reste tragen können, ausgewählt aus der Gruppe bestehend aus: Halogen, C₁-C₄-Alkyl, C₁-C₂-Halogenalkyl, C₃-C₆-Cycloalkyl, C₁-C₄-Alkoxy, C₁-C₂-Halogenalkoxy, Halogen, Aryl und Aryloxy;
- R², R³: Wasserstoff, Halogen, Cyano, Nitro, C₁-C₄-Alkyl, C₁-C₂-Halogenalkyl, C₁-C₄-Alkoxy, C₁-C₂-Halogenalkoxy, oder, sofern R² und R³ benachbart sind, zusammen einen Oxymethylidenoxy- oder Oxyethylidenoxy-Brücke, wobei jedes C-Atom dieser Brücken gewünschtenfalls ein oder zwei Halogenatome und/oder Methylreste tragen kann;
- R⁴: Cyano, Chlor, Brom, C₁-C₆-Alkoxy, C₁-C₆-Alkylthio, C₁-C₄-Halogenalkoxy, C₁-C₄-Alkoxy-C₁-C₄-alkoxy, C₃-C₆-Cycloalkyl-C₁-C₂-alkoxy, C₁-C₆-Alkylamino, Di- (C₁-C₆-alkyl) amino, C₃-C₆-Alkenyloxy, C₃-C₆-Alkinyloxy, Aryloxy, Arylthio wobei die aromatischen Ringe ein bis drei Reste tragen können, ausgewählt aus der Gruppe bestehend aus C₁-C₄-Alkyl, C₁-C₄-Halogenalkyl, C₁-C₄-Alkoxy, C₁-C₄-Halogenalkoxy und C₁-C₄-Alkylthio, und wobei die aromatischen Ringe zusätzlich so viele Halogenatome tragen können, daß die Gesamtzahl der Reste 4 oder 5 beträgt;
- R⁵: Nitro, Cyano, Halogen, C₁-C₄-Alkyl, C₁-C₄-Halogenalkyl, C₁-C₄-Alkoxy, C₁-C₄-Halogenalkoxy, C₁-C₄-Alkylthio, Phenyl oder Phenoxy, wobei die aromatischen Ringe ein bis drei Reste tragen können, ausgewählt aus der Gruppe bestehend aus C₁-C₄-Alkyl, C₁-C₄-Halogenalkyl, C₁-C₄-Alkoxy, C₁-C₄-Halogenalkoxy und C₁-C₄-Alkylthio, und wobei die aromatischen Ringe zusätzlich so viele Halogenatome tragen können, daß die Gesamtzahl der Reste 4 oder 5 beträgt;
oder, sofern n für 2, 3, oder 4 steht, eine an zwei benachbarte C-Atome des Grundkörpers annellierte 1,3-Butadien-1,4-diylgruppe oder eine mono- oder dihalogenierte 1,3-Butadien-1,4-diylgruppe, wobei diese annellierten Ringe ihrerseits ein oder zwei Reste tragen können, ausgewählt aus der Gruppe Nitro, Cyano, Halogen, C₁-C₄-Halogenalkyl, C₁-C₄-Alkoxy, C₁-C₄-Halogenalkoxy und C₁-C₄-Alkylthio;
- n: 0, 1, 2, 3 oder 4, wobei die Reste R⁵ gleich oder verschieden sein können, wenn n für 2, 3 oder 4 steht;
- Y: Sauerstoff, -NH- oder -N(CH₃)-;
- R⁶: Wasserstoff oder C₁-C₄-Alkyl.

Außerdem betrifft die Erfindung Verfahren und Zwischenprodukte zur Herstellung der Verbindungen I, deren Verwendung als Fungizide und zur Bekämpfung von Schädlingen, fungizide Mittel und Mittel zur Bekämpfung von Schädlingen, welche diese Verbindungen als wirksame Substanzen enthalten, sowie Verfahren zur Bekämpfung von Schadpilzen und Schädlingen.

Verbindungen vom Typ der Phenoxymethylphenyl-Derivate I sind bereits aus den folgenden Druckschriften bekannt:

Die EP-A 386 561 beschreibt [(Iminoalkyl)-phenoxymethyl]-phenylessigesterderivate der Formel I' mit fungiziden und pestiziden Eigenschaften, 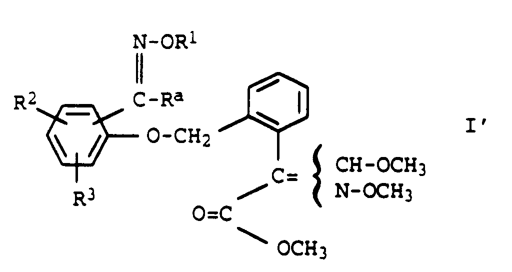 wobei R^{a} für Wasserstoff, Alkyl oder Aryl steht.

Des weiteren ist der EP-A 579 124 zu entnehmen, daß 2-[(Iminoalkyl)-phenoxymethyl]-phenylessigsäureamide der Formel I'' 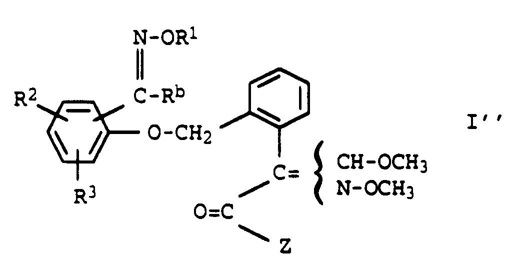 wobei R^{b} Wasserstoff, Alkyl, Cycloalkyl, Halogenalkyl oder Aryl und und Z Amino, Alkylamino oder Dialkylamino bedeuten, zur Bekämpfung von Pilzen und Schädlingen geeignet sind. Zur Bekämpfung von Schädlingen werden zusätzlich Verbindungen als geeignet erachtet, in denen Z für Alkoxy steht.

Die EP-A 513 580 lehrt, daß α-Phenylacrylsäurederivate, die eine gewisse Grundstruktur gemeinsam haben, fungizid und pestizid wirksam sind. Bei geeigneter Wahl der einzelnen Substituenten lassen sich Verbindungen der Formel I''' 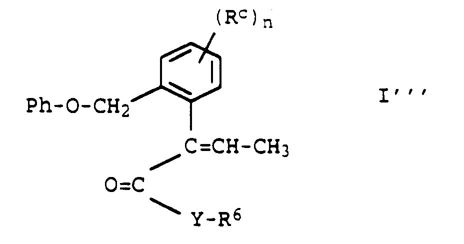 konstruieren, wobei
- R^{c}: Wasserstoff, Nitro, Cyano, Halogen, C₁-C₄-Alkyl, C₁-C₄-Alkoxy, C₁-C₄-Halogenalkyl, C₁-C₄-Halogenalkoxy oder C₁-C₄-Alkylthio bedeutet,
oder, für den Fall, daß n 2, 3 oder 4 bedeutet, zwei benachbarte Substituenten R^{c} zusammen eine gegenbenenfalls substituierte 1,3-Butadien-1,4-diylgruppe bilden,
und wobei Ph für einen Phenylring steht, der neben verschiedenen anderen Substituenten u.a. eine Gruppe -C(R^{d})=NOR^{e} tragen kann, wobei R^{d} für Wasserstoff, C₁-C₆-Alkyl oder gegebenenfalls substituiertes Aryl und R^{e} für gegebenenfalls substituiertes C₁-C₆-Alkyl, C₂-C₆-Alkenyl oder C₂-C₆-Alkinyl, oder für ein 5- oder 6-gliedriges aromatisches oder heteroaromatisches System steht.

Verbindungen von diesem Typ, jedoch mit höchstens einem Rest R^{c}, die am Phenylrest Ph neben der Gruppe -C(R^{d})=NOR^{e} noch ein oder zwei Cyano-, Halogen-, C₁-C₄-Alkyl-, C₁-C₄-Halogenalkyl- und/oder C₁-C₄-Alkoxysubstituenten tragen können werden in der EP-A 513 580 als bevorzugte Fungizide unter der Formel Ib hervorgehoben.

Schließlich offenbart die EP-A 398 692 u.a. substituierte 2-[2-(Phenoxymethyl)phenyl]-2-alkoxyimino-acetamide mit fungiziden Eigenschaften.

Die fungiziden bzw. insektiziden Wirkungen der bekannten Verbindungen können jedoch, besonders bei kleinen Aufwandmengen und -konzentrationen, nur bedingt befriedigen.

Aufgabe der vorliegenden Erfindung waren neue Verbindungen mit verbesserten fungiziden und/oder pestiziden Eigenschaften.

Demgemäß wurden die eingangs definierten substituierten Phenoxymethylphenyl-Derivate I gefunden. Weiterhin wurden Verfahren und Zwischenprodukte zur Herstellung dieser Verbindungen, sie enthaltende Mittel und Verfahren zu deren Verwendung zur Bekämpfung von Schadpilzen und Schädlingen gefunden.

Die vorstehend für die Substituenten R¹ bis R⁶ aufgeführten Bedeutungen stellen Sammelbegriffe für individuelle Aufzählungen der einzelnen Gruppenmitglieder dar. Sämtliche Kohlenstoffketten, also alle Alkyl-, Alkenyl-, Alkinyl-, Halogenalkyl-, Halogenalkenyl-, Alkoxy-, Halogenalkoxy-, Alkenyloxy-, Alkinyloxy-, Alkoxycarbonyl-, Alkylthio- und Alkylamino-Teile können geradkettig oder verzweigt sein. Halogenierte Substituenten tragen vorzugsweise ein bis fünf gleiche oder verschiedene Halogenatome.

Im einzelnen bedeuten beispielsweise:
- Halogen: Fluor, Chlor, Brom, Jod, vorzugsweise Fluor oder Chlor;
- C₁-C₄-Alkyl: Methyl, Ethyl, n-Propyl, 1-Methylethyl, n-Butyl, 1-Methylpropyl, 2-Methylpropyl, 1,1-Dimethylethyl, vorzugsweise Methyl;
- C₁-C₆-Alkyl: C₁-C₄-Alkyl wie vorstehend genannt, sowie n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, vorzugsweise Methyl, Ethyl, n-Propyl, 1-Methylethyl, n-Butyl oder 1,1-Dimethylethyl;
- C₂-C₆-Alkenyl: Ethenyl, Prop-1-en-1-yl, Prop-2-en-1-yl, 1-Methylethenyl, n-Buten-1-yl, n-Buten-2-yl, n-Buten-3-yl, 1-Methylprop-1-en-1-yl, 2-Methylprop-1-en-1-yl, 1-Methylprop-2-en-1-yl, 2-Methylprop-2-en-1-yl, n-Penten-1-yl, n-Penten-2-yl, n-Penten-3-yl, n-Penten-4-yl, 1-Methylbut-1-en-1-yl, 2-Methylbut-1-en-1-yl, 3-Methylbut-1-en-1-yl, 1-Methylbut-2-en-1-yl, 2-Methylbut-2-en-1-yl, 3-Methylbut-2-en-1-yl, 1-Methylbut-3-en-1-yl, 2-Methylbut-3-en-1-yl, 3-Methylbut-3-en-1-yl, 1,1-Dimethyl-prop-2-en-1-yl, 1,2-Dimethyl-prop-1-en-1-yl, 1,2-Dimethyl-prop-2-en-1-yl, 1-Ethylprop-1-en-2-yl, 1-Ethylprop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methylpent-1-en-1-yl, 2-Methylpent-1-en-1-yl, 3-Methylpent-1-en-1-yl, 4-Methylpent-1-en-1-yl, 1-Methylpent-2-en-1-yl, 2-Methylpent-2-en-1-yl, 3-Methylpent-2-en-1-yl, 4-Methylpent-2-en-1-yl, 1-Methylpent-3-en-1-yl, 2-Methylpent-3-en-1-yl, 3-Methylpent-3-en-1-yl, 4-Methylpent-3-en-1-yl, 1-Methylpent-4-en-1-yl, 2-Methylpent-4-en-1-yl, 3-Methylpent-4-en-1-yl, 4-Methylpent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethylbut-1-en-1-yl, 1-Ethylbut-2-en-1-yl, 1-Ethylbut-3-en-1-yl, 2-Ethylbut-1-en-1-yl, 2-Ethylbut-2-en-1-yl, 2-Ethylbut-3-en-1-yl, 1,1,2-Trimethylprop-2-en-1-yl, 1-Ethyl-1-methyl-prop-2-en-1-yl, 1-Ethyl-2-methyl-prop-1-en-1-yl, 1-Ethyl-2-methyl-prop-2-en-1-yl, vorzugsweise Ethenyl oder Prop-2-en-1-yl;
- C₃-C₆-Alkinyl: Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-i n-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl, 4-Methyl-pent-2-in-5-yl, vorzugsweise Prop-2-in-1-yl;
- C₁-C₂-Halogenalkyl: z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, vorzugsweise Difluormethyl oder Trifluormethyl;
- C₁-C₆-Halogenalkyl: C₁-C₆-Alkyl wie vorstehend genannt, das partiell oder vollständig durch Fluor, Chlor und/oder Brom substituiert ist, also z.B. die vorstehend genannten C₁-C₂-Halogenalkylreste, sowie 3-Chlorpropyl oder Heptafluorpropyl, vorzugsweise Trifluormethyl, Pentafluorethyl oder Heptafluorpropyl;
- C₃-C₆-Halogenalkenyl: C₃-C₆-Alkenyl wie vorstehend genannt, das partiell oder vollständig durch Fluor, Chlor und/oder Brom substituiert ist, also z.B. 2-Chlorallyl, 3-Chlorallyl oder 3,3-Dichlorallyl;
- C₁-C₄-Alkoxy: Methoxy, Ethoxy, n-Propoxy, 1-Methylethoxy, n-Butoxy, 1-Methylpropoxy, 2-Methylpropoxy, 1,1-Dimethylethoxy, vorzugsweise Methoxy oder Ethoxy;
- C₁-C₆-Alkoxy: C₁-C₄-Alkoxy wie vorstehend genannt, sowie n-Pentoxy, 1-Methylbutoxy, 2-Methylbutoxy, 3-Methylbutoxy, 2,2-Dimethylpropoxy, 1-Ethylpropoxy, n-Hexoxy, 1,1-Dimethylpropoxy, 1,2-Dimethylpropoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, vorzugsweise C₁-C₄-Alkoxy wie vorstehend genannt;
- C₁-C₄-Halogenalkoxy: C₁-C₄-Alkoxy wie vorstehend genannt, das partiell oder vollständig durch Fluor, Chlor und/oder Brom substituiert ist, also z.B. Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 3-Chlorpropoxy, vorzugsweise Difluormethoxy, Trifluormethoxy, 2,2,2-Trifluorethoxy oder Pentafluorethoxy:
- C₁-C₆-Alkoxycarbonyl im Substituenten C₁-C₆-Alkoxycarbonyl-C₁-C₆-alkyl: Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl, und 1,1-Dimethylethoxycarbonyl, n-Pentoxycarbonyl, 1-Methylbutoxycarbonyl, 2-Methylbutoxycarbonyl, 3-Methylbutoxycarbonyl, 2,2-Di-methylpropoxycarbonyl, 1-Ethylpropoxycarbonyl, n-Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methylpropoxycarbonyl, 1-Ethyl-2-methylpropoxycarbonyl, vorzugsweise C₁-C₄-Alkoxycarbonyl; Beispiele für C₁-C₆-Alkoxycarbonyl-C₁-C₆-alkyl sind Ethoxycarbonylmethyl, tert.-Butoxycarbonylmethyl und tert.-Butoxycarbonylpropyl;
- C₃-C₆-Cycloalkyl: Cyclopropyl, Cyclobutyl, Cyclopentyl, Cyclohexyl, vorzugsweise Cyclopropyl, Cylopentyl oder Cyclohexyl;
- C₃-C₆-Cycloalkyl-C₁-C₄-alkyl: z.B. Cyclopropylmethyl, Cyclobutylmethyl, Cyclopentylmethyl, Cyclohexylmethyl, 2-(Cyclopropyl)ethyl, 2-(Cyclobutyl)ethyl, 2-(Cyclopentyl)ethyl, 2-(Cyclohexyl)ethyl, 3-(Cyclopropyl)propyl, 3-(Cyclobutyl)propyl, 3-(Cyclopentyl)propyl, 3-(Cyclohexyl)propyl, 4-(Cyclopropyl)butyl, 4-(Cyclobutyl)butyl, 4-(Cyclopentyl)butyl, 4-(Cyclohexyl)butyl, vorzugsweise Cyclopropylmethyl oder Cyclopentylmethyl;
- Cyano-C₁-C₆-alkyl: z . B. Cyanomethyl, 1-Cyanoeth-1-yl, 2-Cyanoeth-1-yl, 1-Cyano-prop-1-yl, 2-Cyano-prop-1-yl, 3-Cyano-prop-1-yl, 1-Cyano-prop-2-yl, 2-Cyano-prop-2-yl, 1-Cyano-but-1-yl, 2-Cyano-but-1-yl, 3-Cyano-but-1-yl, 4-Cyano-but-1-yl, 1-Cyano-but-2-yl, 2-Cyano-but-2-yl, 1-Cyano-but-3-yl, 2-Cyano-but-3-yl, 1-Cyano-2-methyl-prop-3-yl, 2-Cyano-2-methyl-prop-3-yl, 3-Cyano-2-methyl-prop-3-yl, 2-Cyanomethyl-prop-2-yl, vorzugsweise Cyanomethyl;
- Aryl-C₁-C₆-alkyl: Arylmethyl, 1-Arylethyl, 2-Arylethyl, 1-Arylprop-1-yl, 2-Arylprop-1-yl, 3-Arylprop-1-yl, 1-Arylbut-1-yl, 2-Arylbut-1-yl, 3-Arylbut-1-yl, 4-Arylbut-1-yl, 1-Arylbut-2-yl, 2-Arylbut-2-yl, 3-Arylbut-2-yl, 3-Arylbut-2-yl, 4-Arylbut-2-yl, 1-(Arylmethyl)-eth-1-yl, 1-(Arylmethyl)-1-(methyl)-eth-1-yl, 1-(Arylmethyl)-prop-1-yl, vorzugsweise Arylmethyl oder 1-Arylethyl;
- Heteroaryl-C₁-C₆-alkyl: Heteroarylmethyl, 1-(Heteroaryl) ethyl, 2-(Heteroaryl)ethyl, 1-(Heteroaryl)prop-1-yl, 2-(Heteroaryl)prop-1-yl, 3-(Heteroaryl)prop-1-yl, 1-(Heteroaryl)but-1-yl, 2-(Heteroaryl)but-1-yl, 3-(Heteroaryl)but-1-yl, 4-(Heteroaryl)but-1-yl, 1-(Heteroaryl)but-2-yl, 2-(Heteroaryl)but-2-yl, 3-(Heteroaryl)but-2-yl, 3-(Heteroaryl)but-2-yl, 4-(Heteroaryl)but-2-yl, 1-(Heteroarylmethyl)-eth-1-yl, 1-(Heteroarylmethyl)-1-(methyl)-eth-1-yl, 1-(Heteroarylmethyl)-prop-1-yl, vorzugsweise Heteroarylmethyl;
- C₁-C₄-Alkylthio: Methylthio, Ethylthio, n-Propylthio, 1-Methylethylthio, n-Butylthio, 1-Methyl-propylthio, 2-Methylpropylthio, 1,1-Dimethylethylthio, vorzugsweise Methylthio oder Ethylthio;
- C₁-C₆-Alkylthio: C₁-C₄-Alkylthio wie vorstehend genannt, sowie n-Pentylthio, 1-Methylbutylthio, 2-Methylbutylthio, 3-Methylbutylthio, 2,2-Dimethylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio, 1-Ethyl-2-methylpropylthio, vorzugsweise C₁-C₄-Alkylthio wie vorstehend genannt;
- C₁-C₄-Alkoxy-C₁-C₆-alkyl: Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, 2-Methoxyethyl, 2-Ethoxyethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)-ethyl, 2-(Methoxy)propyl, 3-(Methoxy)propyl, 2-(Ethoxy)propyl, 3-(Ethoxy)propyl, 3-Propoxypropyl, 3-Butoxypropyl, 4-(Methoxy)butyl, 4-(Ethoxy)butyl, 4-(n-Propoxy)butyl, 4-(n-Butoxy)butyl, 5-(Methoxy)pentyl, 5-(Ethoxy)pentyl, 5-(n-Propoxy)pentyl, 5-(n-Butoxy)pentyl, 6-(Methoxy)hexyl, 6-(Ethoxy)hexyl, 6-(n-Propoxy)hexyl, 6-(n-Butoxy)hexyl, vorzugsweise Methoxymethyl, Ethoxymethyl, (1,1-Dimethylethoxy)methyl oder 2-Methoxyethyl;
- C₁-C₆-Alkylamino: Methylamino, Ethylamino, n-Propylamino, 1-Methylethylamino, n-Butylamino, 1-Methylpropylamino, 2-Methylpropylamino, 1,1-Dimethylethylamino, n-Pentylamino, 1-Methylbutylamino, 2-Methylbutylamino, 3-Methylbutylamino, 2,2-Dimethylpropylamino, 1-Ethylpropylamino, n-Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2Trimethylpropylamino, 1-Ethyl-1-methylpropylamino, 1-Ethyl-2-methylpropylamino, vorzugsweise C₁-C₄-Alkylamino wie Methylamino, Ethylamino, n-Propylamino und n-Butylamino;
- Di-(C₁-C₆)-alkylamino: z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)amino, N-Butyl-N-ethylamino, N-Ethyl-N-(1-methyl-propyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-ButylN-(1-methylpropyl)amino, N-Butyl-N-(2-methylpropyl)amino, N-Butyl-N-(1,1-dimethylethyl)amino, N-(1-Methylpropyl)-N-(2-methyl-propyl)amino, N-(1,1-Dimethylethyl)-N-(1-methylpropyl)amino, N-(1,1-Dimethylethyl)-N-(2-methylpropyl)amino, vorzugsweise Di- (C₁-C₄) -alkylamino wie N,N-Di-methylamino und N,N-Diethylamino;
- C₃-C₆-Alkenyloxy: Prop-2-en-1-yloxy, n-Buten-2-yloxy, n-Buten-3-yloxy, 1-Methylprop-2-en-1-yloxy, 2-Methylprop-2-en-1-yloxy, n-Penten-2-yloxy, n-Penten-3-yloxy, n-Penten-4-yloxy, 1-Methylbut-2-en-1-yloxy, 2-Methylbut-2-en-1-yloxy, 3-Methylbut-2-en-1-yloxy, 1-Methylbut-3-en-1-yloxy, 2-Methylbut-3-en-1-yloxy, 3-Methylbut-3-en-1-yloxy, 1,1-Dimethyl-prop-2-en-1-yloxy, 1,2-Dimethyl-prop-2-en-1-yloxy, 1-Ethyl-prop-2-en-1-yloxy, n-Hex-2-en-1-yloxy, n-Hex-3-en-1-yloxy, n-Hex-4-en-1-yloxy, n-Hex-5-en-1-yloxy, 3-Methylpent-1-en-1-yloxy, 4-Methylpent-1-en-1-yloxy, 1-Methylpent-2-en-1-yloxy, 2-Methylpent-2-en-1-yloxy, 3-Methylpent-2-en-1-yloxy, 4-Methylpent-2-en-1-yloxy, 1-Methylpent-3-en-1-yloxy, 2-Methylpent-3-en-1-yloxy, 3-Methylpent-3-en-1-yloxy, 4-Methylpent-3-en-1-yloxy, 1-Methylpent-4-en-1-yloxy, 2-Methylpent-4-en-1-yloxy, 3-Methylpent-4-en-1-yloxy, 4-Methylpent-4-en-1-yloxy, 1,1-Dimethyl-but-2-en-1-yloxy, 1,1-Dimethyl-but-3-en-1-yloxy, 1,2-Dimethyl-but-2-en-1-yloxy, 1,2-Dimethyl-but-3-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-2-en-1-yloxy, 2,3-Dimethyl-but-3-en-1-yloxy, 3,3-Dimethyl-but-2-en-1-yloxy, 1-Ethylbut-2-en-1-yloxy, 1-Ethylbut-3-en-1-yloxy, 2-Ethylbut-2-en-1-yloxy, 2-Ethylbut-3-en-1-yloxy, 1,1,2-Trimethylprop-2-en-1-yloxy, 1-Ethyl-1-methyl-prop-2-en-1-yloxy, 1-Ethyl-2-methyl-prop-1-en-1-yloxy, 1-Ethyl-2-methyl-prop-2-en-1-yloxy, vorzugsweise Prop-2-en-1-yloxy;
- C₃-C₆-Alkinyloxy: Prop-2-in-3-yloxy, n-But-1-in-4-yloxy, n-But-2-in-1-yloxy, n-Pent-1-in-3-yloxy, n-Pent-1-in-4-yloxy, n-Pent-1-in-5-yloxy, n-Pent-2-in-1-yloxy, n-Pent-2-in-4-yloxy, n-Pent-2-in-5-yloxy, 3-Methyl-but-1-in-3-yloxy, 3-Methyl-but-1-in-4-yloxy, n-Hex-1-in-3-yloxy, n-Hex-1-in-4-yloxy, n-Hex-1-in-5-yloxy, n-Hex-1-in-6-yloxy, n-Hex-2-in-1-yloxy, n-Hex-2-in-4-yloxy, n-Hex-2-in-5-yloxy, n-Hex-2-in-6-yloxy, n-Hex-3-in-1-yloxy, n-Hex-3-in-2-yloxy, 3-Methyl-pent-1-in-3-yloxy, 3-Methyl-pent-1-in-4-yloxy, 3-Methyl-pent-1-in-5-yloxy, 4-Methyl-pent-2-in-4-yloxy, 4-Methyl-pent-2-in-5-yloxy, vorzugsweise Prop-2-in-1-yloxy;
- Aryloxy-C₁-C₆-alkyl: Aryloxymethyl, 1-(Aryloxy) ethyl, 2-(Aryloxy)ethyl, 1-(Aryloxy)prop-1-yl, 2-(Aryloxy)prop-1-yl, 3-(Aryloxy)prop-1-yl, 1-(Aryloxy)but-1-yl, 2-(Aryloxy)but-1-yl, 3-(Aryloxy)but-1-yl, 4-(Aryloxy)but-1-yl, 1-(Aryloxy)but-2-yl, 2-(Aryloxy)but-2-yl, 3-(Aryloxy)but-2-yl, 3-(Aryloxy)but-2-yl, 4-(Aryloxy)but-2-yl, 1-(Aryloxymethyl)-eth-1-yl, 1-(Aryloxymethyl)-1-(methyl)-eth-1-yl, 1-(Aryloxymethyl)-prop-1-yl, vorzugsweise Aryloxymethyl und 1-(Aryloxy)ethyl;
- Aryl-C₃-C₆-alkenyl: z.B. 3-Aryl-allyl, 4-Aryl-but-2-enyl, 4-Aryl-but-3-enyl, 5-Aryl-pent-4-enyl, vorzugsweise 3-Aryl-allyl oder 4-Aryl-but-2-enyl;
- eine gesättigte oder ungesättigte 4- bis 6-gliedrige Heterocyclyl-C₁-C₄-alkylgruppe: (Heterocyclyl)methyl, 1-(Heterocyclyl)ethyl, 2-(Heterocyclyl)ethyl, 1-(Heterocyclyl)prop-1-yl, 2-(Heterocyclyl)prop-1-yl, 3-(Heterocyclyl)prop-1-yl, 1-(Heterocyclyl)but-1-yl, 2-(Heterocyclyl)but-1-yl, 3-(Heterocyclyl)but-1-yl, 4-(Heterocyclyl)but-1-yl, 1-(Heterocyclyl)but-2-yl, 2-(Heterocyclyl)but-2-yl, 3-(Heterocyclyl)but-2-yl, 3-(Heterocyclyl)but-2-yl, 4-(Heterocyclyl)but-2-yl, 1-[(Heteroyclyl)methyl]-eth-1-yl, 1-[(Heterocyclyl)methyl]-1-(methyl)-eth-1-yl, 1-[(Heterocyclyl)methyl]-prop-1-yl, vorzugsweise (Heterocyclyl)methyl und 1-(Heterocyclyl)ethyl.

Aryl steht vorzugsweise für Phenyl und Naphthyl, insbesondere für Phenyl;
Aryloxy steht vorzugsweise für Phenoxy, 1-Naphtyloxy und 2-Naphtyloxy, insbesondere für Phenoxy;
Arylthio steht vorzugsweise für Phenylthio, 1-Naphtylthio und 2-Naphtylthio, insbesondere für Phenylthio;
Heteroaryl steht vorzugsweise für einen 5- oder 6-gliedrigen aromatischen Heterocyclus mit einem Sauerstoff- und einem Schwefelatom oder ein 5- oder 6-gliedriger aromatischer Heterocyclus mit 1 oder 2 Heteroatomen, ausgewählt aus einer Gruppe bestehend aus 2 Stickstoffatomen und einem Sauerstoff- oder Schwefelatom: wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, insbesondere Pyridyl, Pyrimidyl, Furanyl und Thienyl.

Unter den gesättigten oder ungesättigten 4- bis 6-gliedrigen Heterocyclen, die neben C-Atomen jeweils ein oder zwei Ringglieder tragen, ausgewählt aus der Gruppe bestehend aus einem Sauerstoff- oder Schwefelatom und ein oder zwei Stickstoffatomen und ein oder zwei Gruppen -N(CH₃)- sind die folgenden Ringe besonders bevorzugt: Oxetanyl, Tetrahydrofuranyl, Tetrahydrothienyl, Pyrrolidinyl, Isoxazolidinyl, Isothiazolidinyl, Pyrazolidinyl, Oxazolidinyl, Thiazolidinyl, Imidazolidinyl, 1,2,4-Oxadiazolidinyl, 1,3,4-Oxadiazolidinyl, 1,2,4-Thiadiazolidinyl, 1,3,4-Thiadiazolidinyl, 1,2,4-Triazolidinyl, 1,3,4-Triazolidinyl, 2,3-Dihydrofuryl, 2,4-Dihydrofuryl, 2,3-Dihydrothienyl, 2,4-Dihydrothienyl, 2,3-Pyrrolinyl, 2,4-Pyrrolinyl, 2,3-Isoxazolinyl, 3,4-Isoxazolinyl, 4,5-Isoxazolinyl, 2,3-Isothiazolinyl, 3,4-Isothiazolinyl, 4,5-Isothiazolinyl, 2,3-Dihydropyrazolyl, 3,4-Dihydropyrazolyl, 4,5-Dihydropyrazolyl, 2,3-Dihydrooxazolyl, 3,4-Dihydrooxazolyl, Thiazolyl, Imidazolyl, 1,2,4-Oxadiazolyl, 1,3,4-Oxadiazolyl, 1,2,4-Thiadiazolyl, 1,3,4-Thiadiazolyl, 1,2,4-Triazolyl und 1,3,4-Triazolyl, 1,3-Dioxolanyl, Piperidinyl, Tetrahydropyridazinyl, Tetrahydropyrimidinyl, Tetrahydropyrazinyl, 1,3,5-Tetrahydrotriazinyl und 1,2,4-Tetrahydrotriazinyl.

Die Arylring und alle gesättigten, ungesättigten oder aromatischen Heterocyclen können gewünschtenfalls an jedem substituierbaren C-Atom einen der folgenden Reste tragen:
- Halogen, vorzugsweise Fluor oder Chlor;
- C₁-C₄-Alkyl,vorzugsweise Methyl;
- C₁- oder C₂-Halogenalkyl, vorzugsweise Trifluormethyl;
- C₃-C₆-Cycloalkyl, vorzugsweise Cyclopropyl;
- C₁-C₄-Alkoxy, vorzugsweise Methoxy;
- C₁- oder C₂-Halogenalkoxy, vorzugsweise Difluormethoxy.

Bevorzugt sind die Arylringe und die Heterocyclen unsubstituiert oder tragen 1 bis 3 der vorstehend genannten Reste.

Für den Fall daß n 2, 3 oder 4 bedeutet, können zwei benachbarte Reste R⁵ auch zusammen einen annellierten 1,3-Butadien-1,4-diyl-Brücke bilden oder eine mono- oder dihalogenierte 1,3-Butadien-1,4-diyl-Brücke bilden, wobei unter den Halogenatomen Fluor, Chlor und Brom besonders bevorzugt sind.

Der Rest -C(R⁴)=N-O-R¹ kann am Phenylrest in 2-, oder bevorzugt in 3- oder 4-Stellung stehen, bezogen auf auf die Brücke -O-CH₂- in 1-Stellung.

Unter den Verbindungen I, bei denen X für =CH-OCH₃ steht, sind diejenigen besonders bevorzugt, bei denen Y Sauerstoff,
- R¹: C₁-C₆-Alkyl, C₂-C₆-Alkenyl, C₃-C₆-Alkinyl, C₁-C₆-Halogenalkyl oder C₃-C₆-Halogenalkenyl;
- R²: Cyano, Chlor, Fluor, Methyl, Trifluormethyl oder Methoxy;
- R³: Wasserstoff, Cyano, Chlor, Fluor, Methyl oder Methoxy;
- R⁴: Cyano, Chlor, Brom, C₁-C₆-Alkoxy, C₁-C₆-Alkylthio, C₃-C₆-Alkenyloxy, Phenyloxy oder Phenylthio und
- R⁵: Cyano, Halogen, C₁-C₆-Alkyl, C₁-C₆-Halogenalkyl, C₁-C₆-Alkoxy oder Phenyl
bedeuten.

Unter den Verbindungen I, bei denen X für =CH-CH₃ steht, sind diejenigen besonders bevorzugt, bei denen Y Sauerstoff,
- R¹: C₁-C₆-Alkyl, C₂-C₆-Alkenyl, C₃-C₆-Alkinyl, C₁-C₆-Halogenalkyl oder C₃-C₆-Halogenalkenyl;
- R²: Cyano, Chlor, Fluor, Methyl, Trifluormethyl oder Methoxy;
- R³: Wasserstoff, Cyano, Chlor, Fluor, Methyl oder Methoxy;
- R⁴: Cyano, Chlor, Brom, C₁-C₆-Alkoxy, C₁-C₆-Alkylthio, C₃-C₆-Alkenyloxy, Phenyloxy oder Phenylthio und
- R⁵: Cyano, Halogen, C₁-C₆-Alkyl, C₁-C₆-Halogenalkyl, C₁-C₆-Alkoxy oder Phenyl
bedeuten.

Unter den Verbindungen I, bei denen X für =N-OCH₃ steht, sind diejenigen besonders bevorzugt, bei denen Y für Sauerstoff oder -NH-steht und
- R¹: C₁-C₆-Alkyl, C₂-C₆-Alkenyl, C₃-C₆-Alkinyl, C₁-C₆-Halogenalkyl oder C₃-C₆-Halogenalkenyl;
- R²: Cyano, Chlor, Fluor, Methyl, Trifluormethyl oder Methoxy;
- R³: Wasserstoff, Cyano, Chlor, Fluor, Methyl oder Methoxy;
- R⁴: Cyano, Chlor, Brom, C₁-C₆-Alkoxy, C₁-C₆-Alkylthio, C₃-C₆-Alkenyloxy, Phenyloxy oder Phenylthio und
- R⁵: Cyano, Halogen, C₁-C₆-Alkyl, C₁-C₆-Halogenalkyl, C₁-C₆-Alkoxy oder Phenyl
bedeuten.

Bei den Zwischenprodukten IVb' und XI' sind diejenigen Bedeutungen der Variablen bevorzugt, die bereits bei den Verbindungen I als bevorzugt angegeben wurden.

Die neuen Verbindungen der Formel I, IVb' und XI' können bei der Herstellung als E/Z-Isomerengemische bezüglich der C=X-Bindung anfallen, die Verbindungen I auch bezüglich der (R¹O)N=C(R⁴)-Bindung. Die Isomerengemische können gewünschtenfalls auf übliche Weise, z. B. durch Kristallisation oder Chromatographie, in die weitgehend reinen Isomeren getrennt werden.

Sowohl die reinen Isomeren als auch alle Mischungen der verschiedenen Isomere werden von der Erfindung umfaßt und sind als Fungizide oder als Schädlingsbekämpfungsmittel verwendbar.

Besonders bevorzugt sind diejenigen Verbindungen I, bei denen
- die C=X-Gruppierung E-konfiguriert ist, also -CO-Y-R⁶ und der Rest -OCH₃ oder -CH₃ trans-ständig sind,

Im Hinblick auf ihre biologische Wirksamkeit gegen Schadpilze und/oder Schädlinge sind die folgenden substituierten Benzoxyphenyl-Derivate I ganz besonders bevorzugt:
- Verbindungen der Formel I-1: 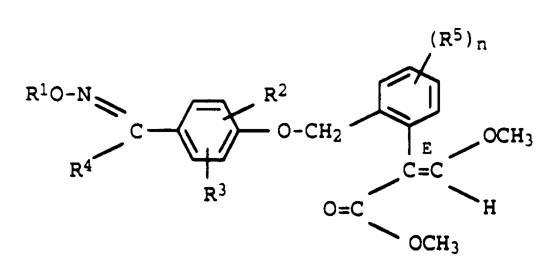 Vertreter dieses Strukturtyps sind die in Tabelle A aufgelisteten Verbindungen Nr. A.0001 bis Nr. A.1958.
- Verbindungen der Formel I-2: 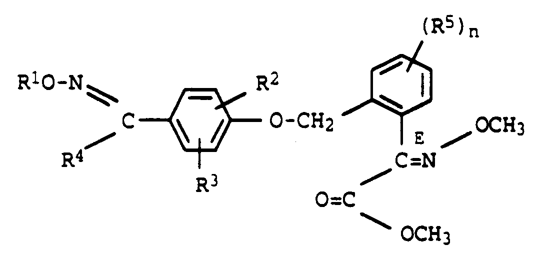 Vertreter dieses Strukturtyps sind die Verbindungen Nr. C.0001 bis Nr. C.1958, die wie die entsprechenden Verbindungen Nr. A.0001 bis Nr. A.1958 der Tabelle A substituiert sind.
- Verbindungen der Formel I-3: 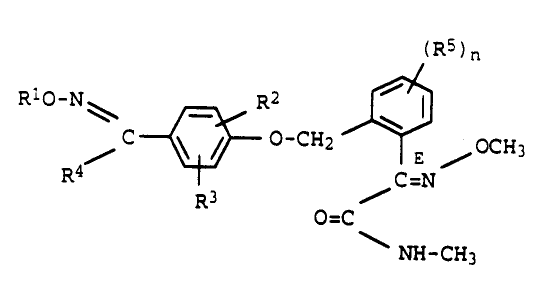 Vertreter dieses Strukturtyps sind die Verbindungen Nr. D.0001 bis Nr. D.1958, die wie die entsprechenden Verbindungen Nr. A.0001 bis Nr. A.1958 der Tabelle A substituiert sind.
- Verbindungen der Formel I-4: 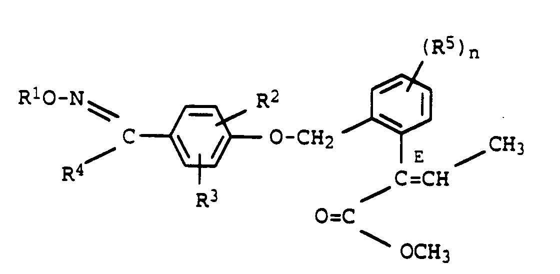 Vertreter dieses Strukturtyps sind die Verbindungen Nr. E.0001 bis Nr. E.1958, die wie die entsprechenden Verbindungen Nr. A.0001 bis Nr. A.1958 der Tabelle A substituiert sind.
- Verbindungen der Formel I-5: 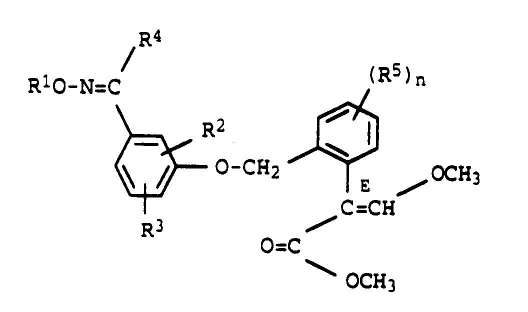 Vertreter dieses Strukturtyps sind die in Tabelle B aufgelisteten Verbindungen Nr. B.001 bis B.544.
- Verbindungen der Formel I-6: 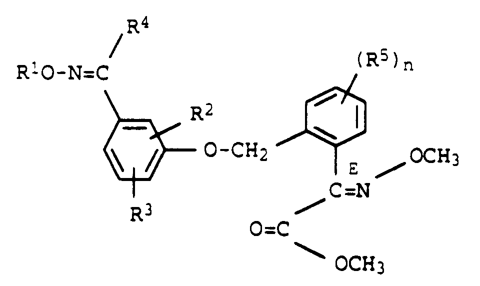 Vertreter dieses Strukturtyps sind die Verbindungen Nr. F.001 bis Nr. F.544, die wie die entsprechenden Verbindungen Nr. B.001 bis Nr. B.544 der Tabelle B substituiert sind.
- Verbindungen der Formel I-7: 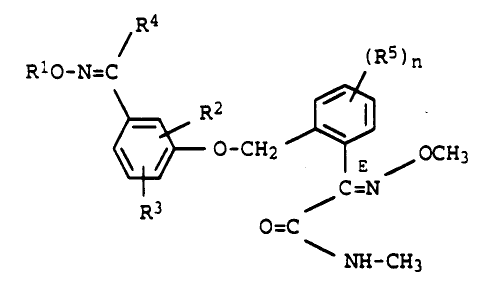 Vertreter dieses Strukturtyps sind die Verbindungen Nr. G.001 bis Nr. G.544, die wie die entsprechenden Verbindungen Nr. B.001 bis Nr. B.544 der Tabelle B substituiert sind.
- Verbindungen der Formel I-8: 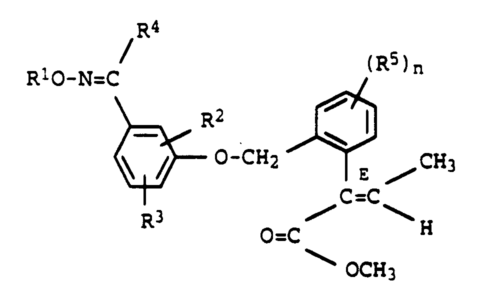 Vertreter dieses Strukturtyps sind die Verbindungen Nr. H.001 bis Nr. H.544, die wie die entsprechenden Verbindungen Nr. B.001 bis Nr. B.544 der Tabelle B substituiert sind.

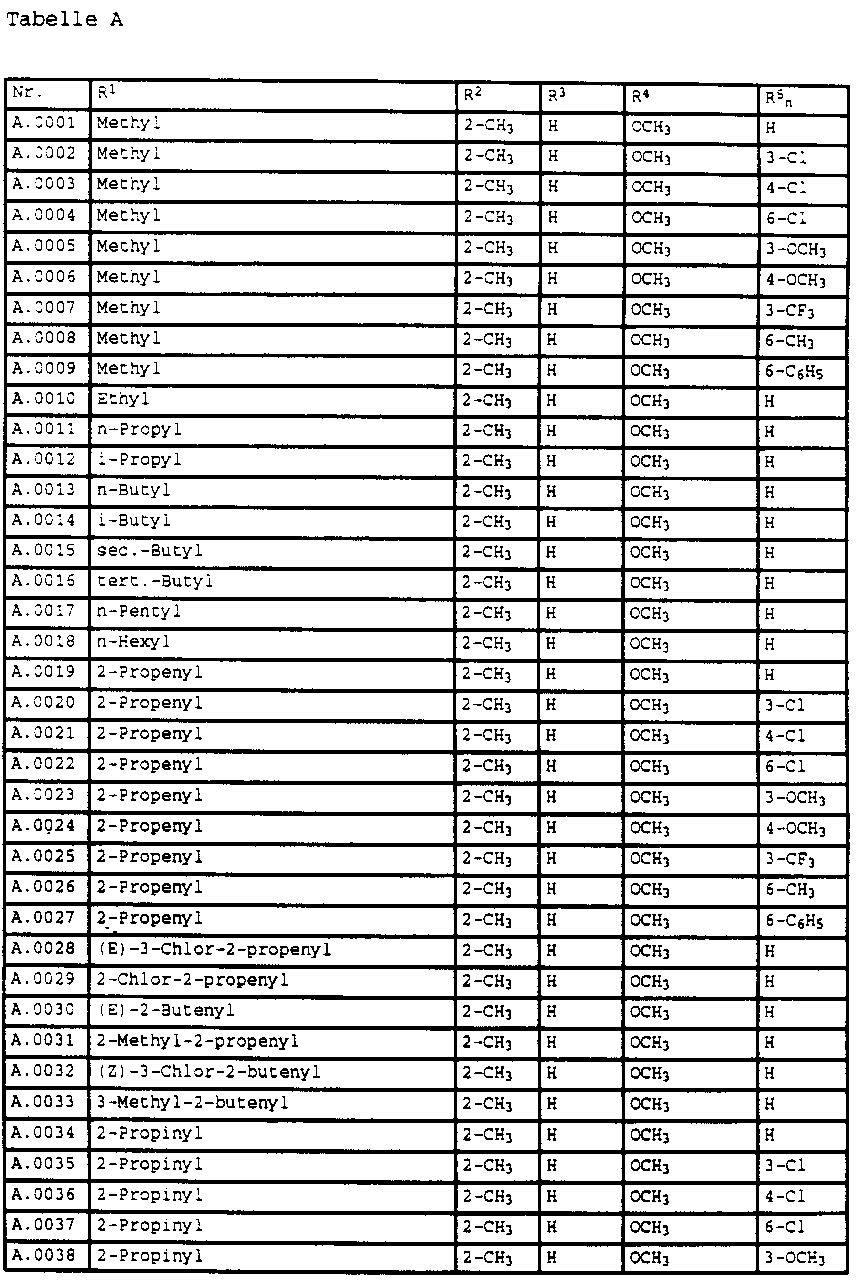

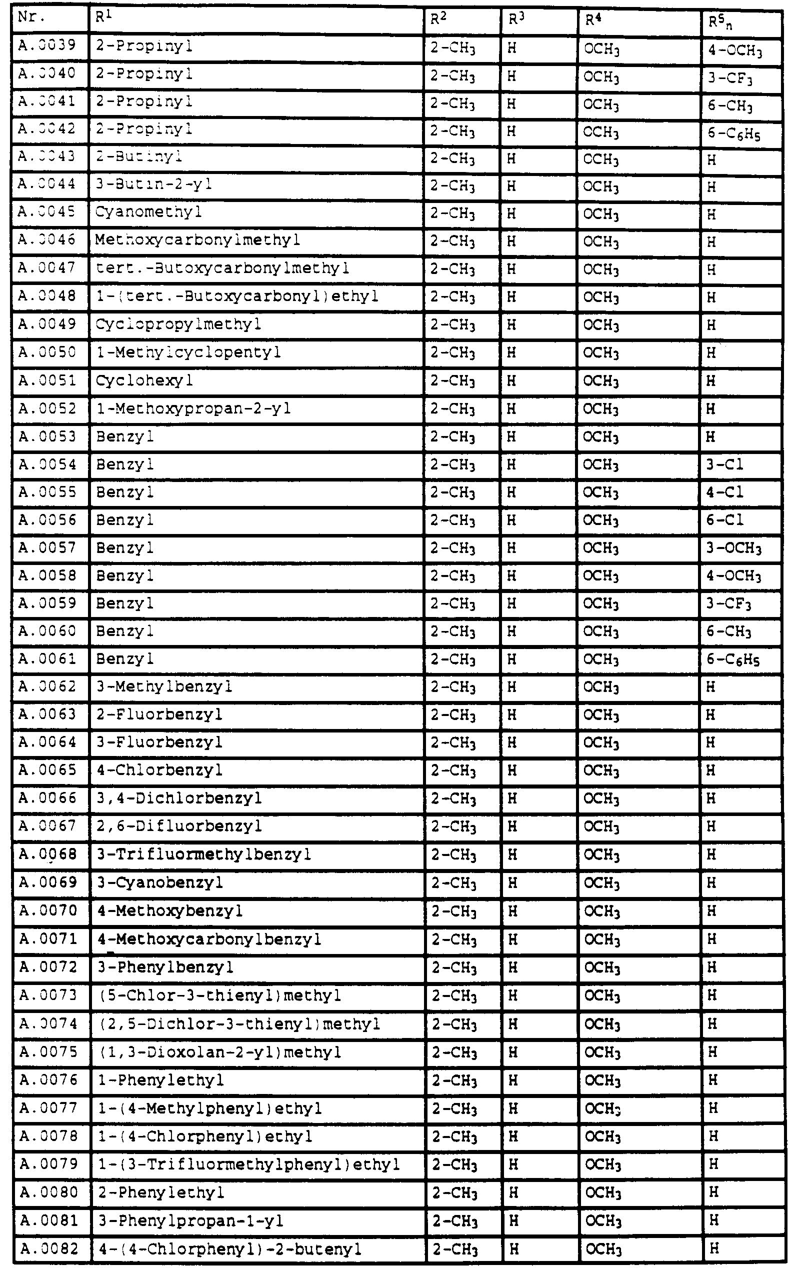

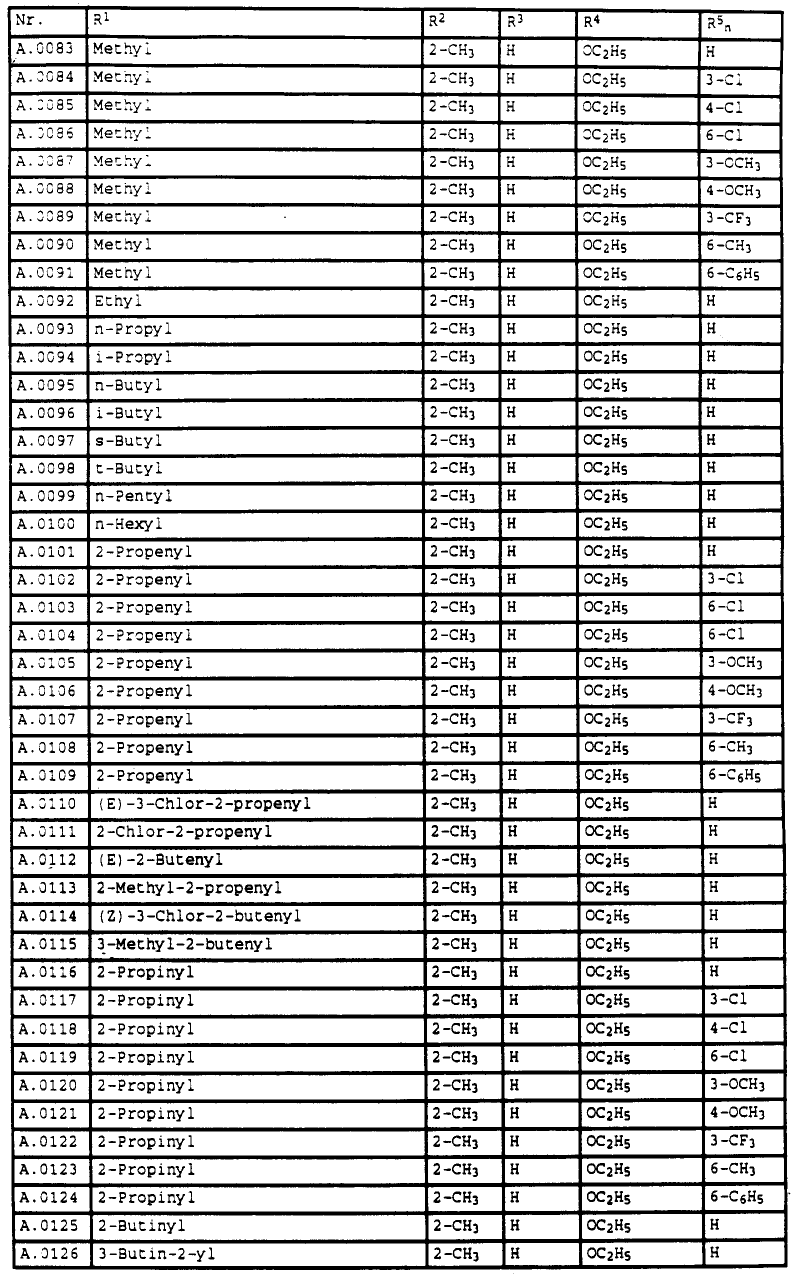

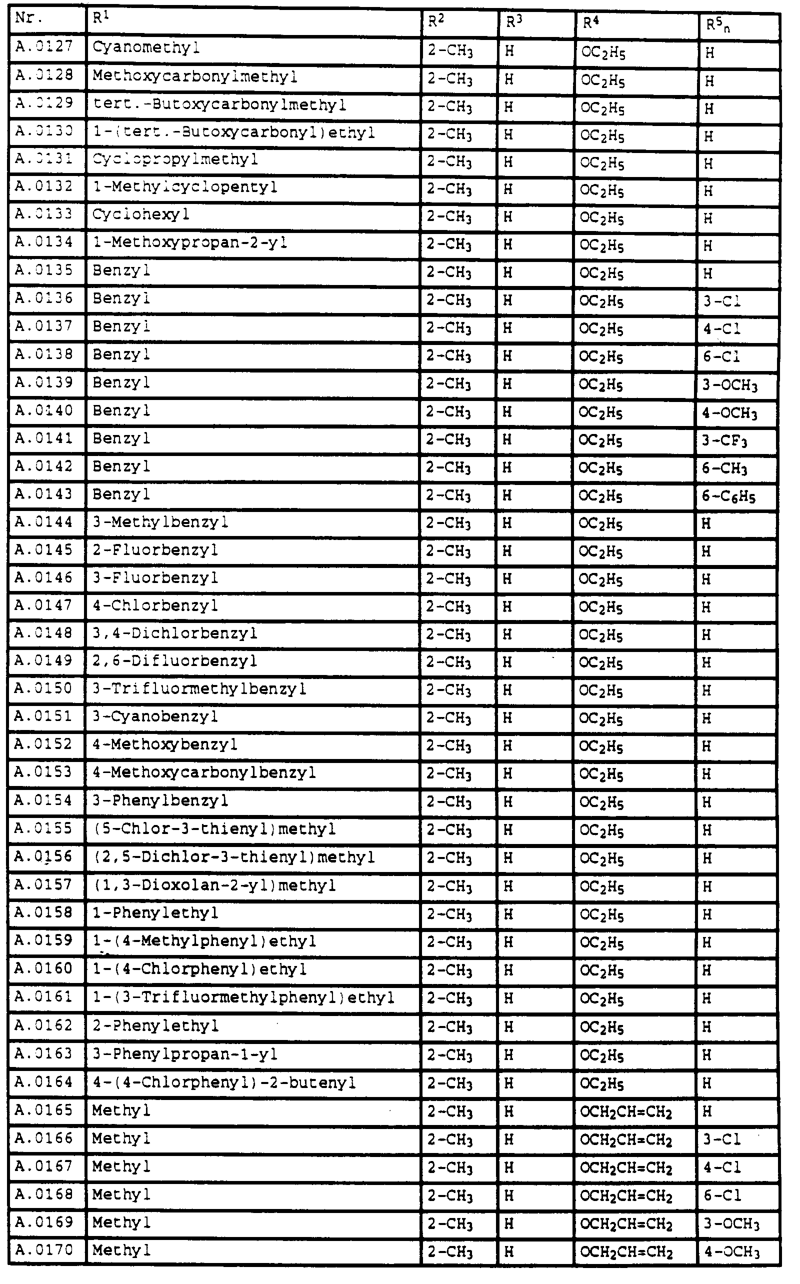

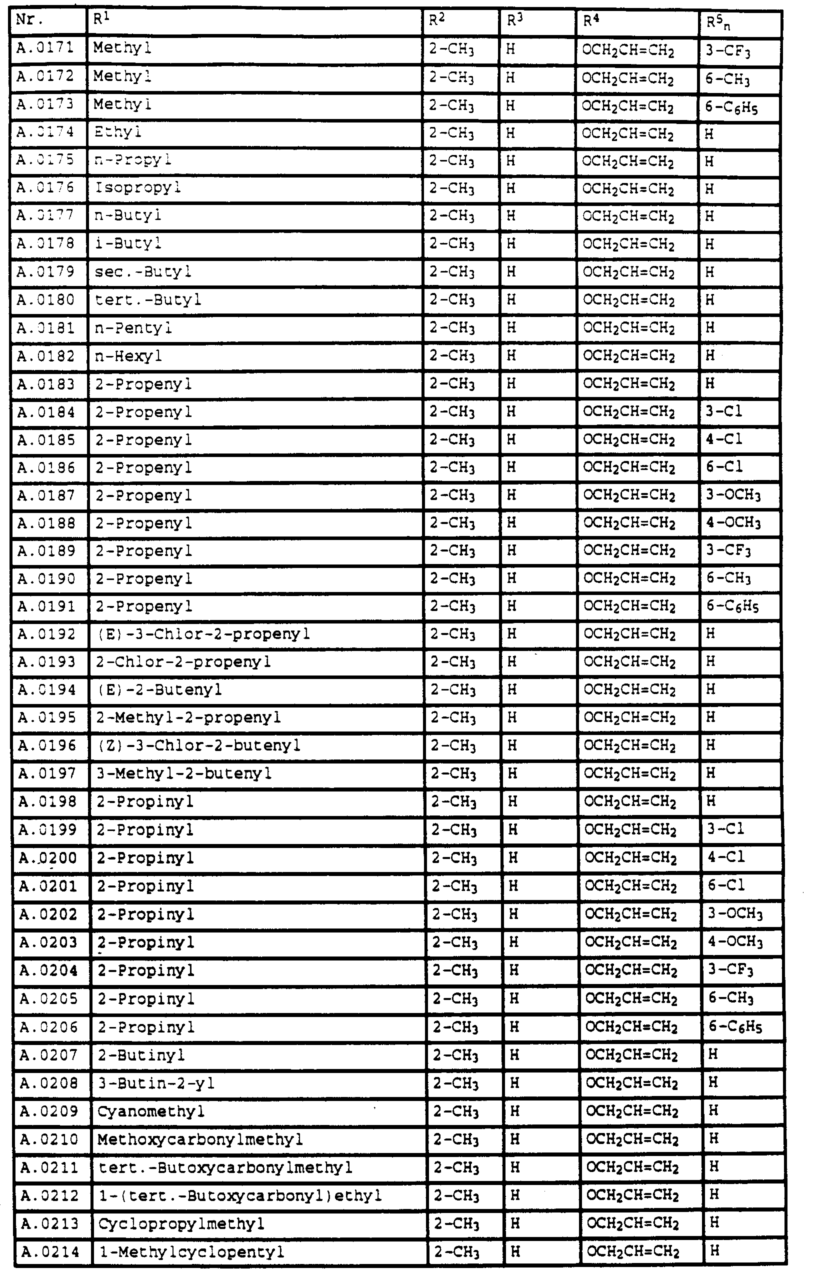

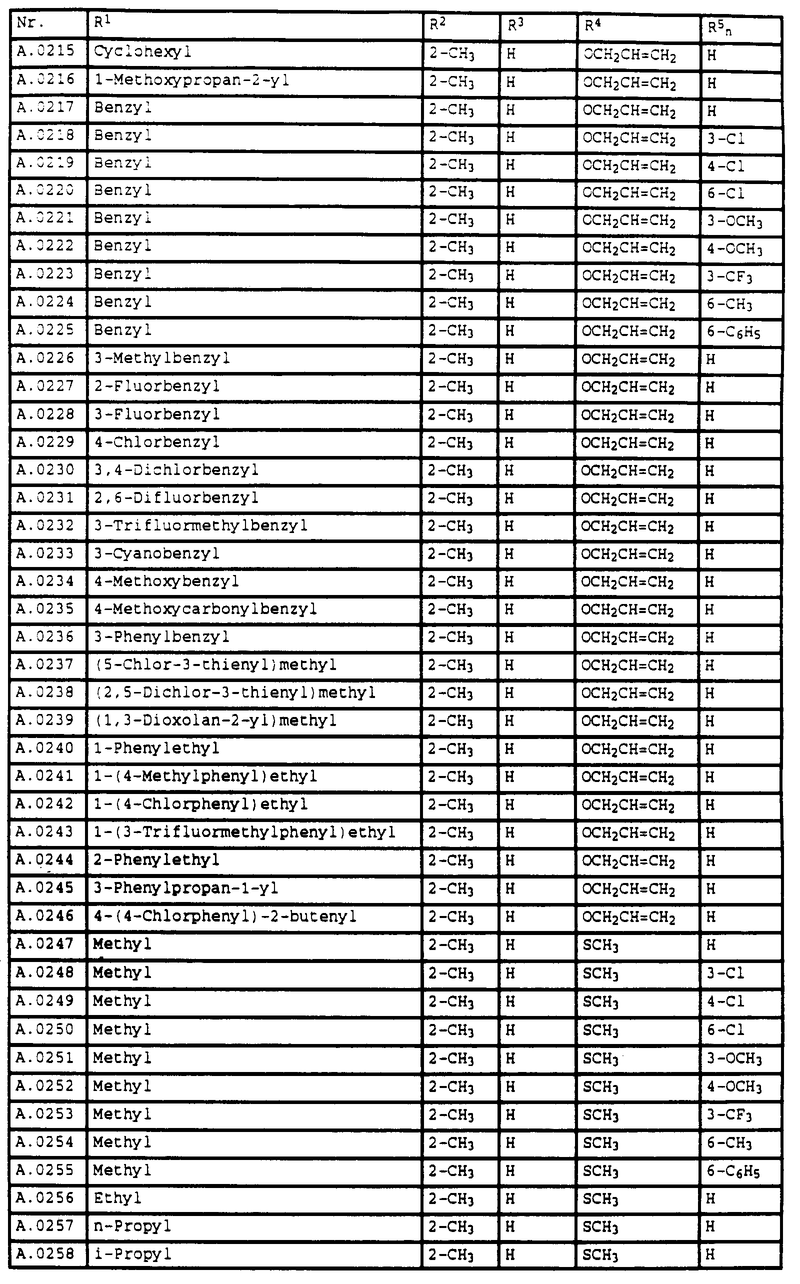

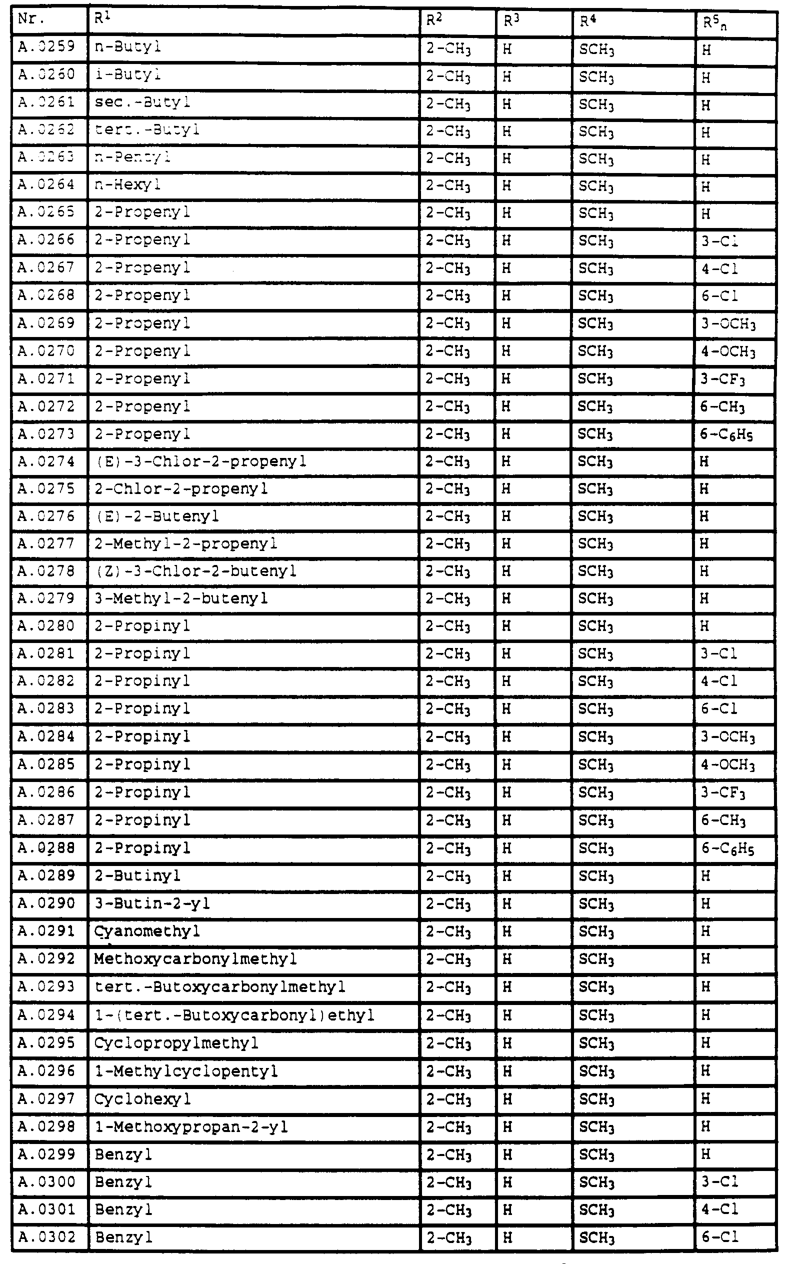

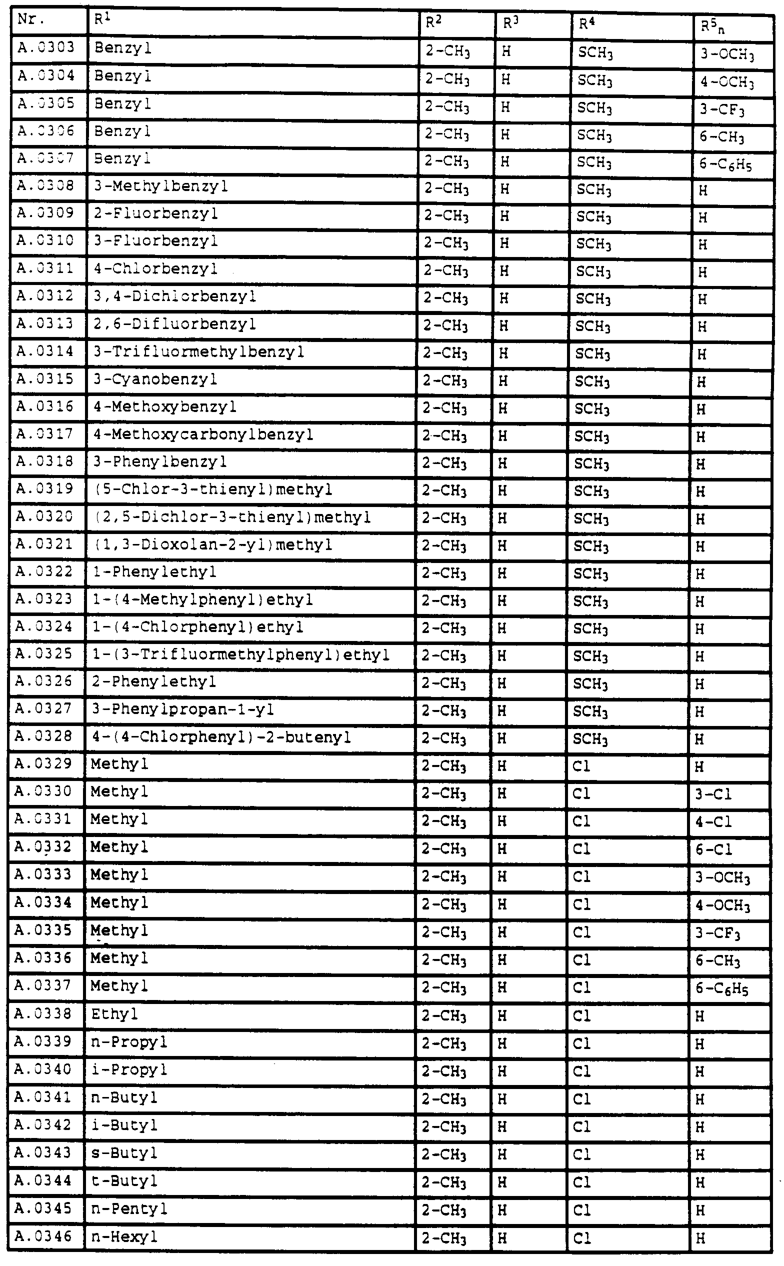

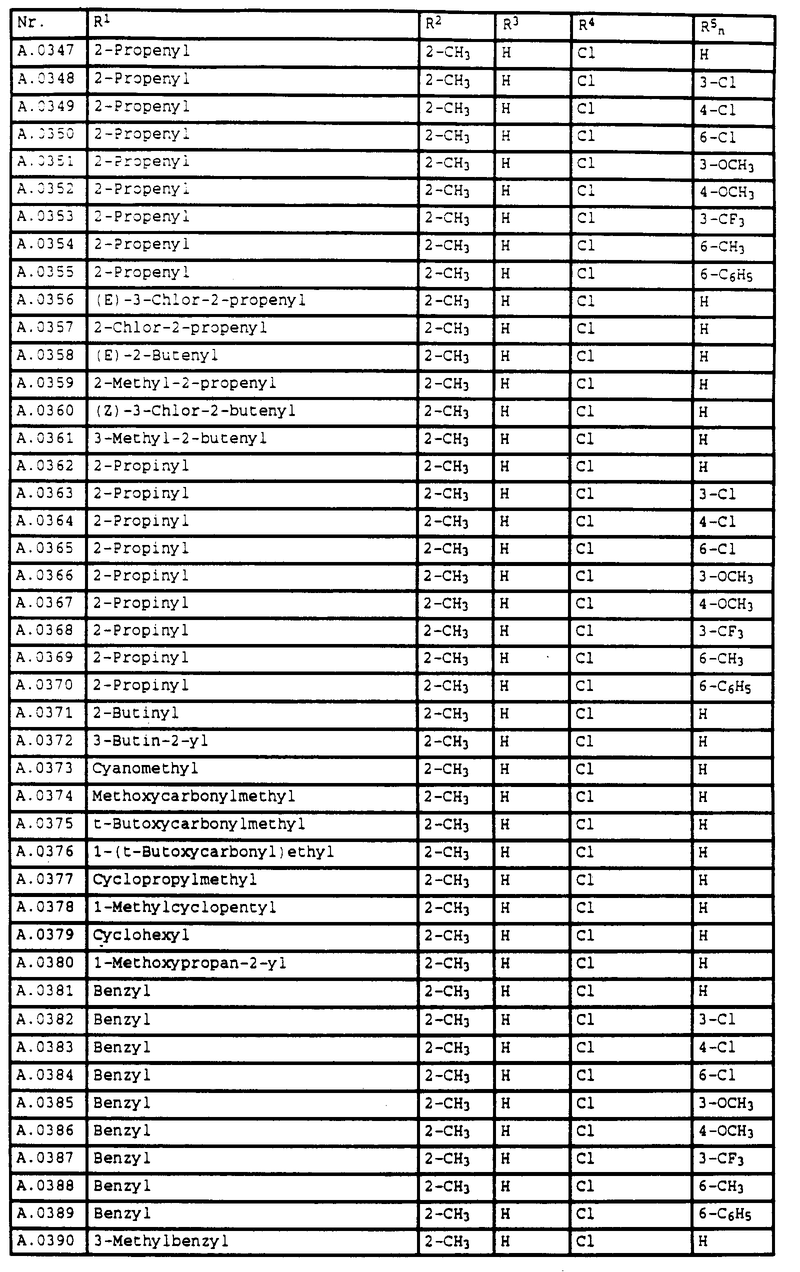

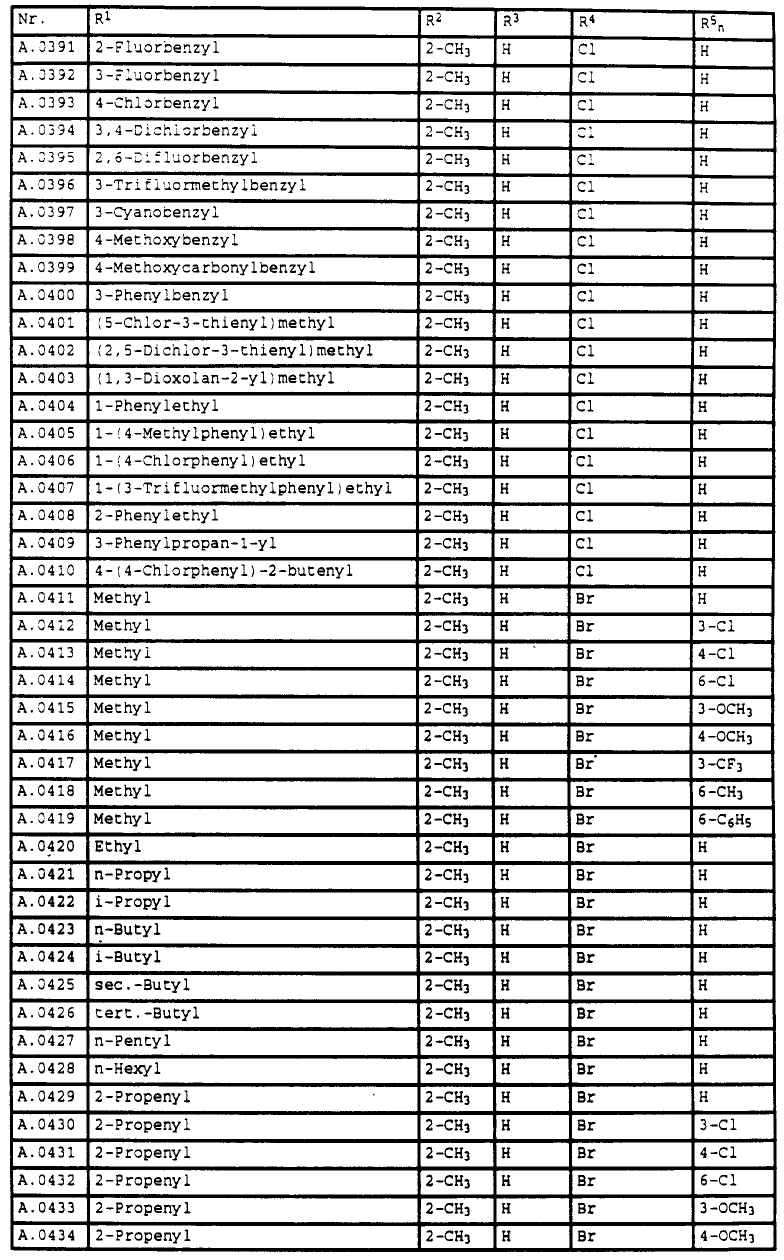

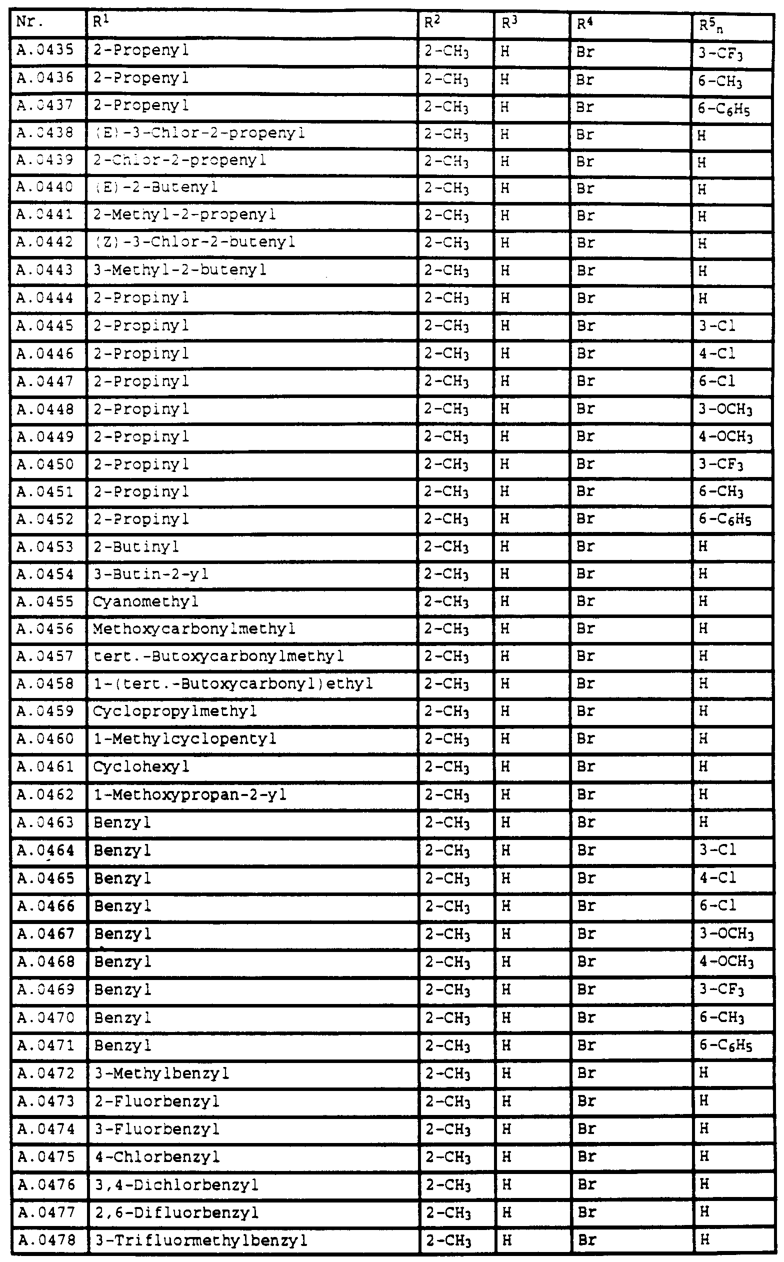

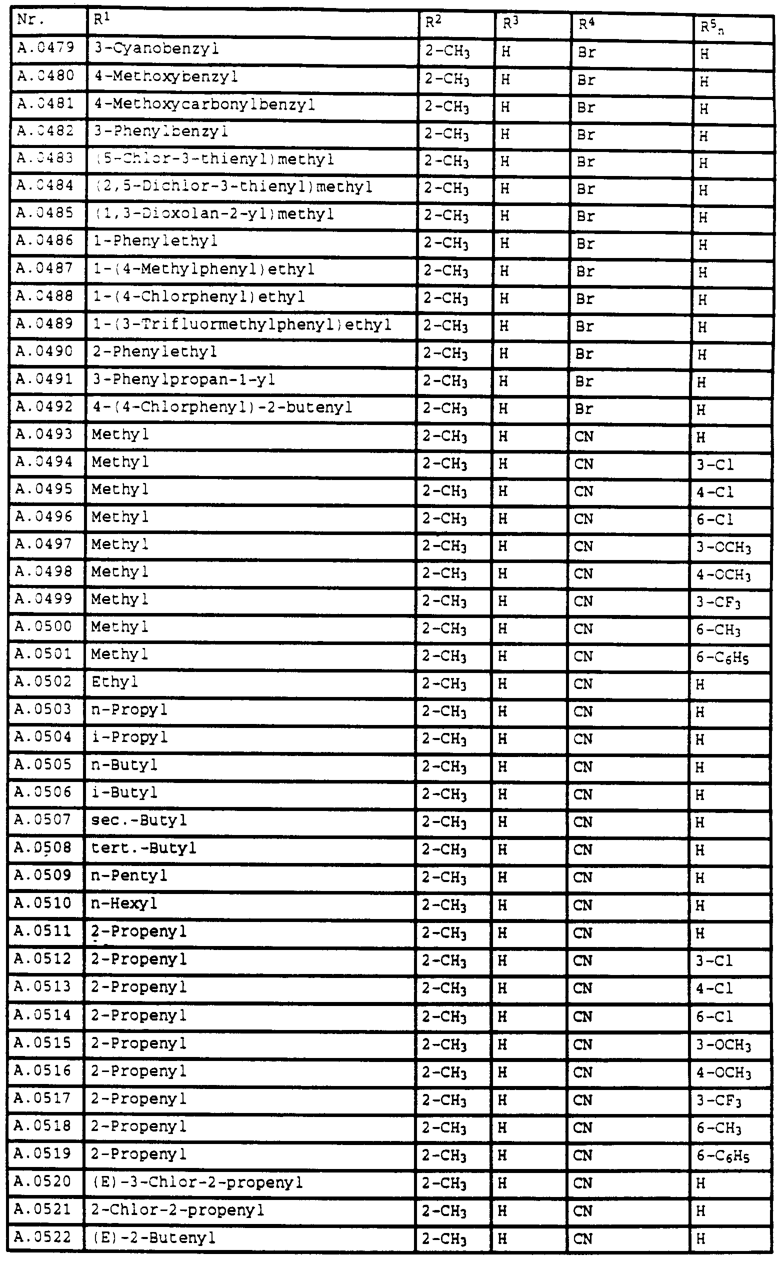

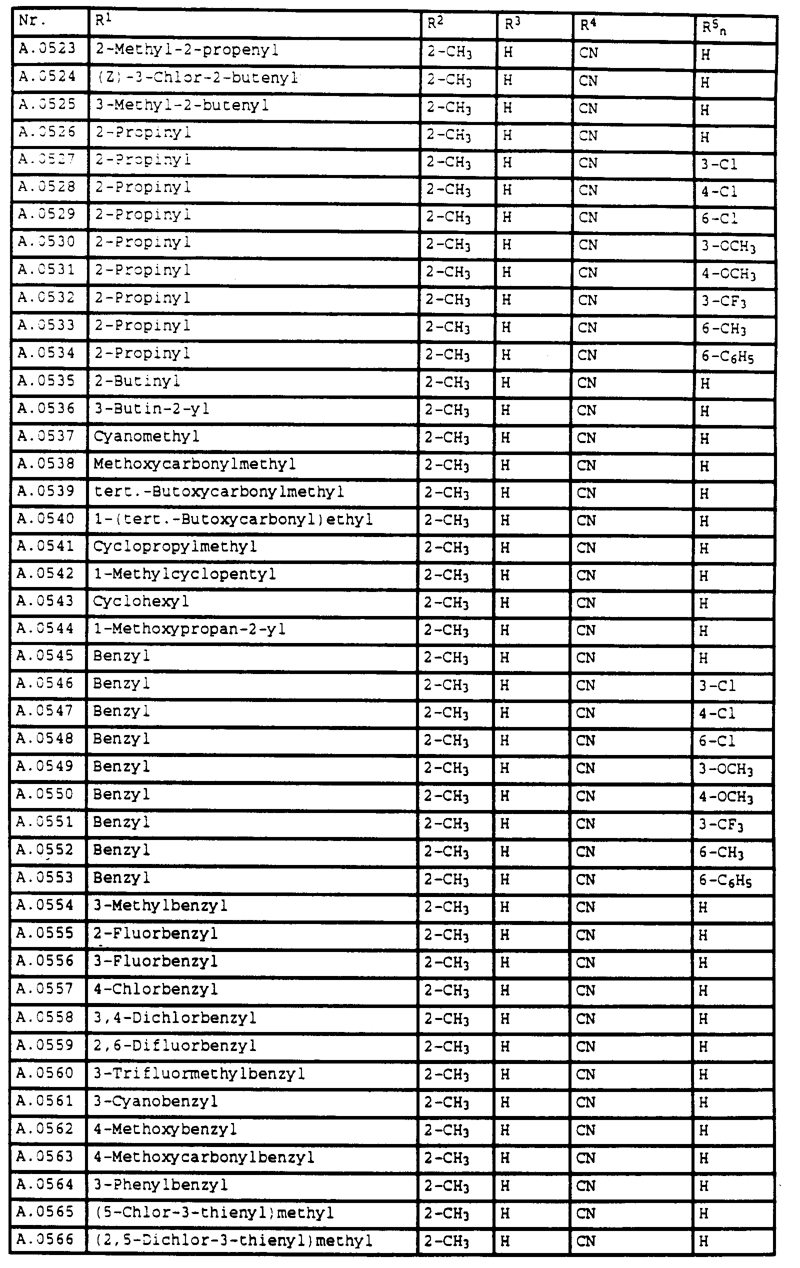

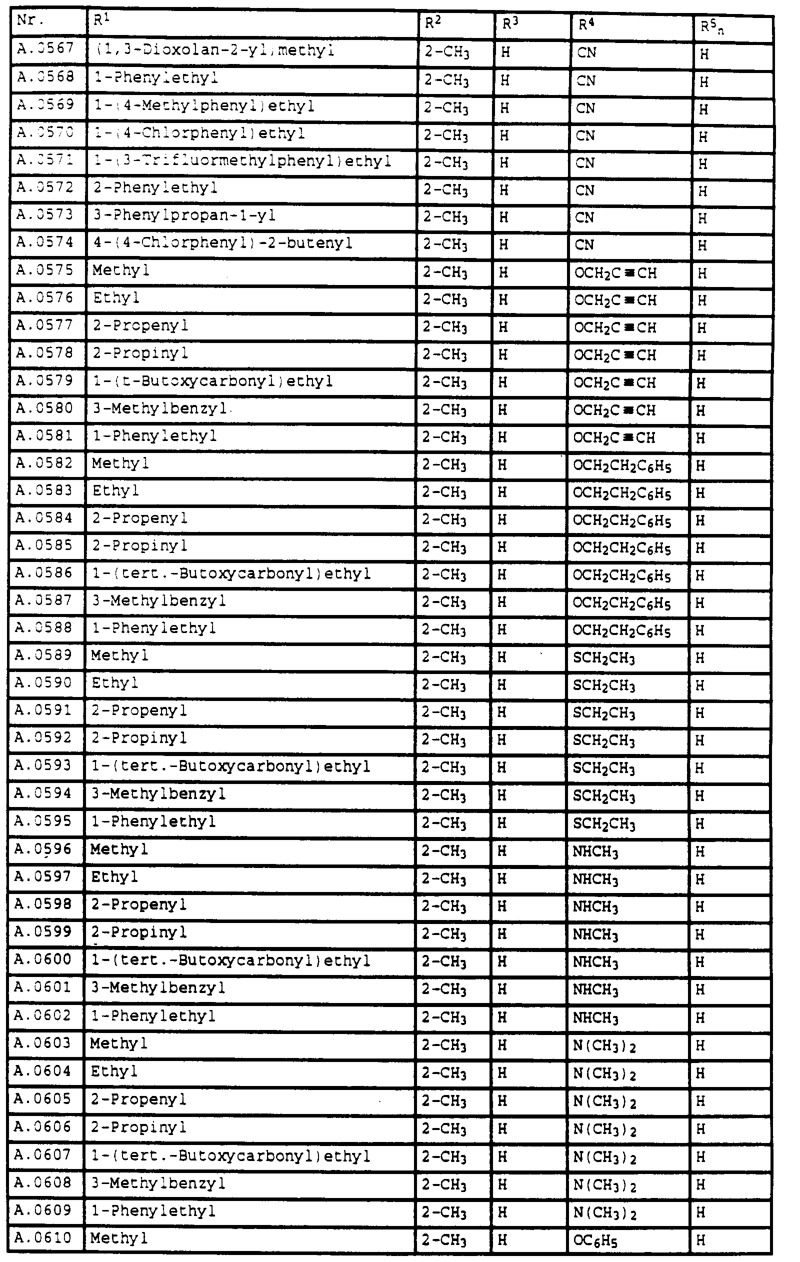

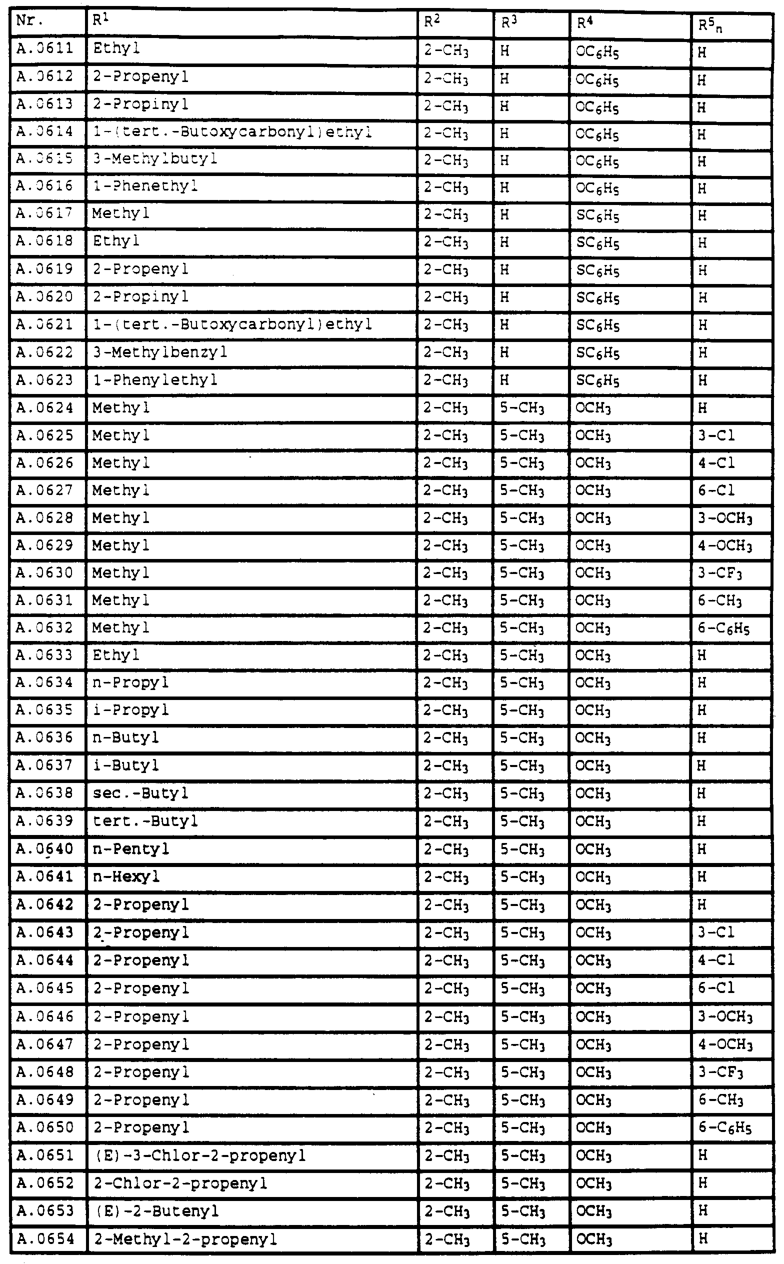

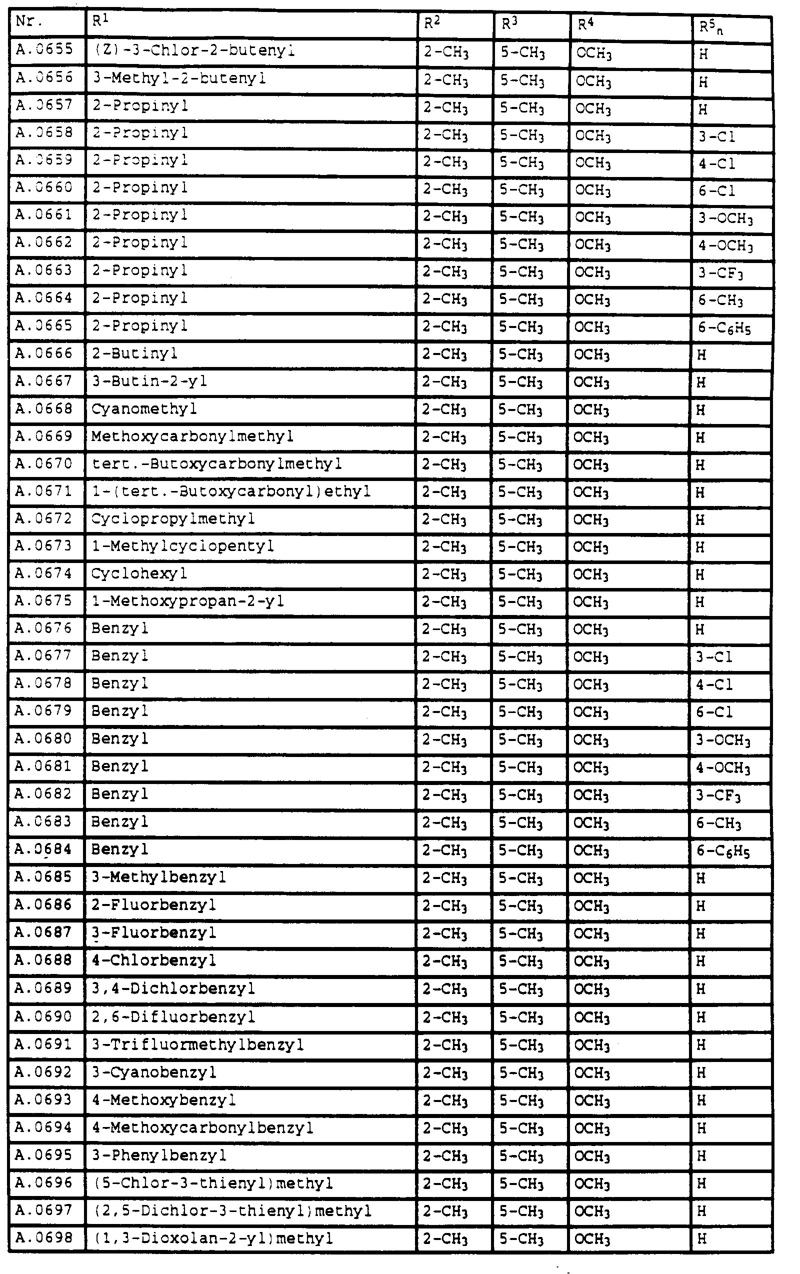

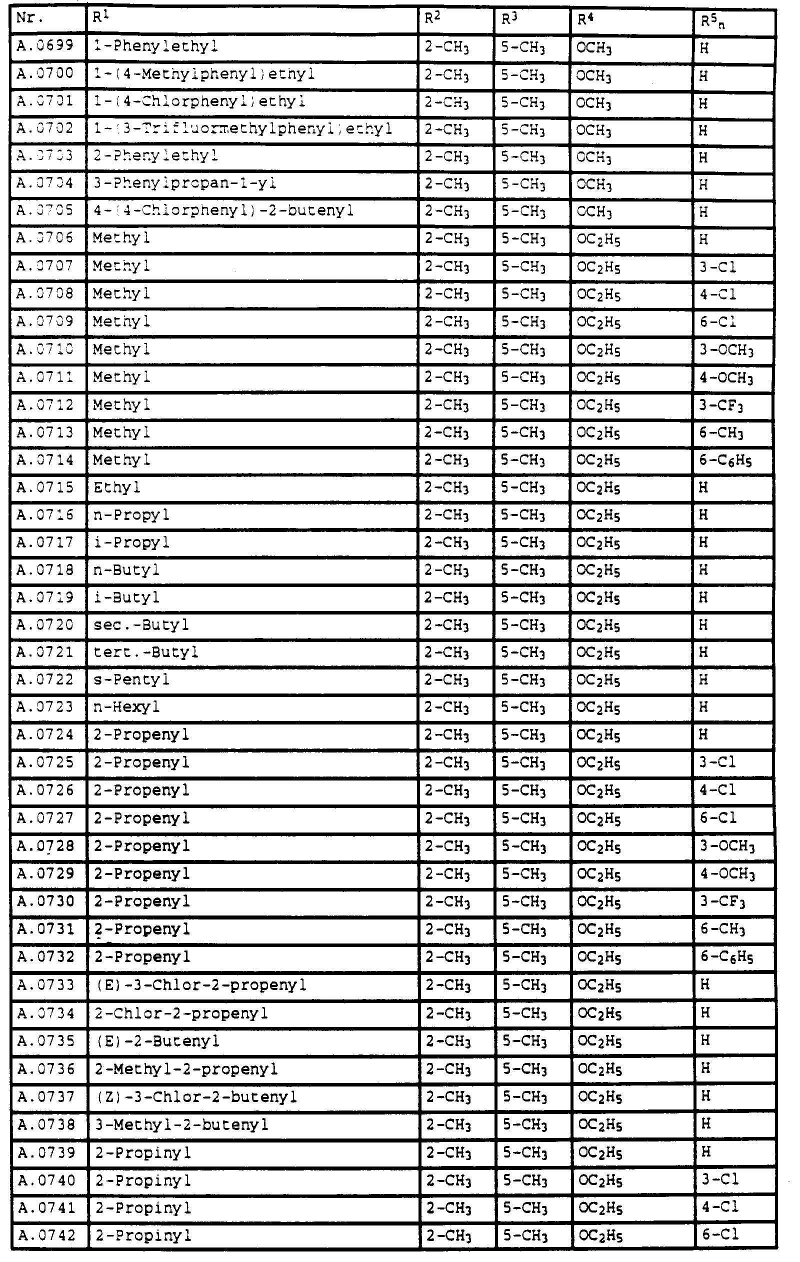

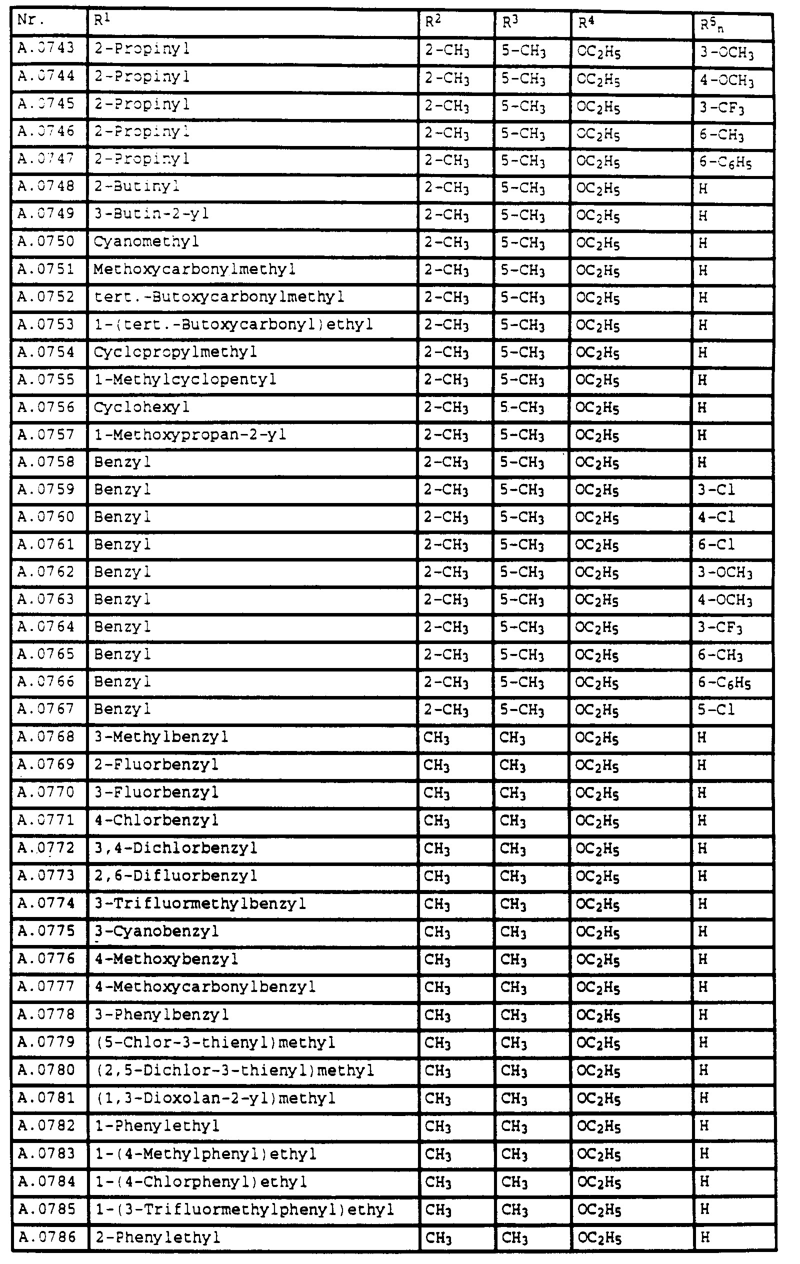

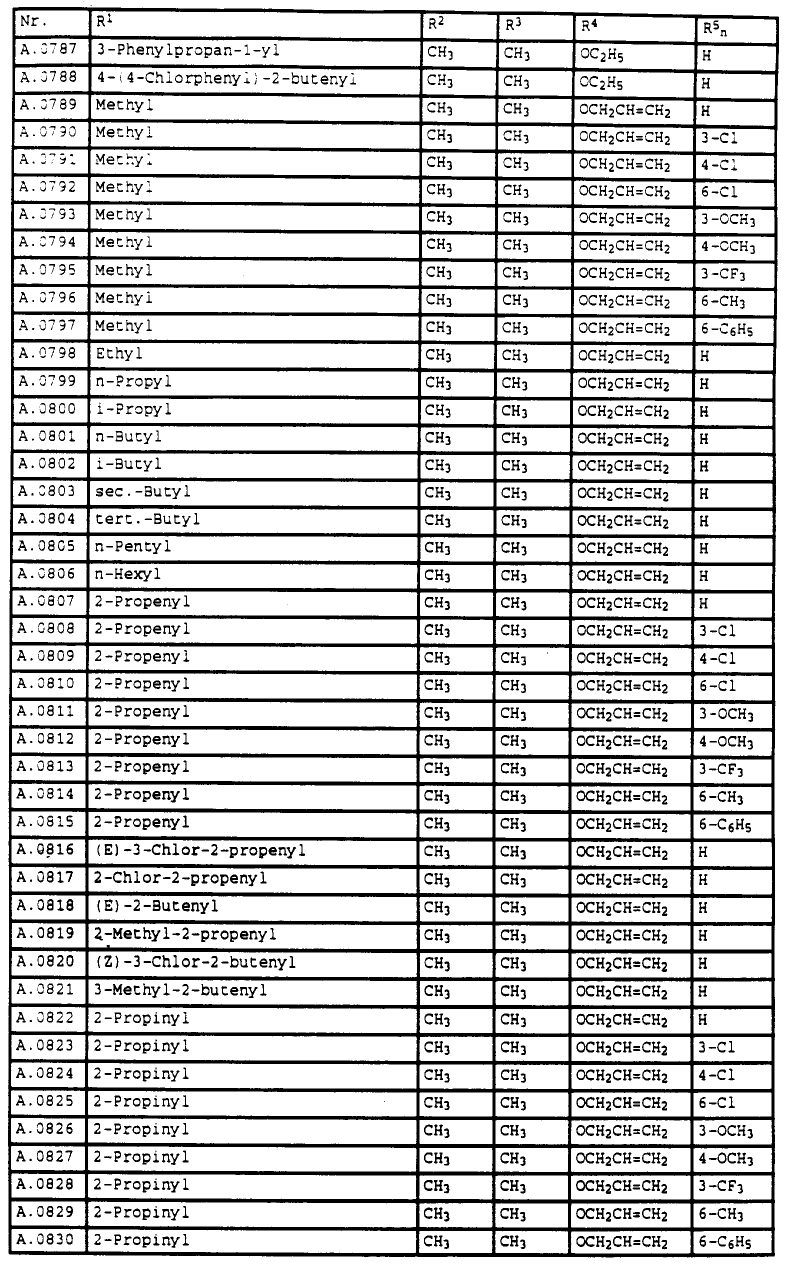

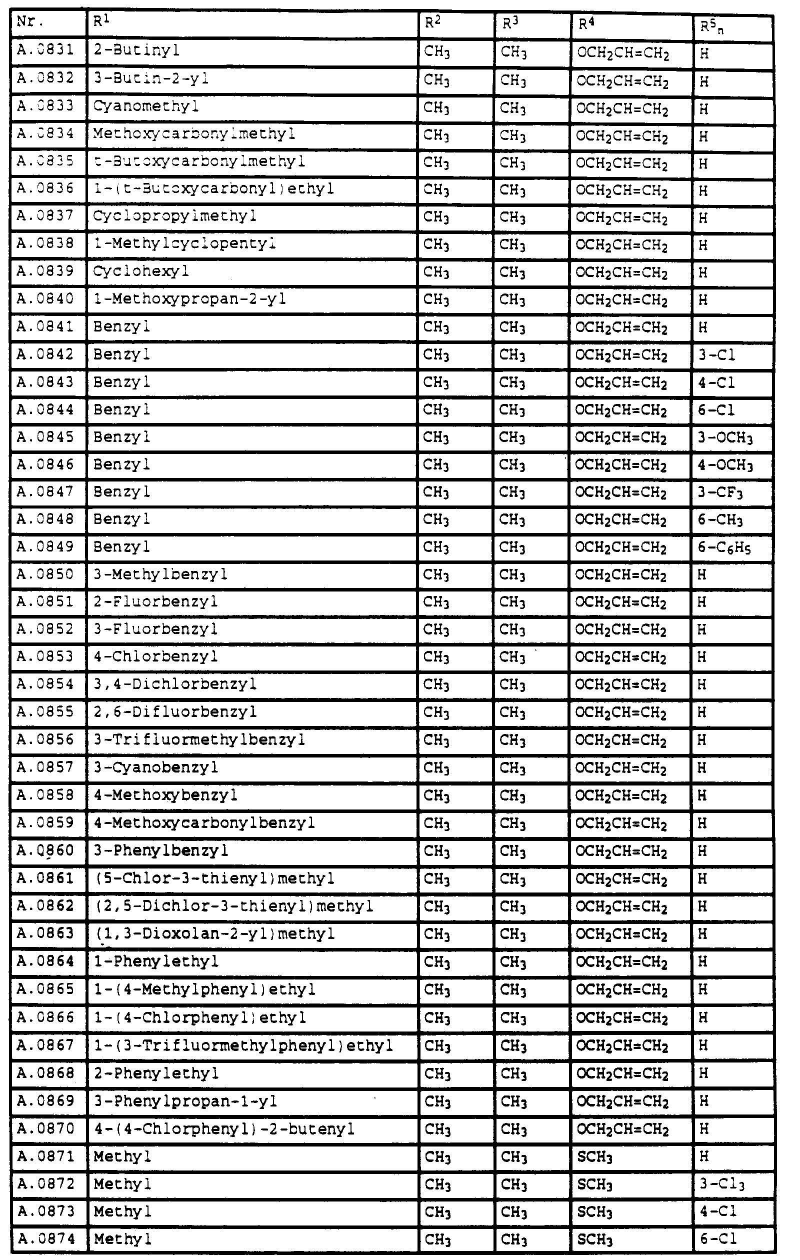

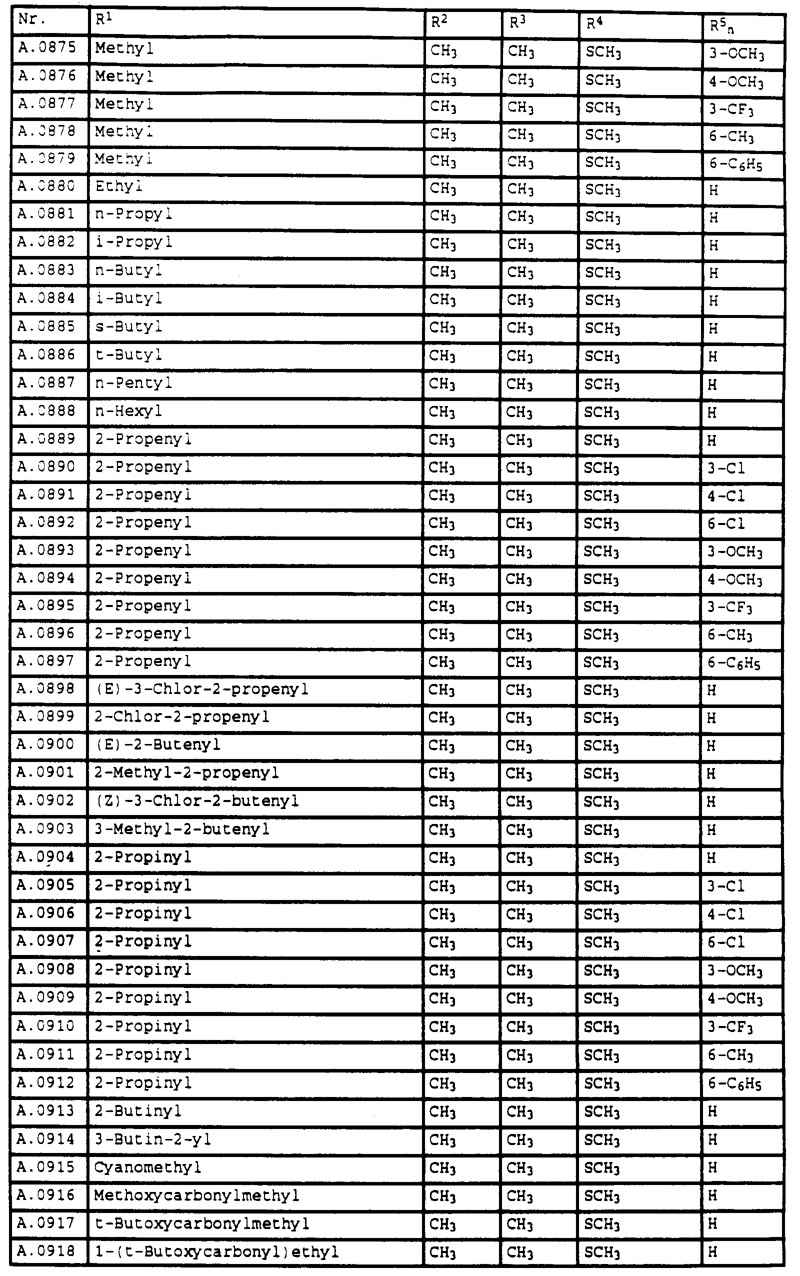

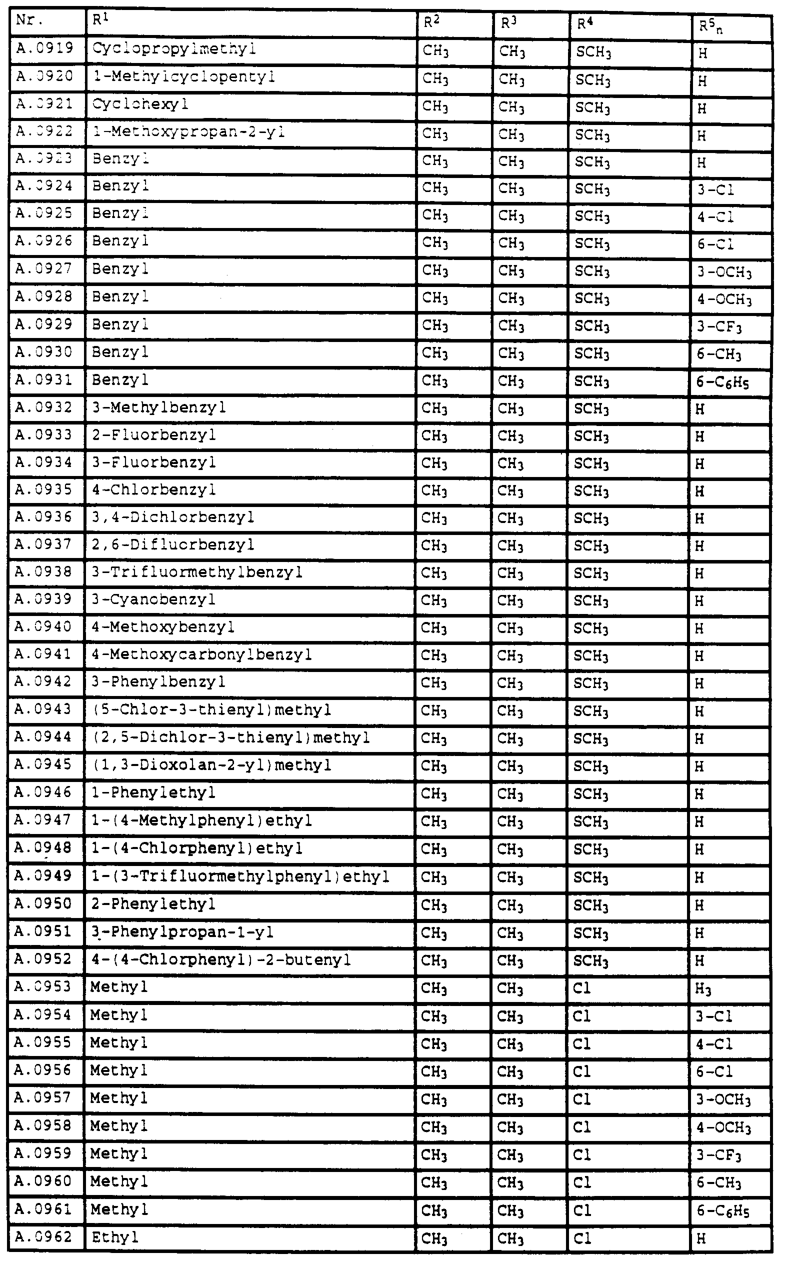

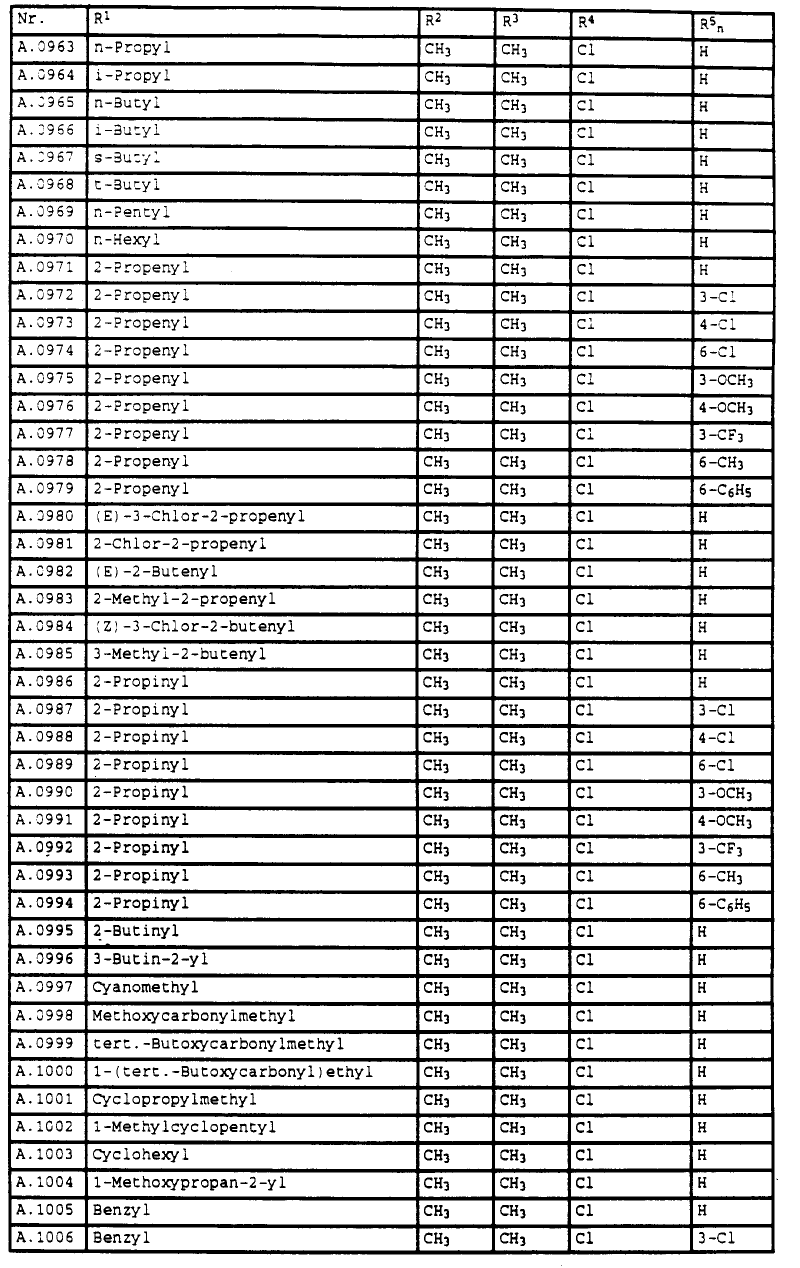

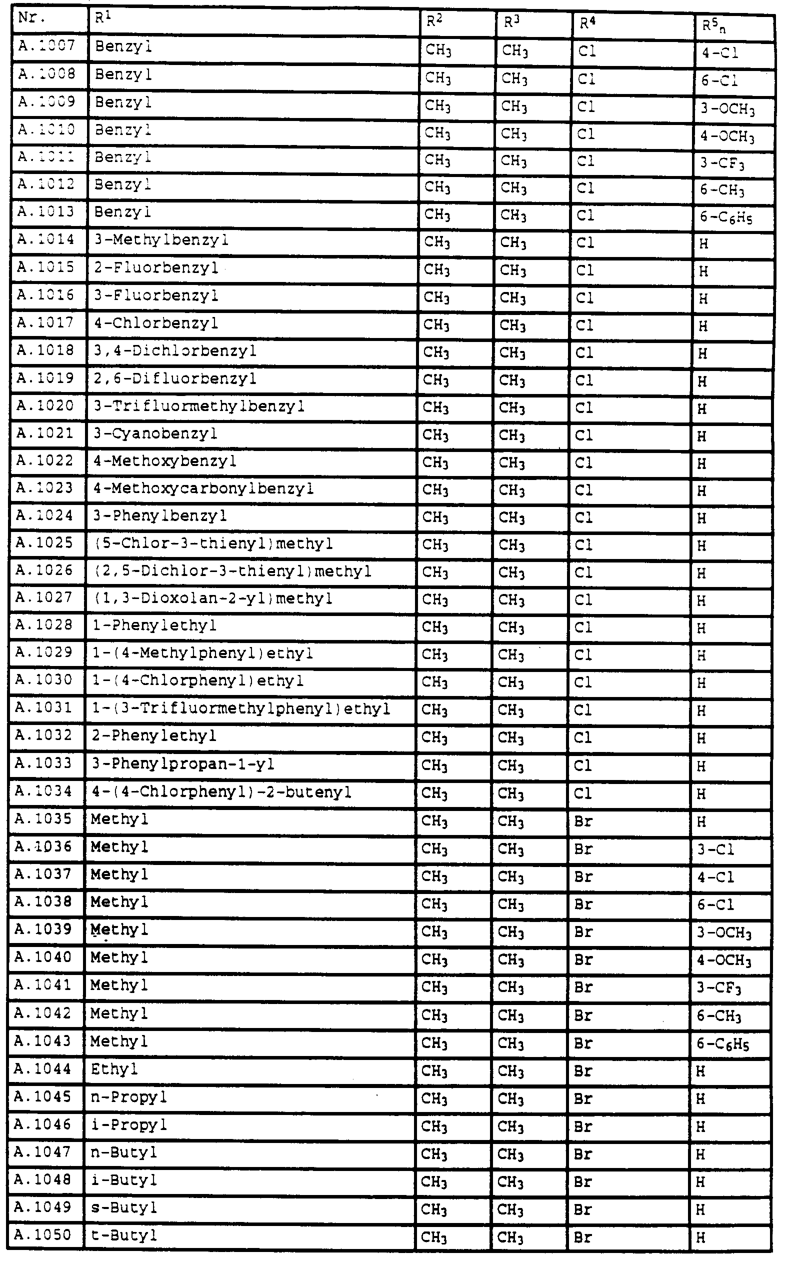

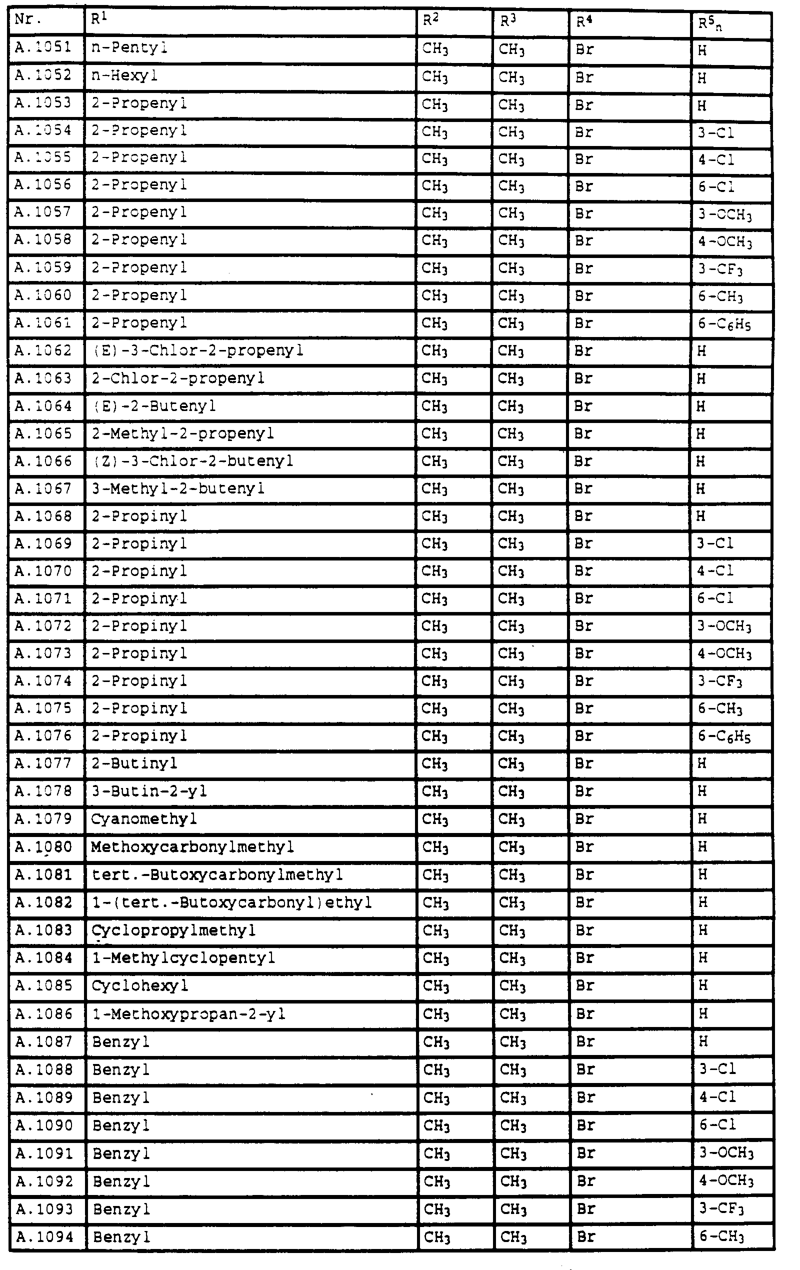

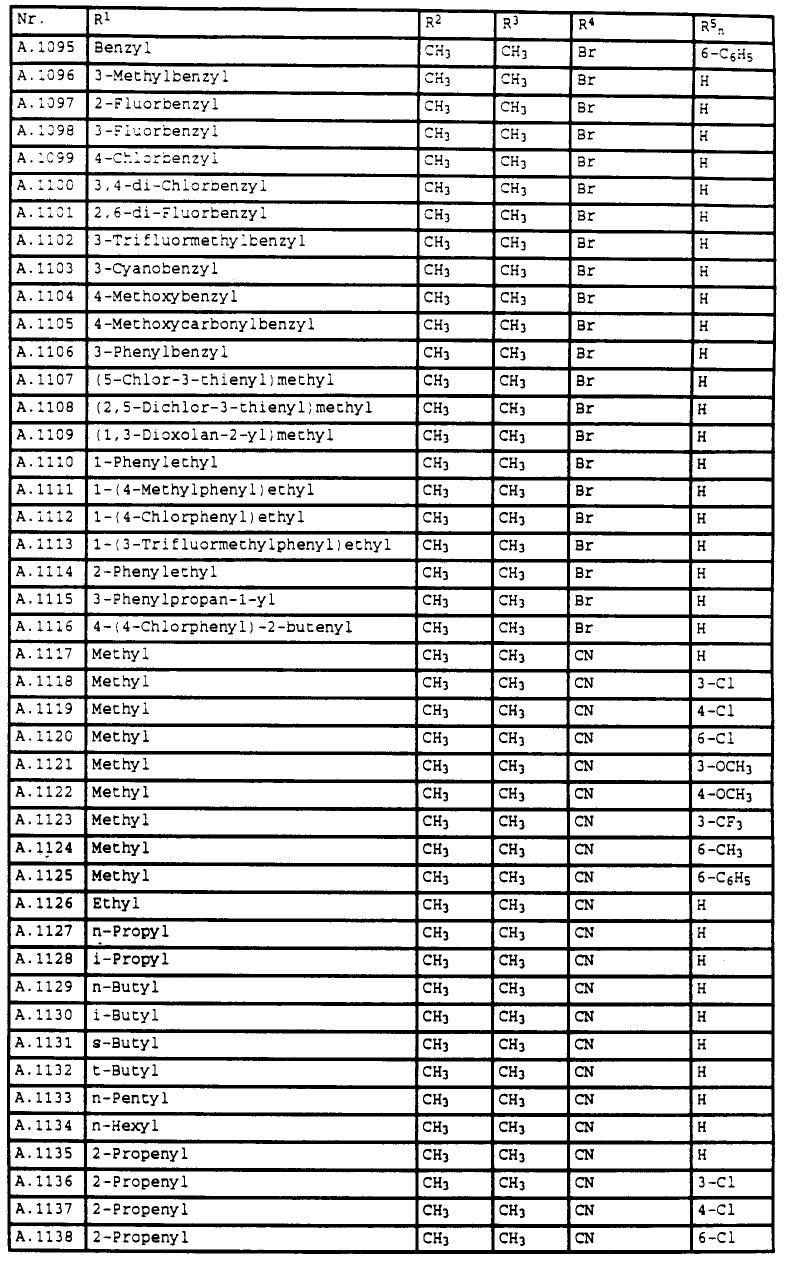

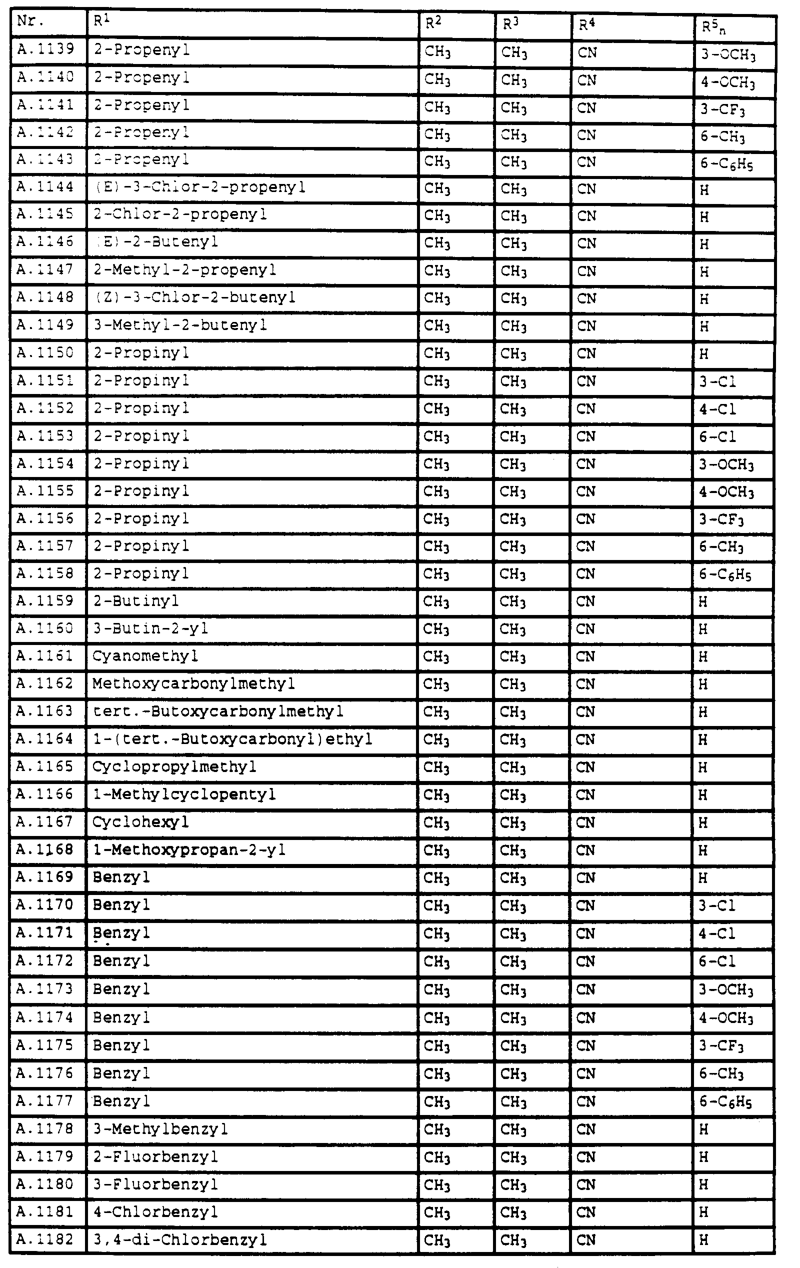

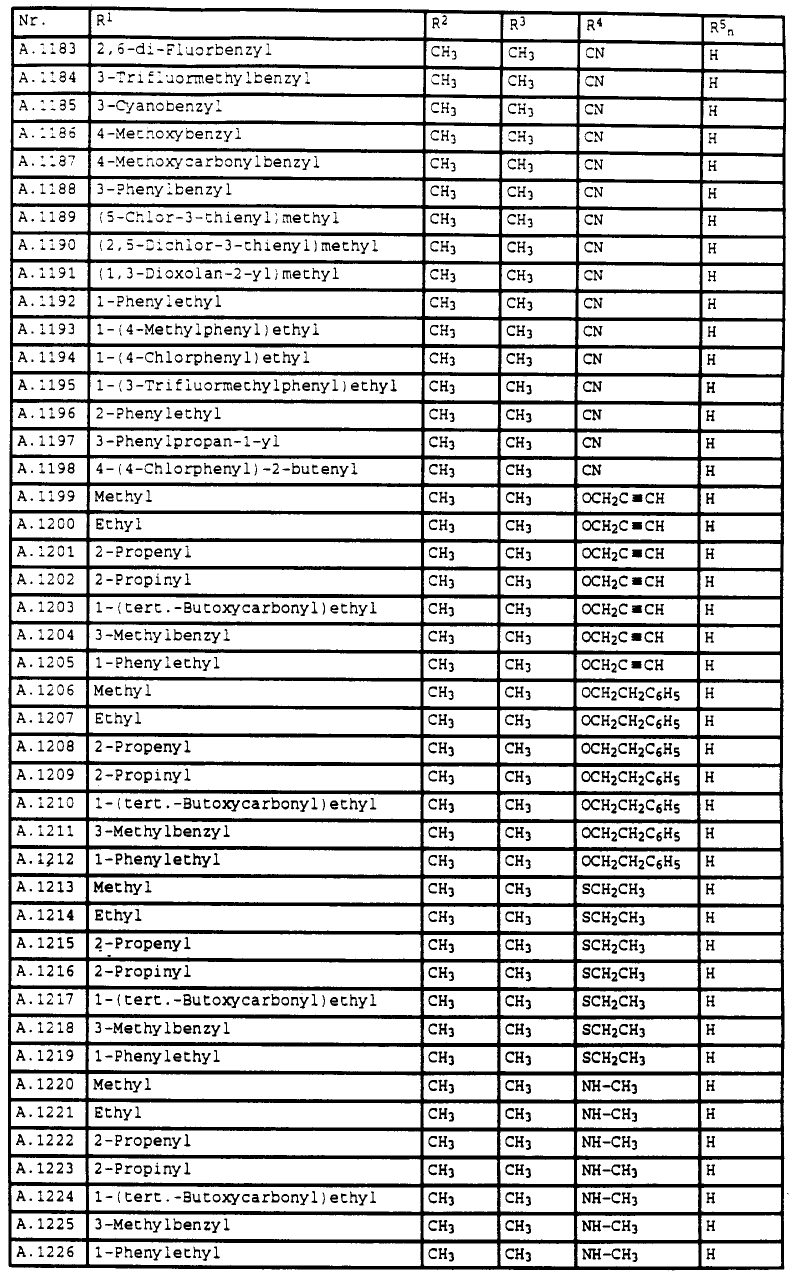

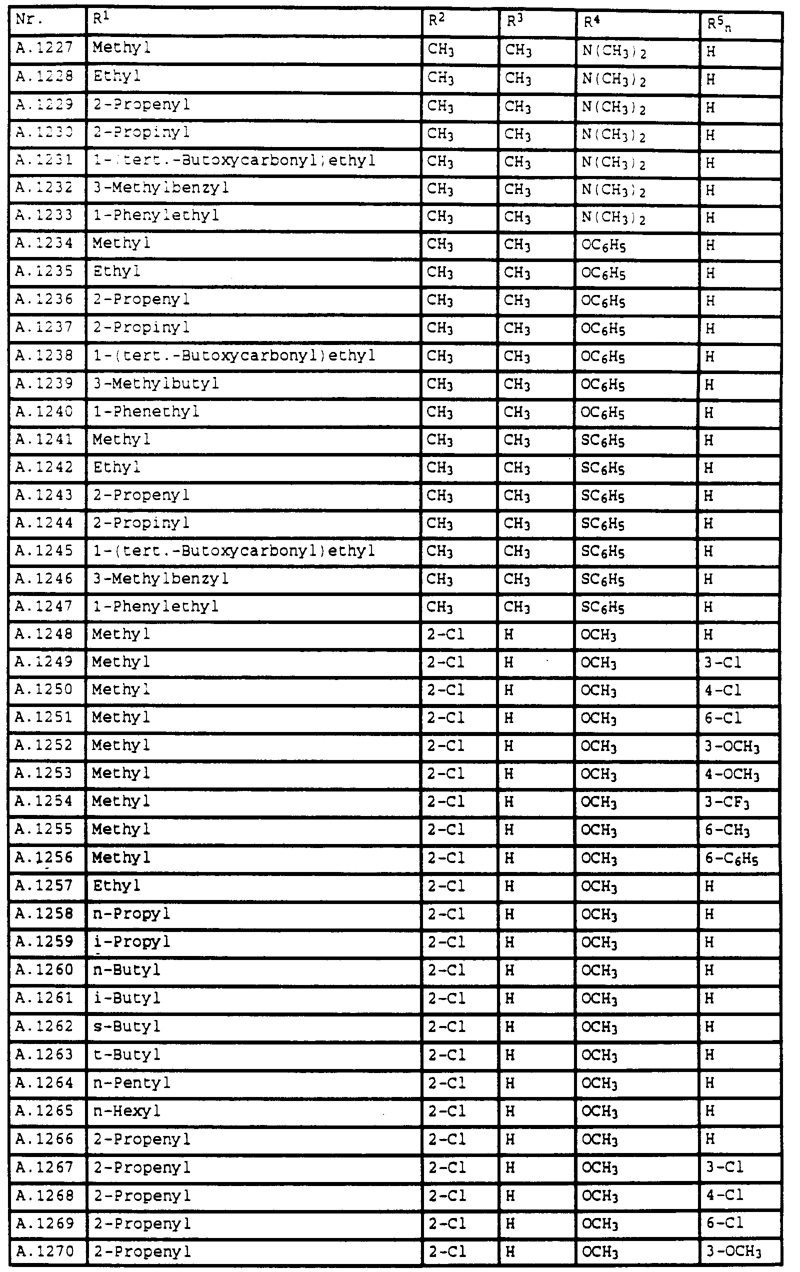

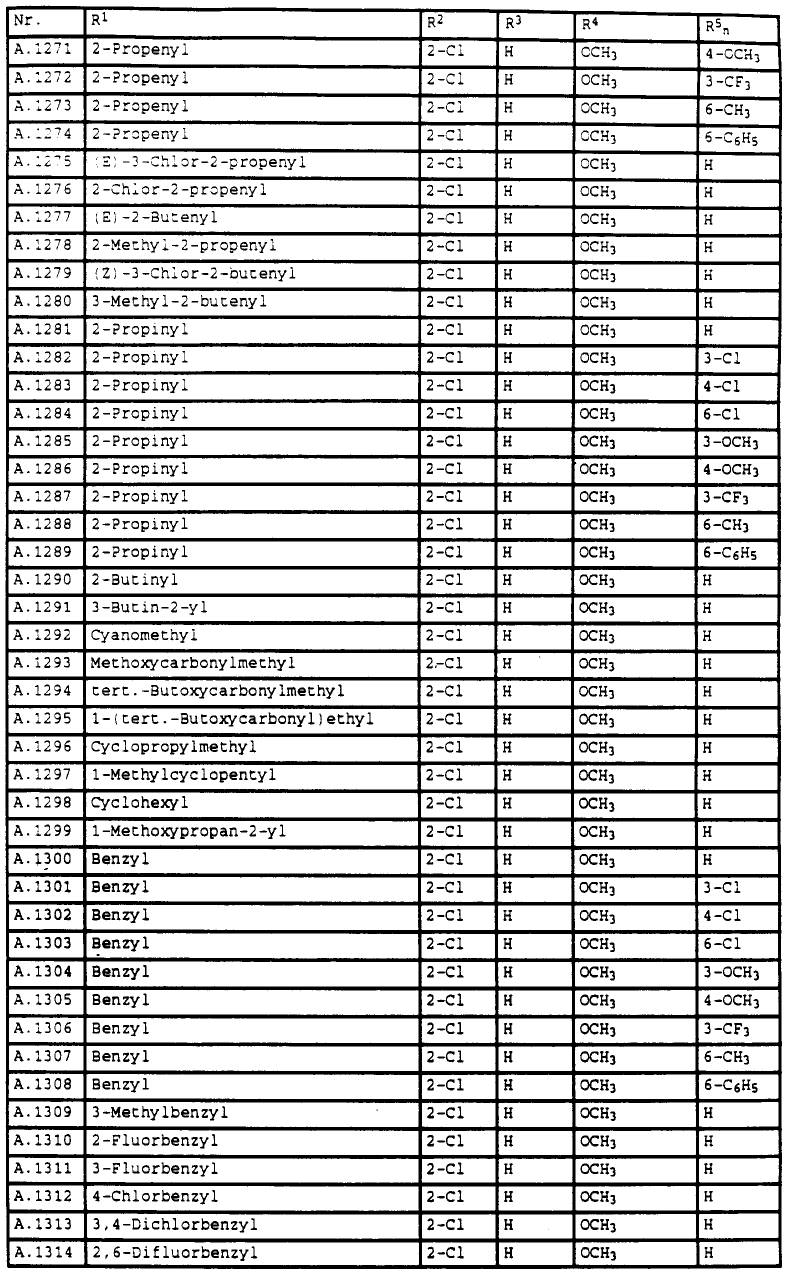

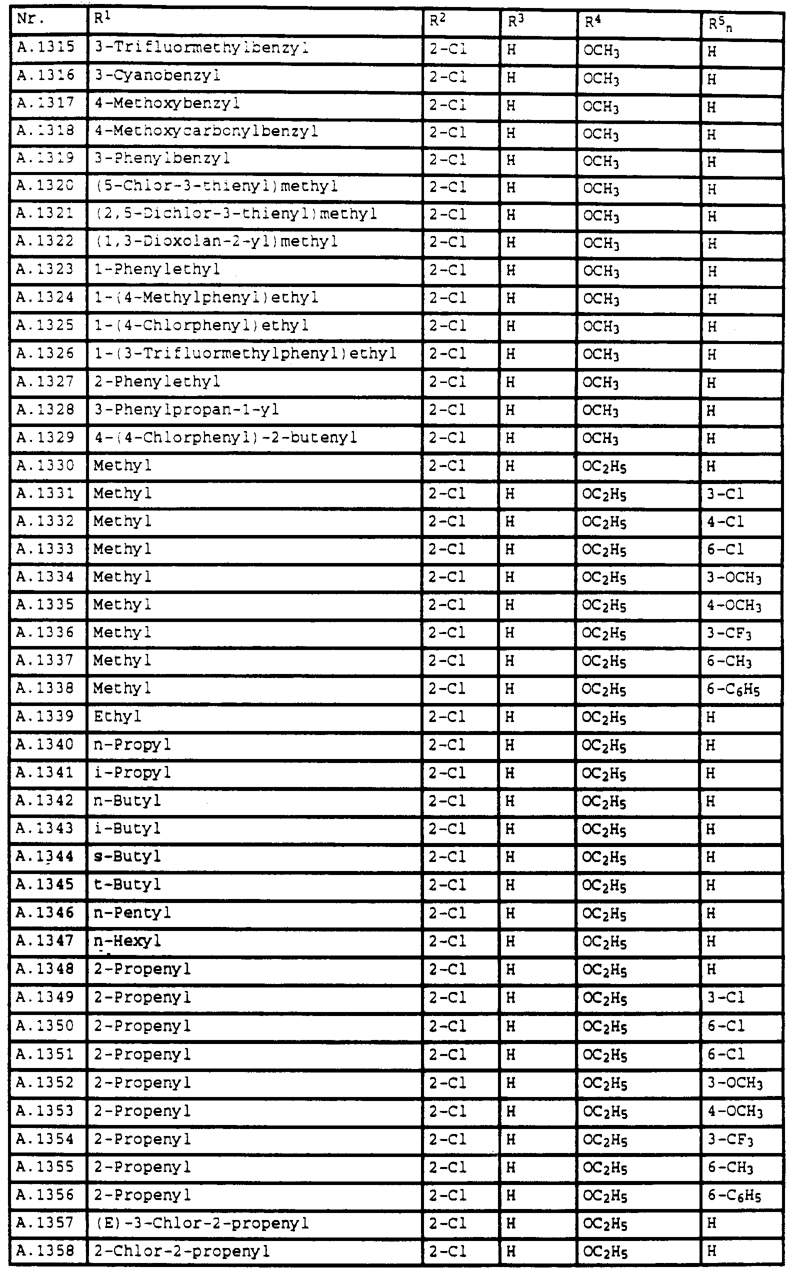

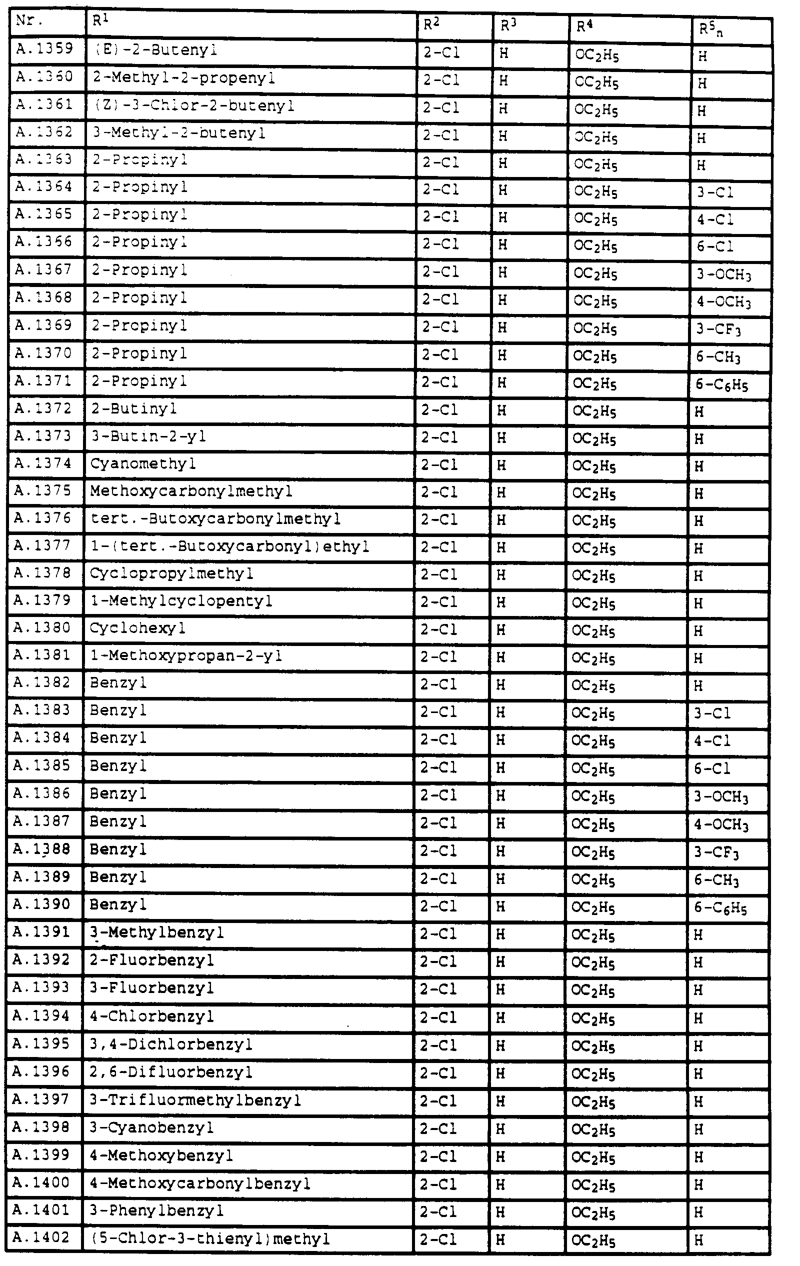

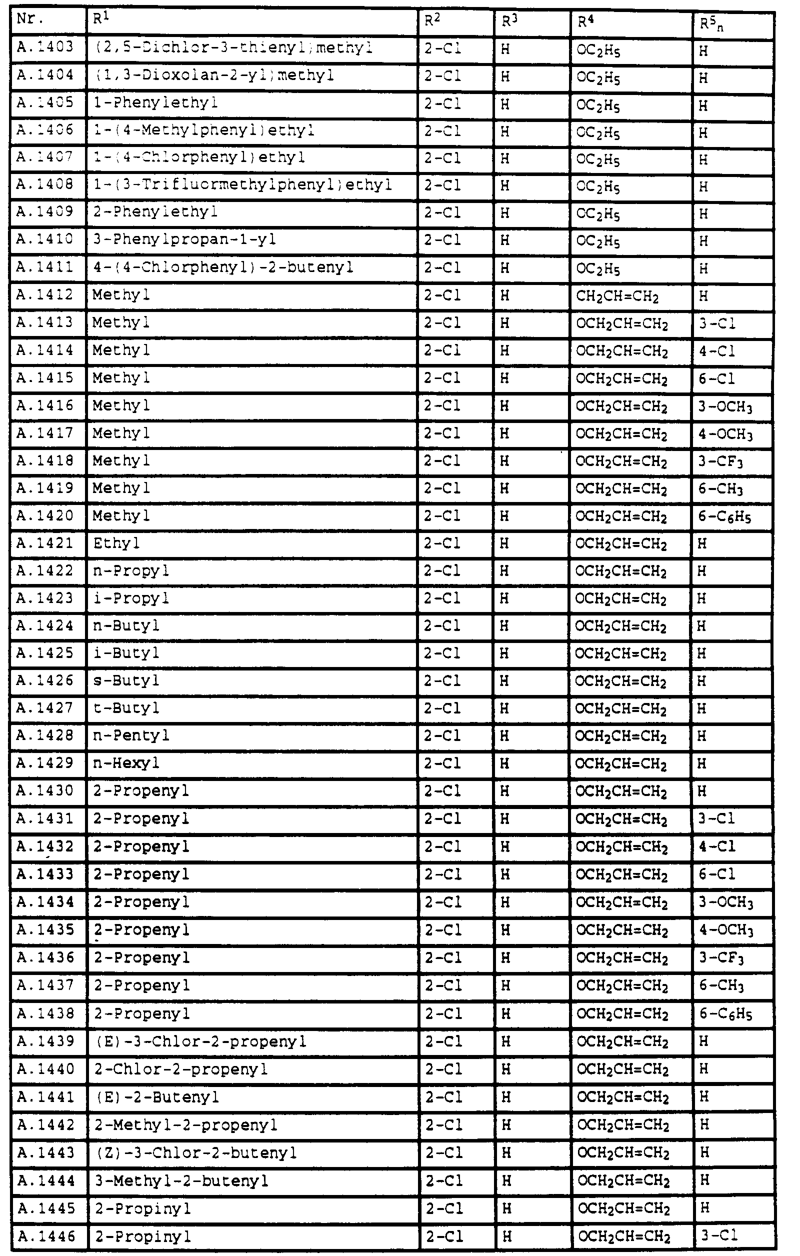

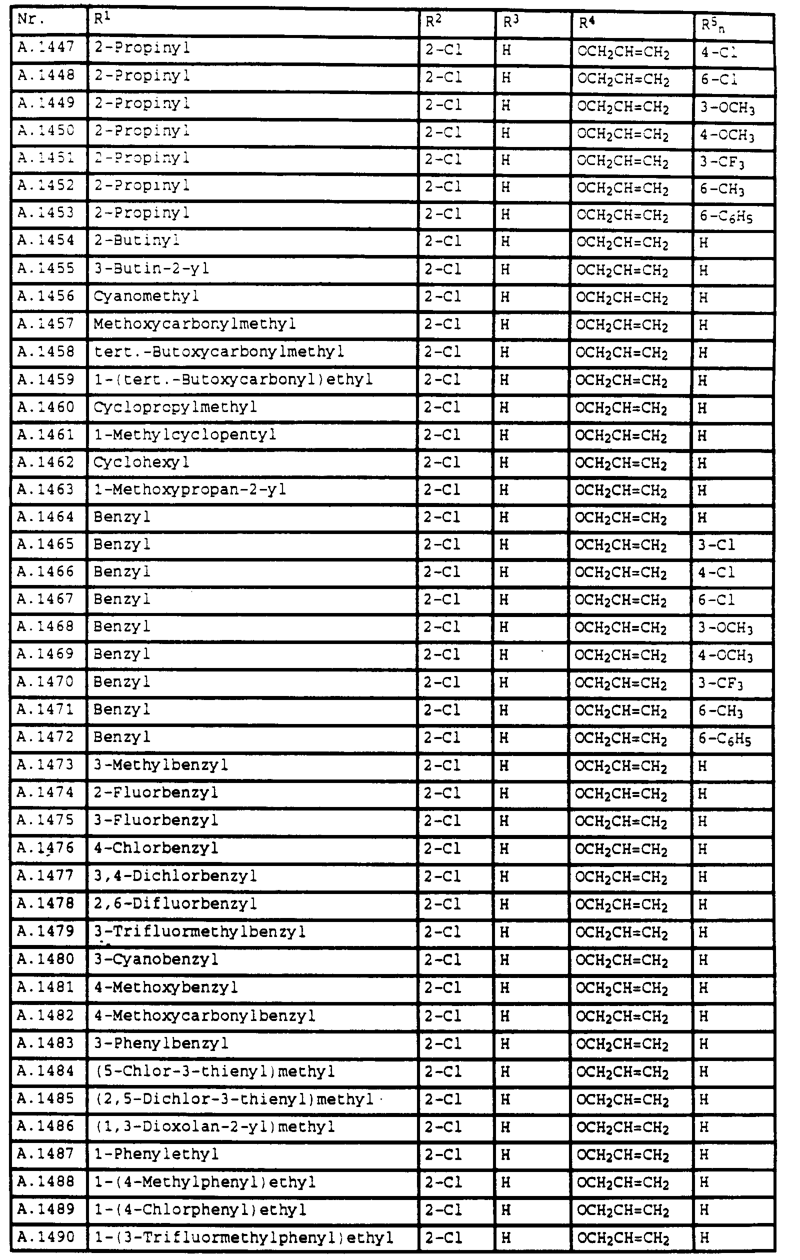

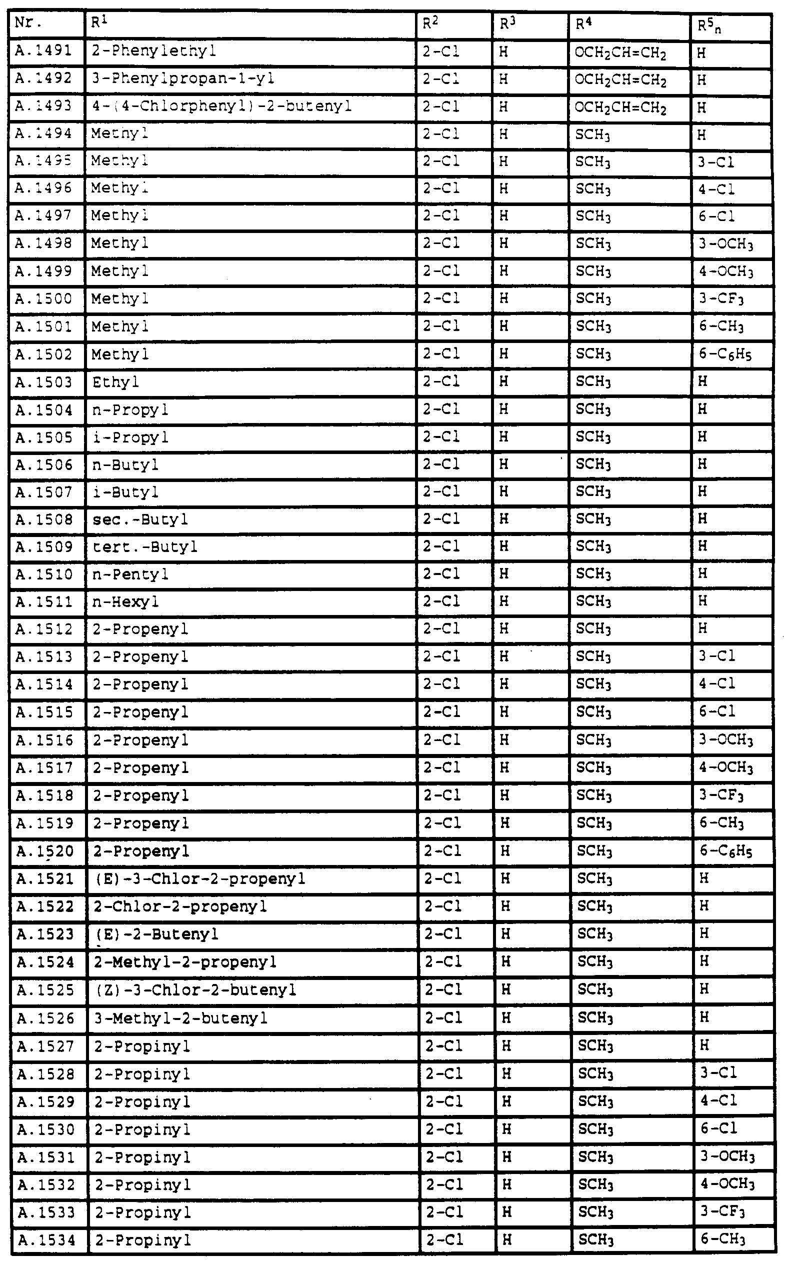

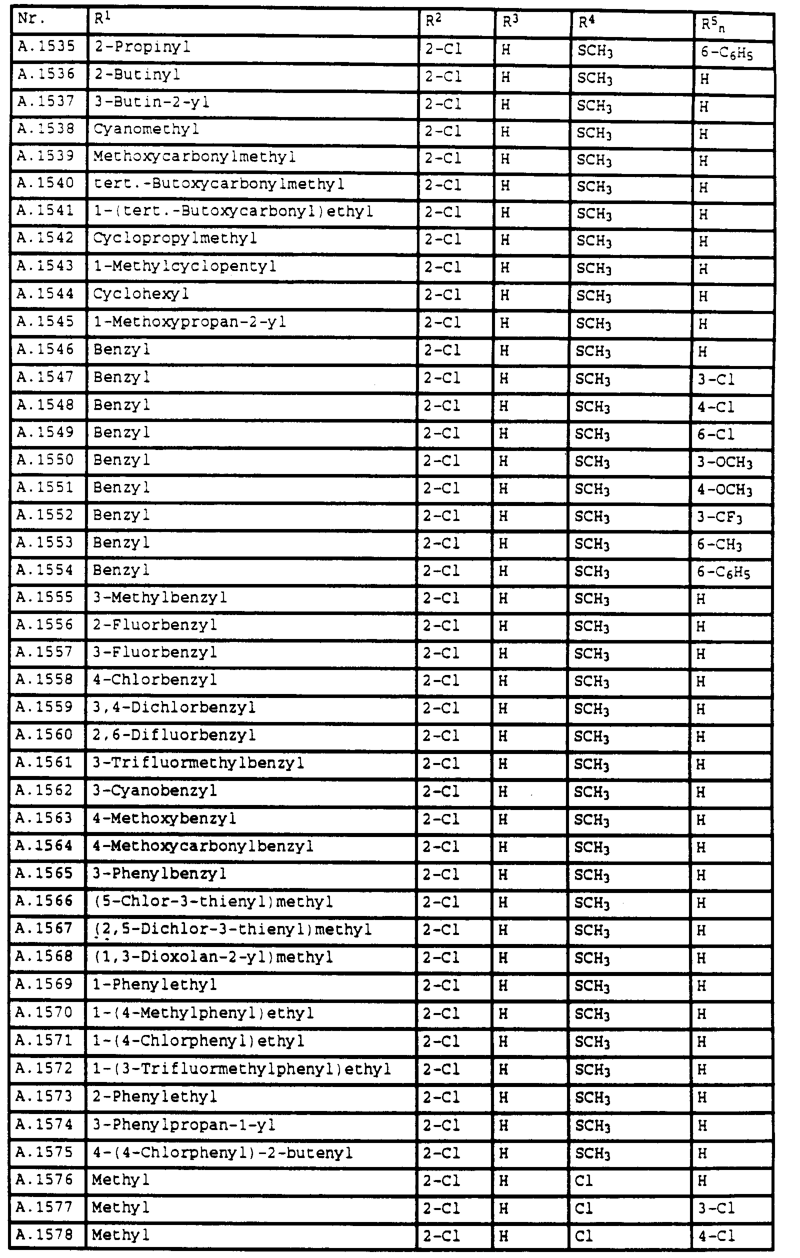

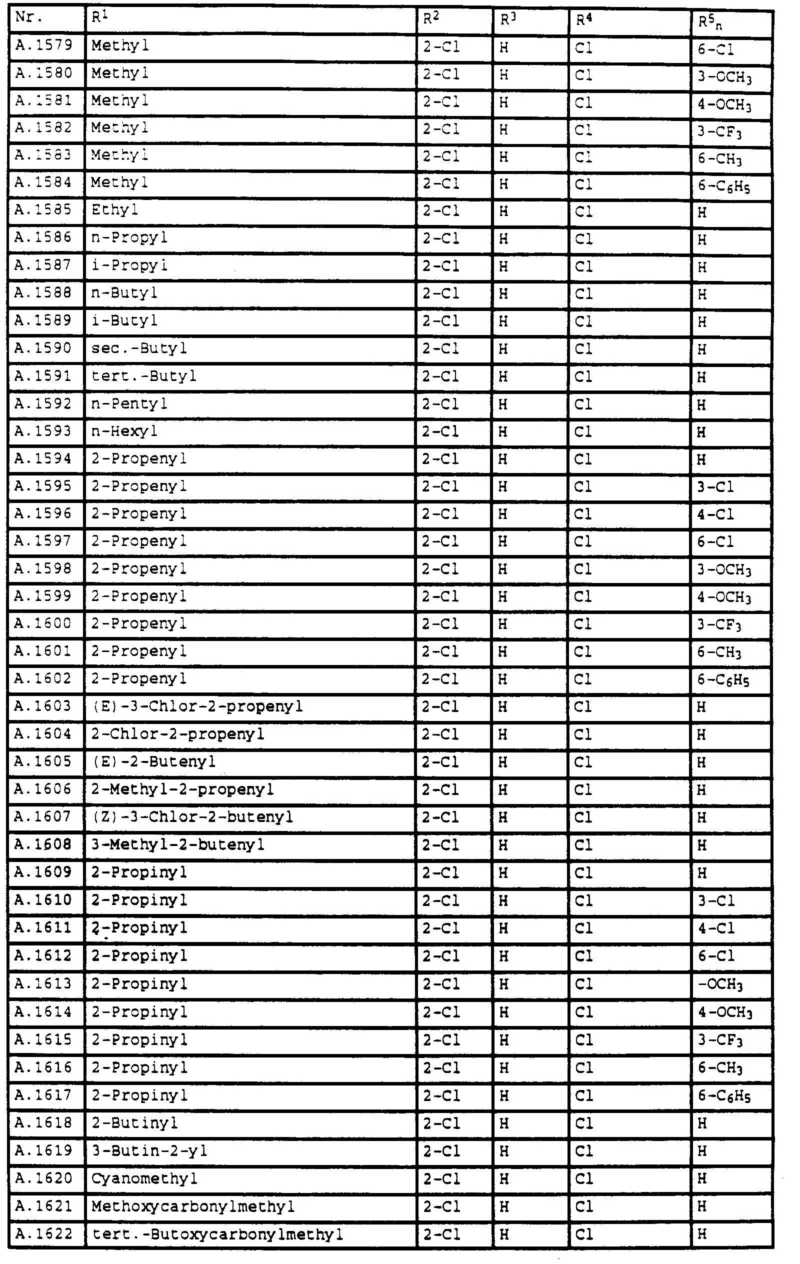

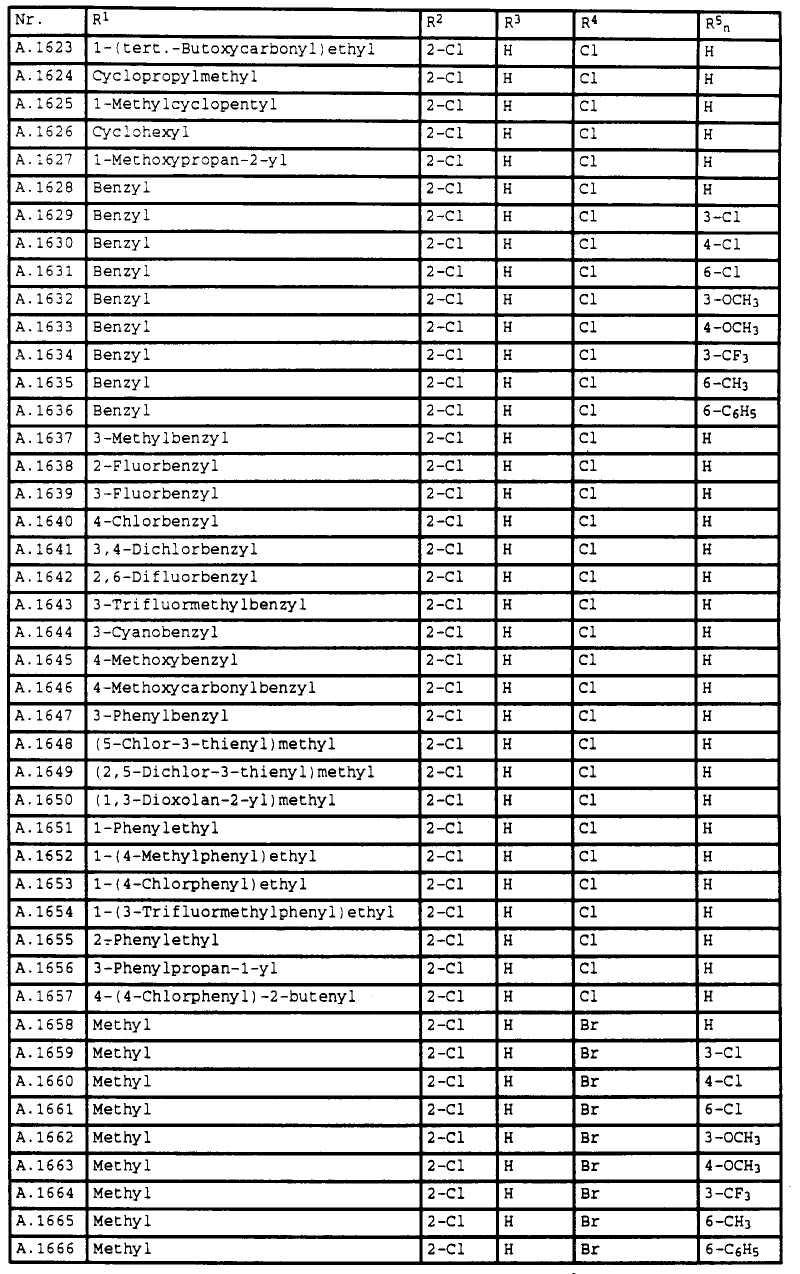

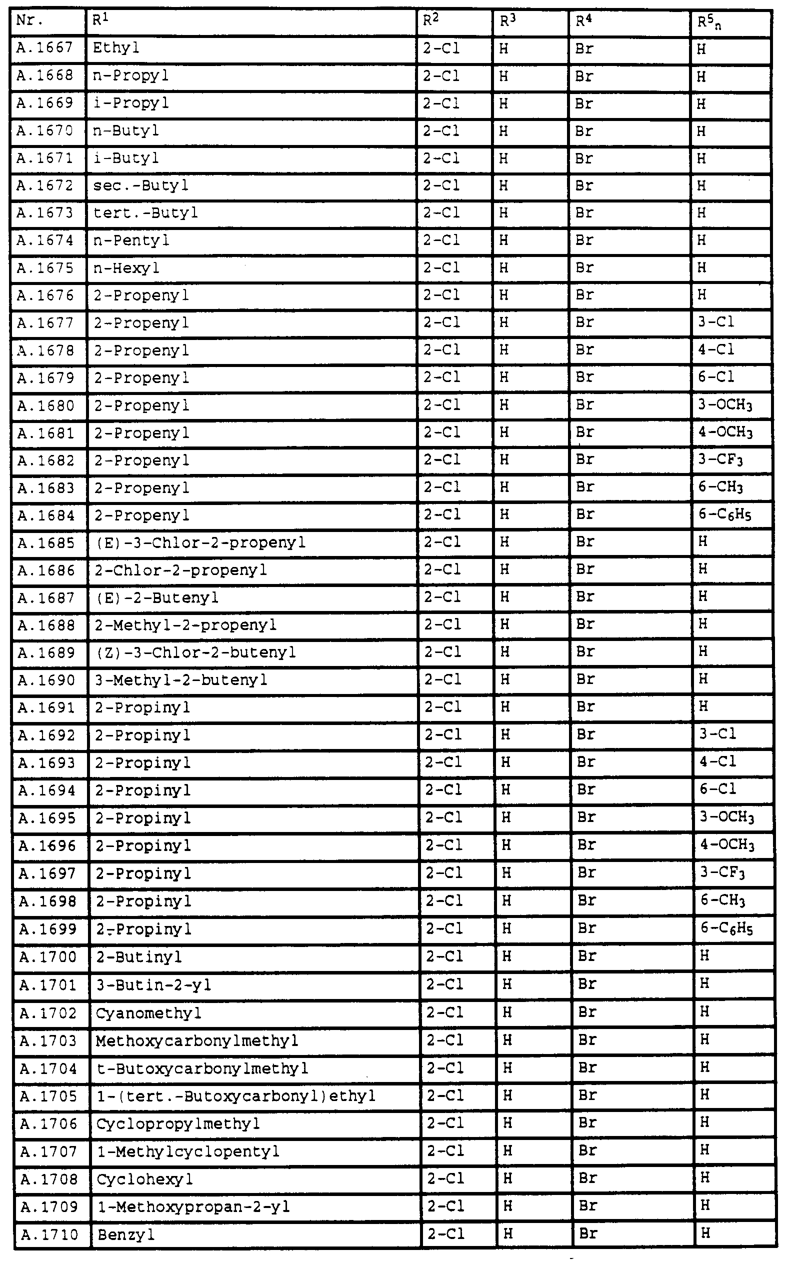

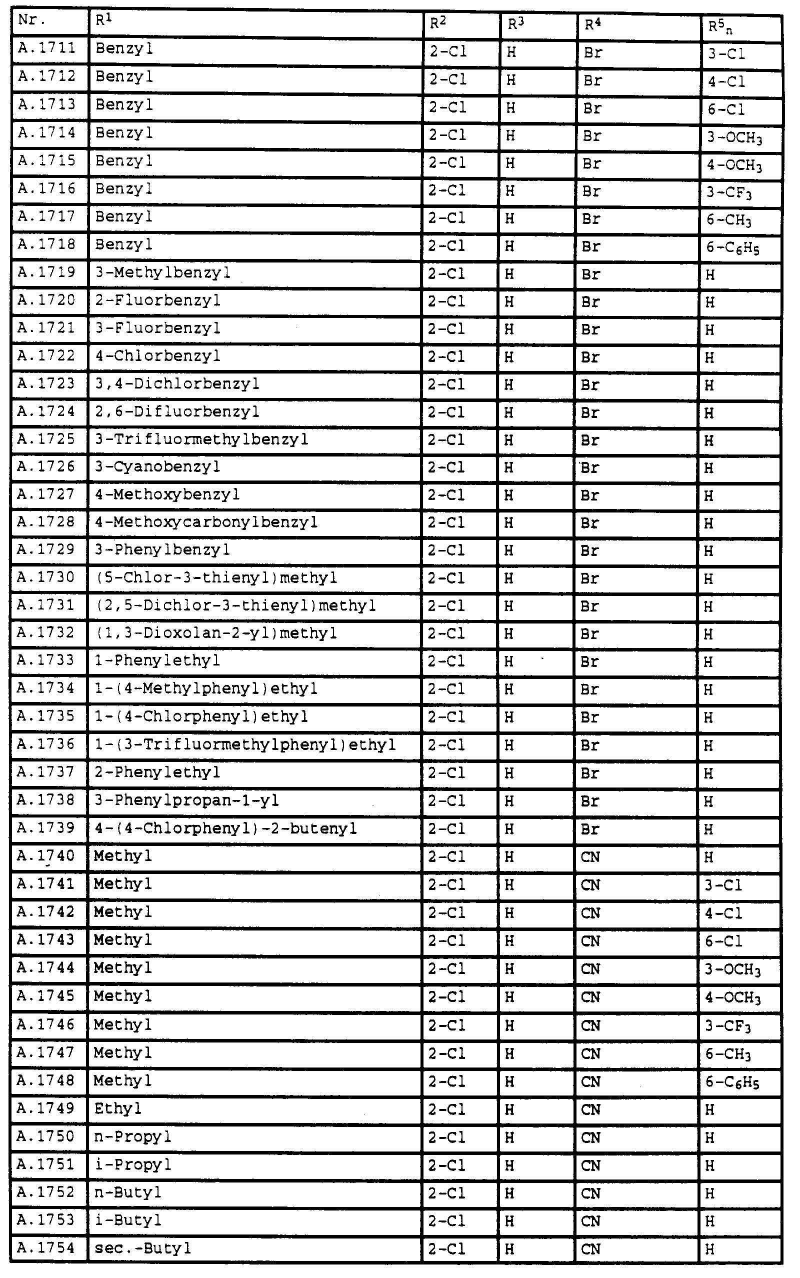

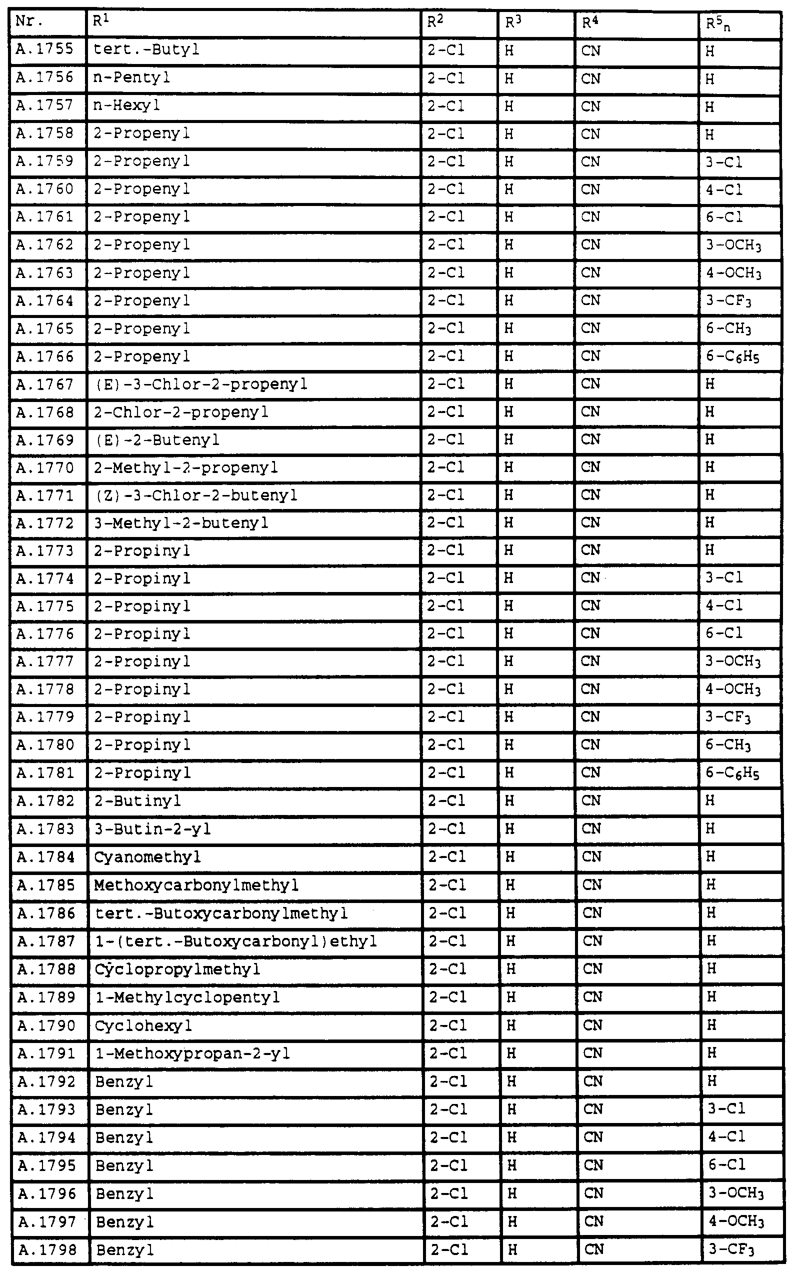

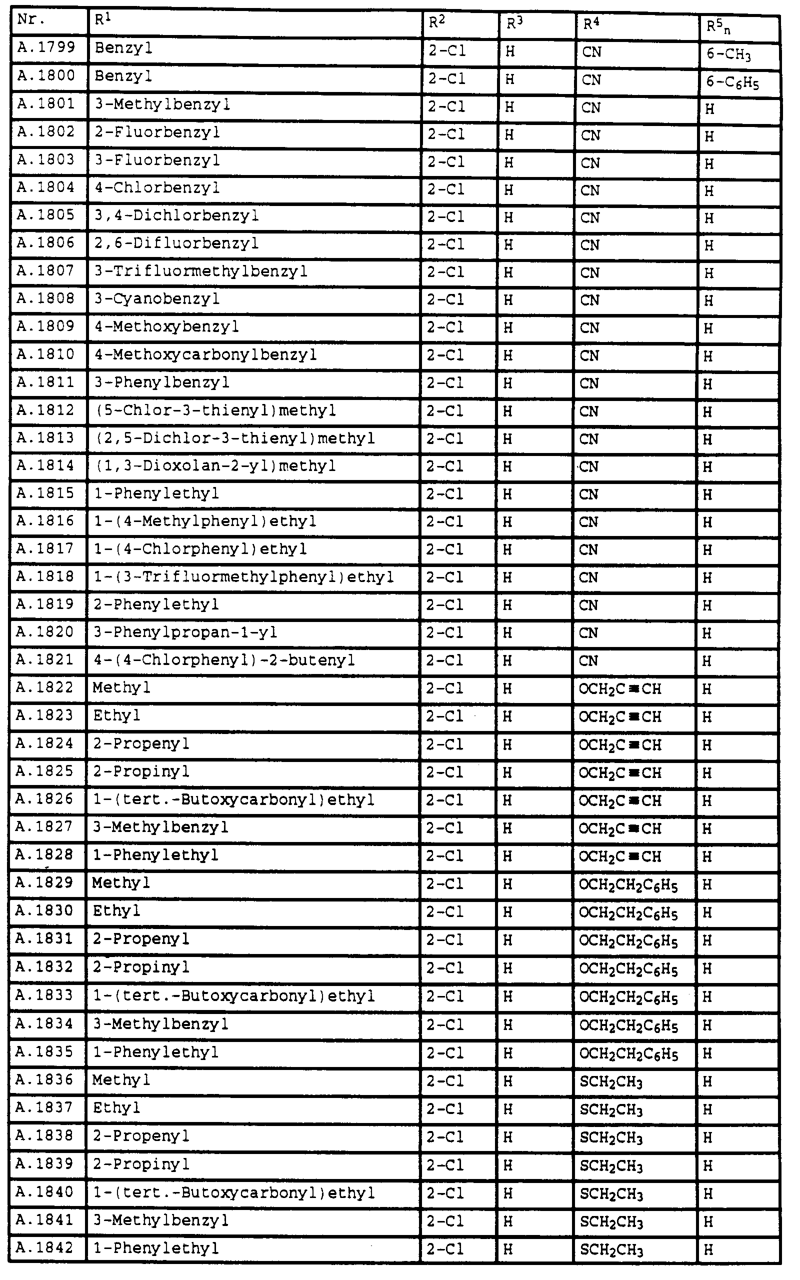

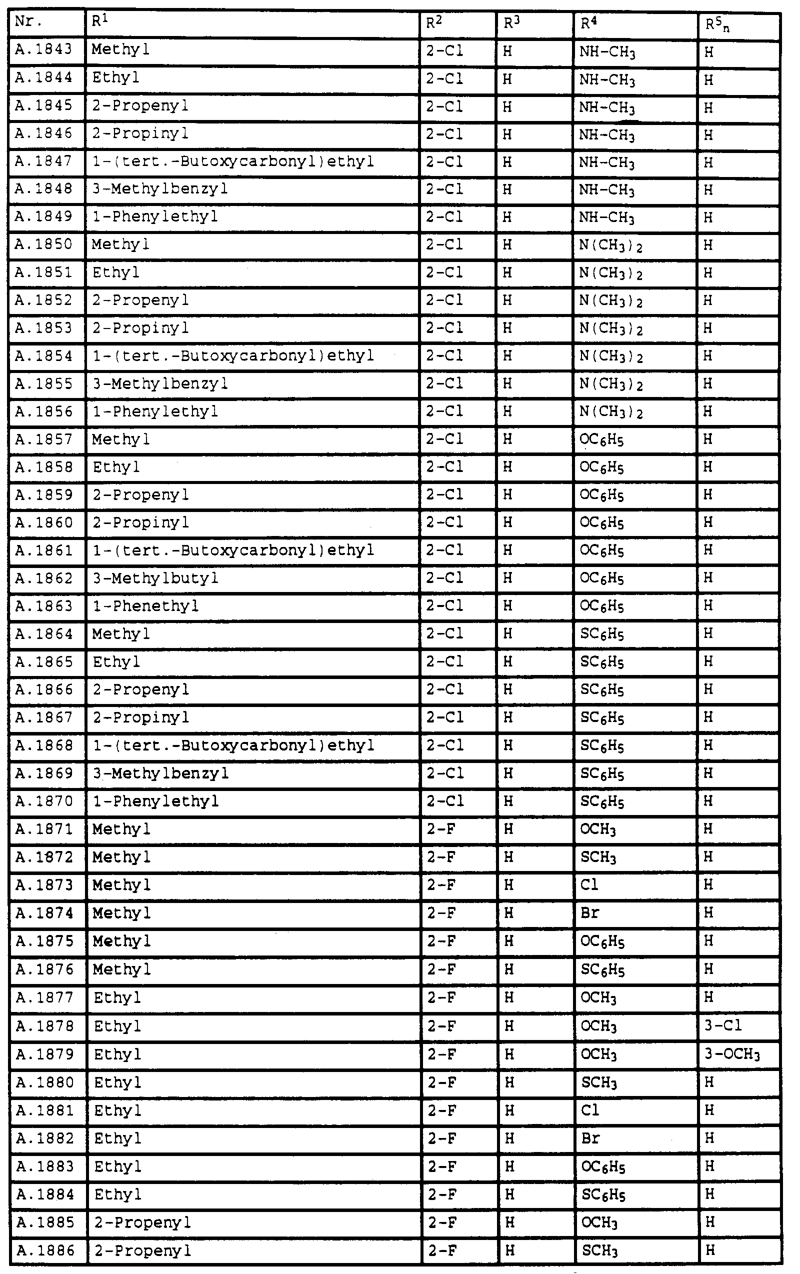

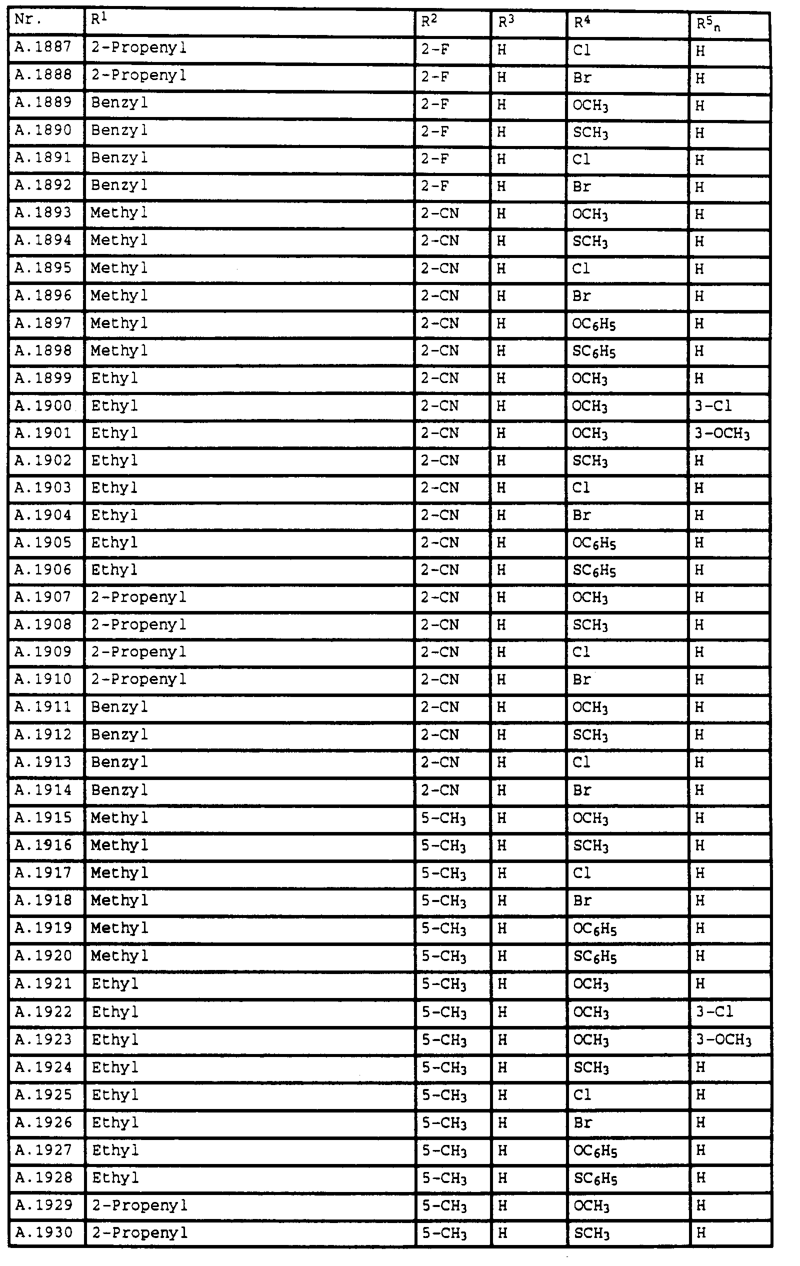

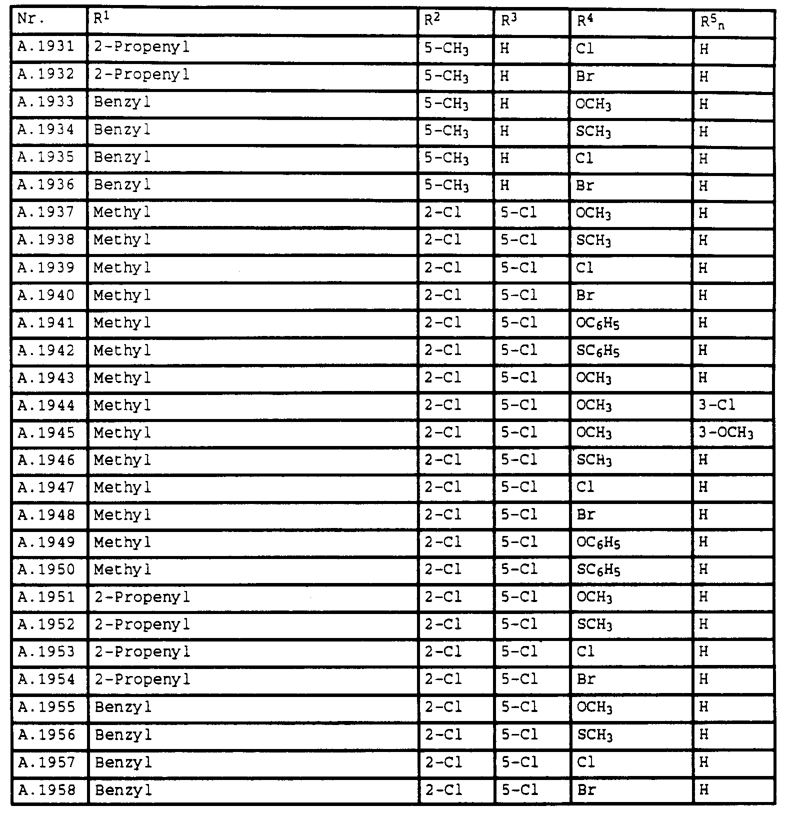

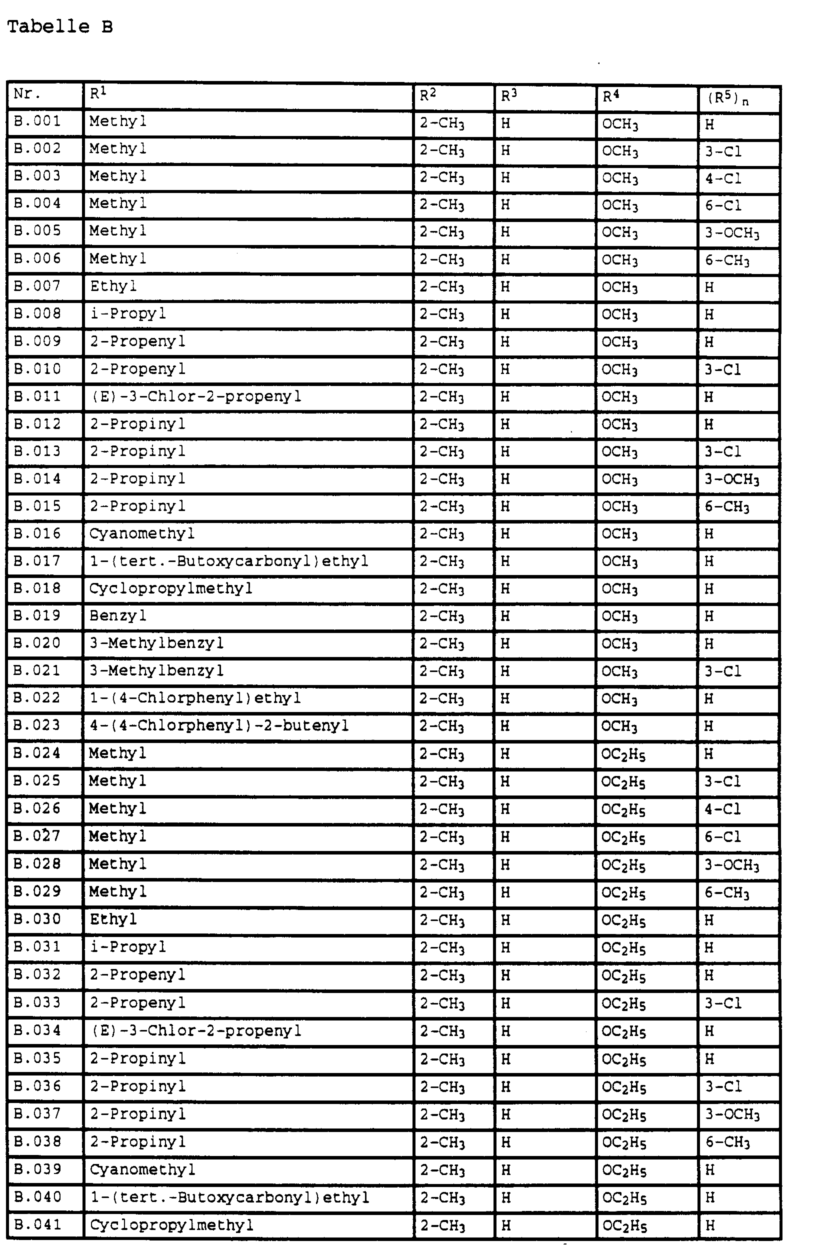

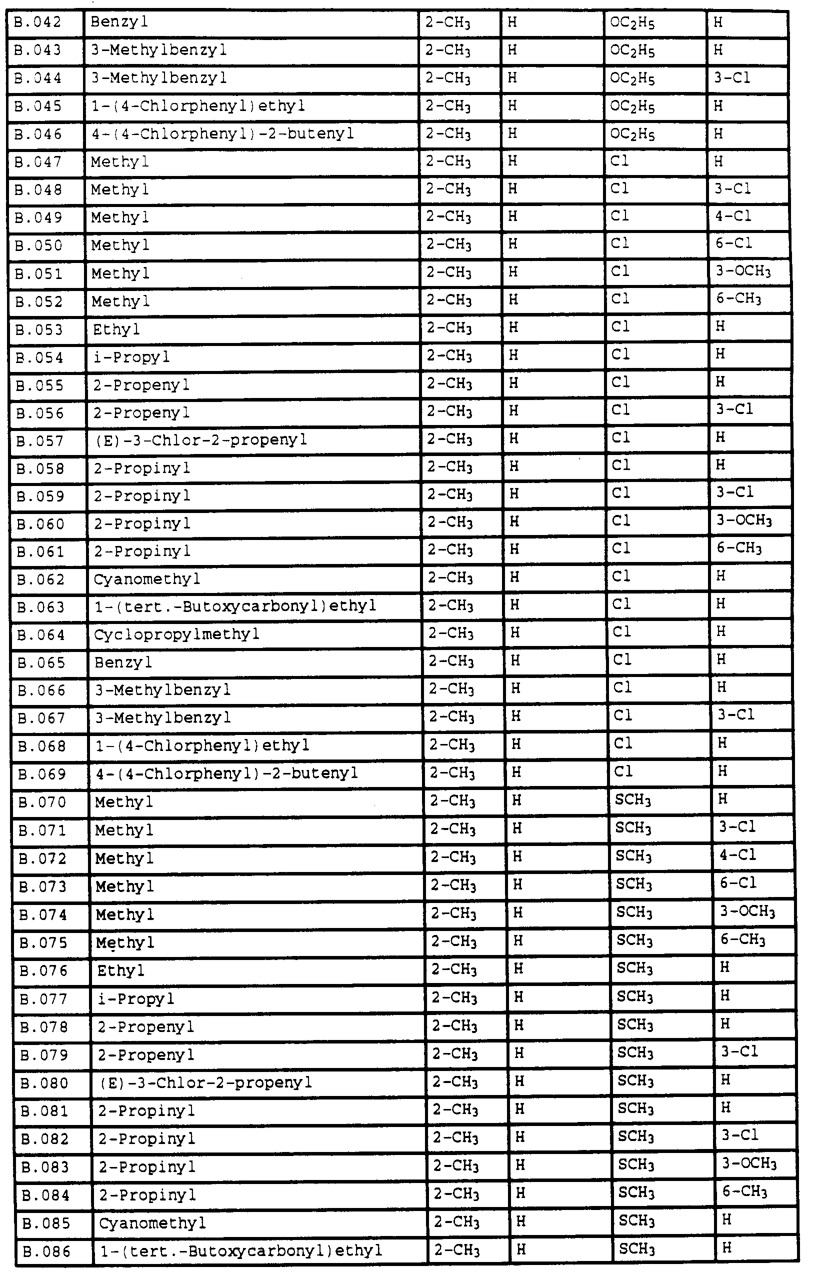

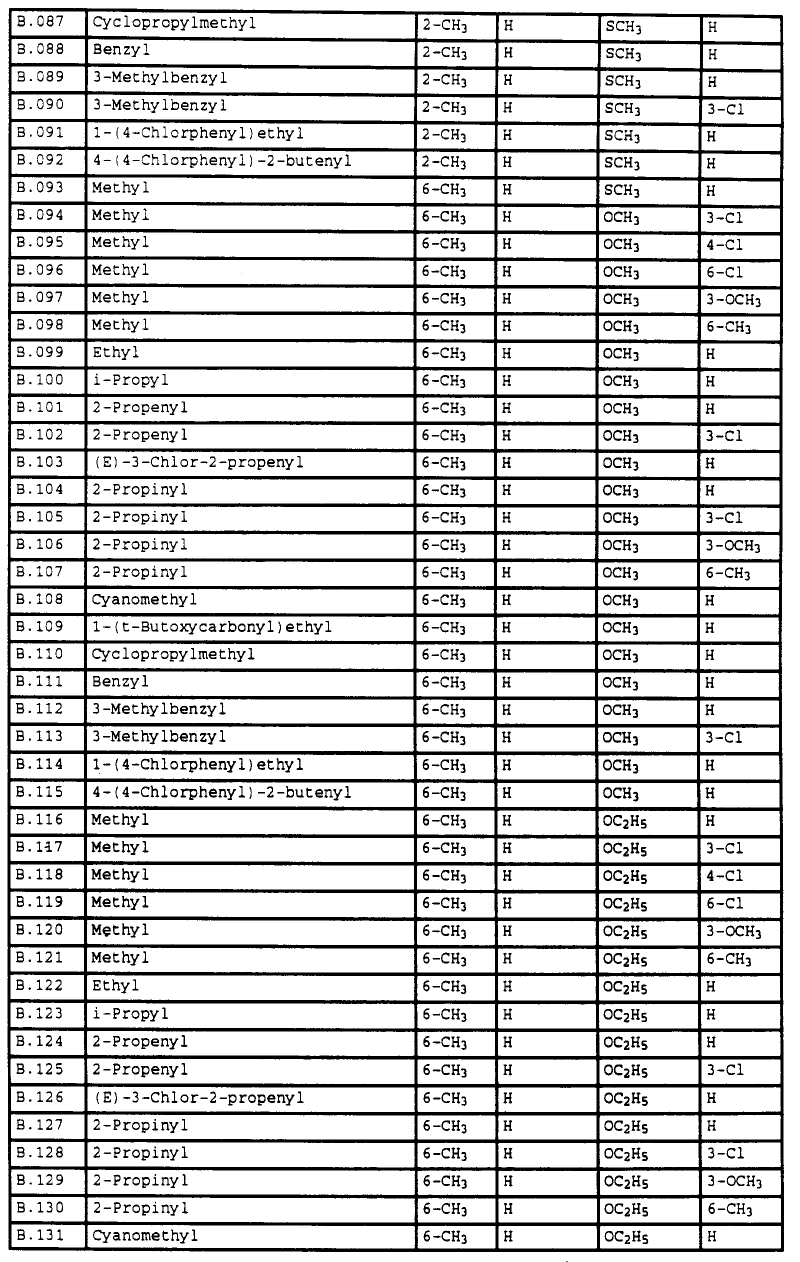

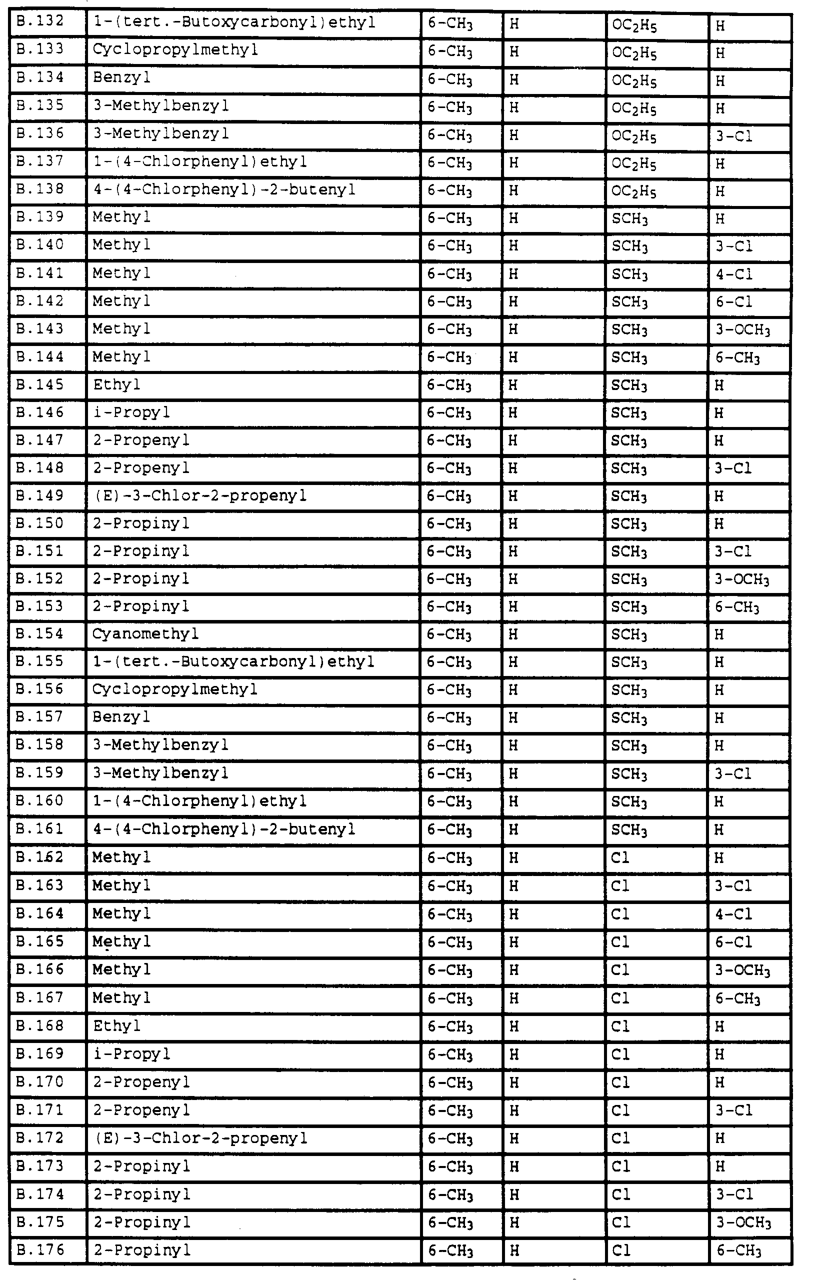

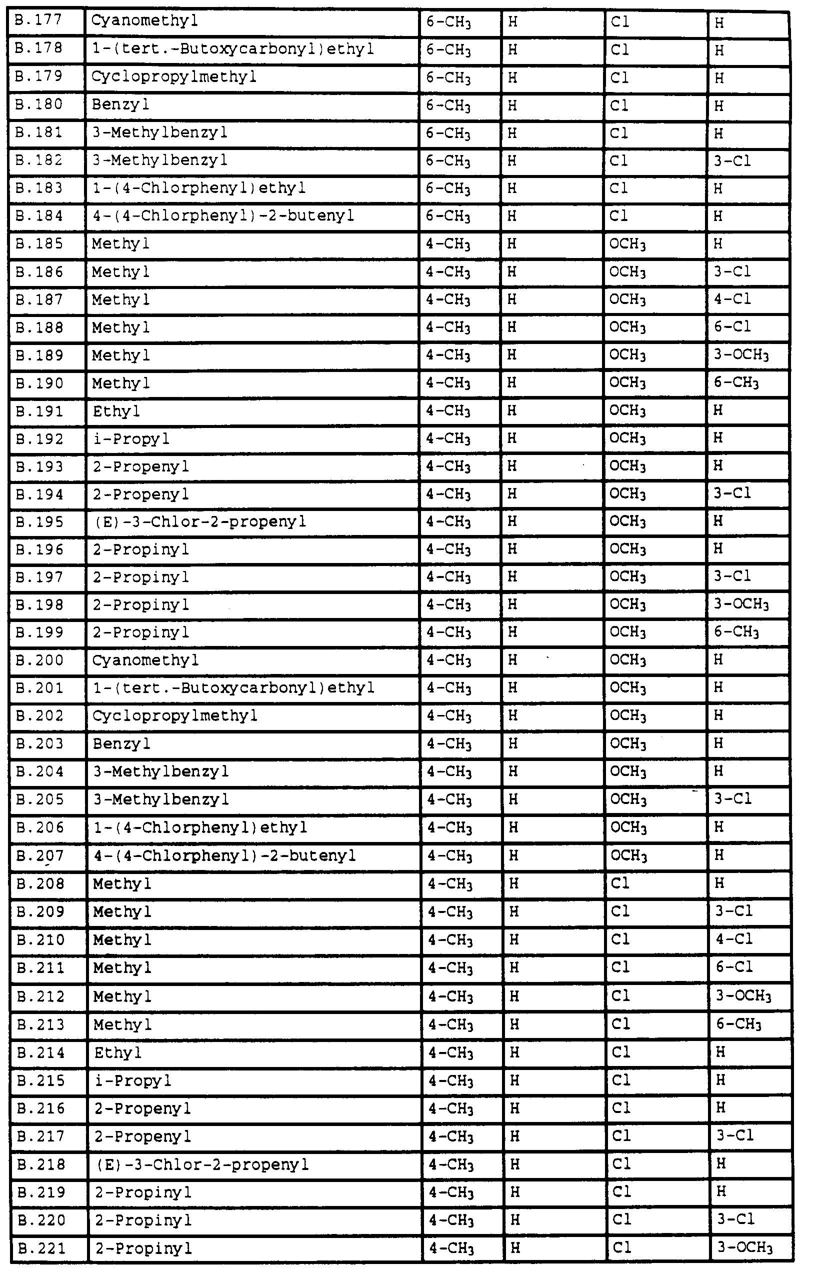

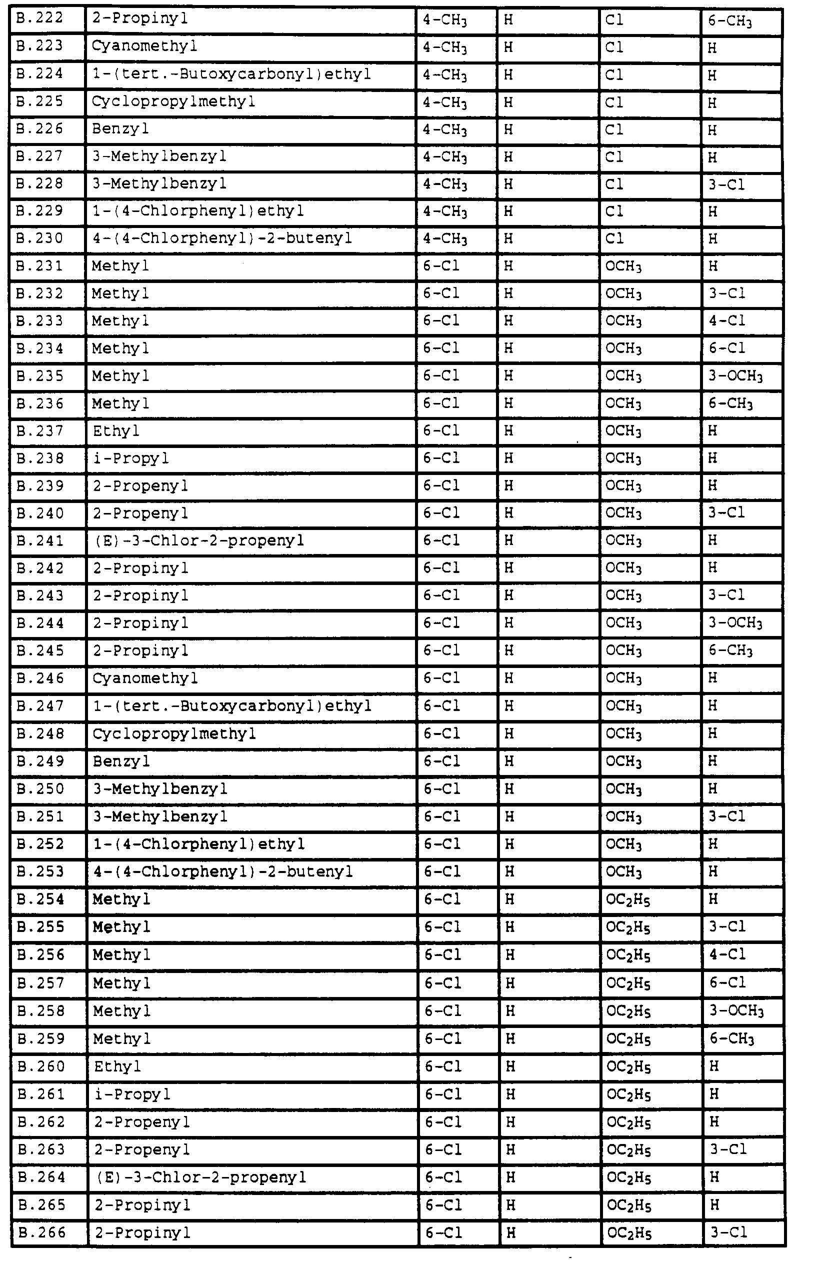

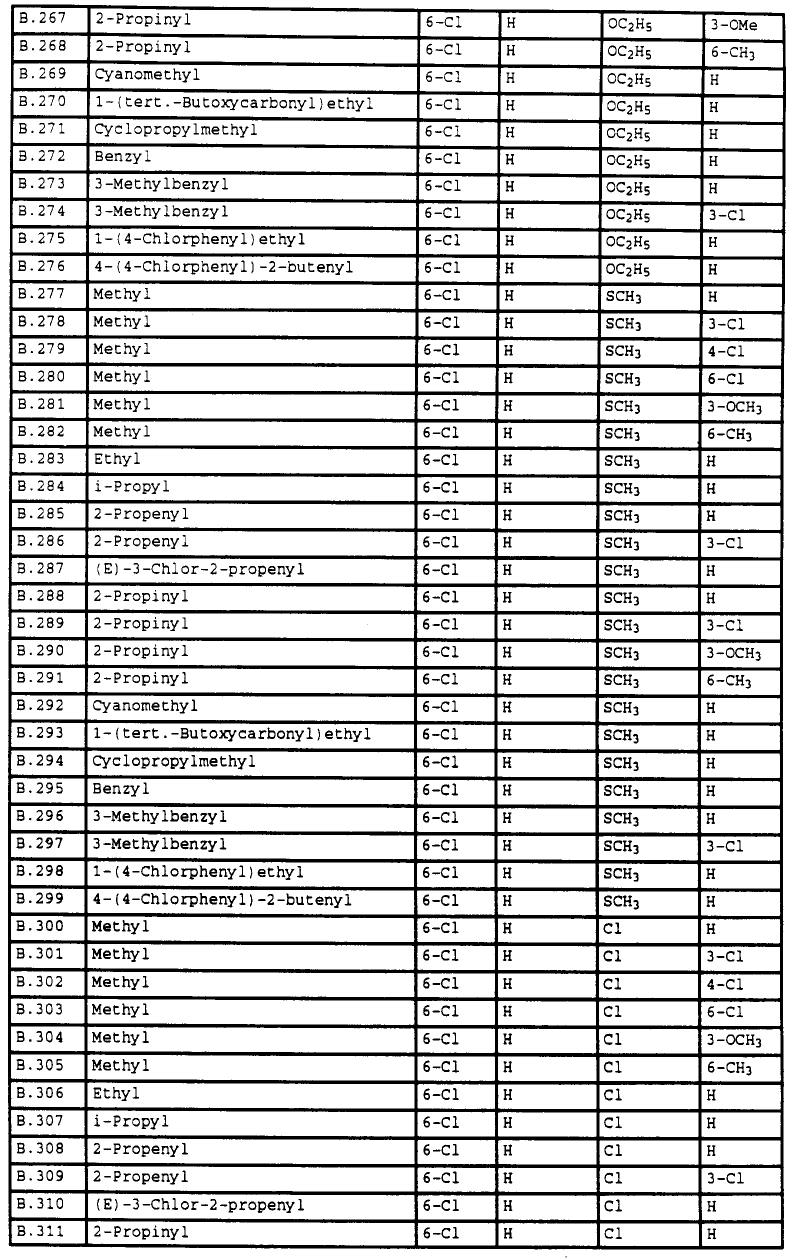

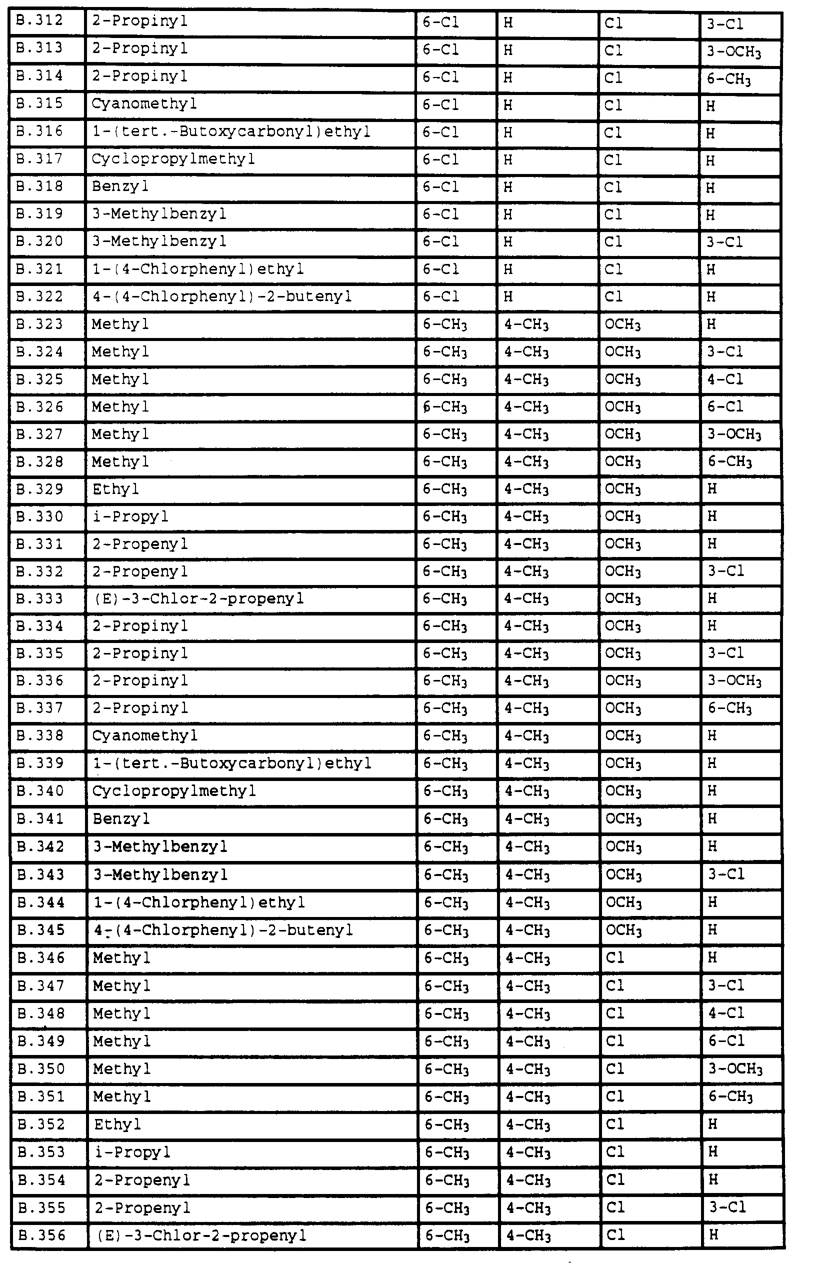

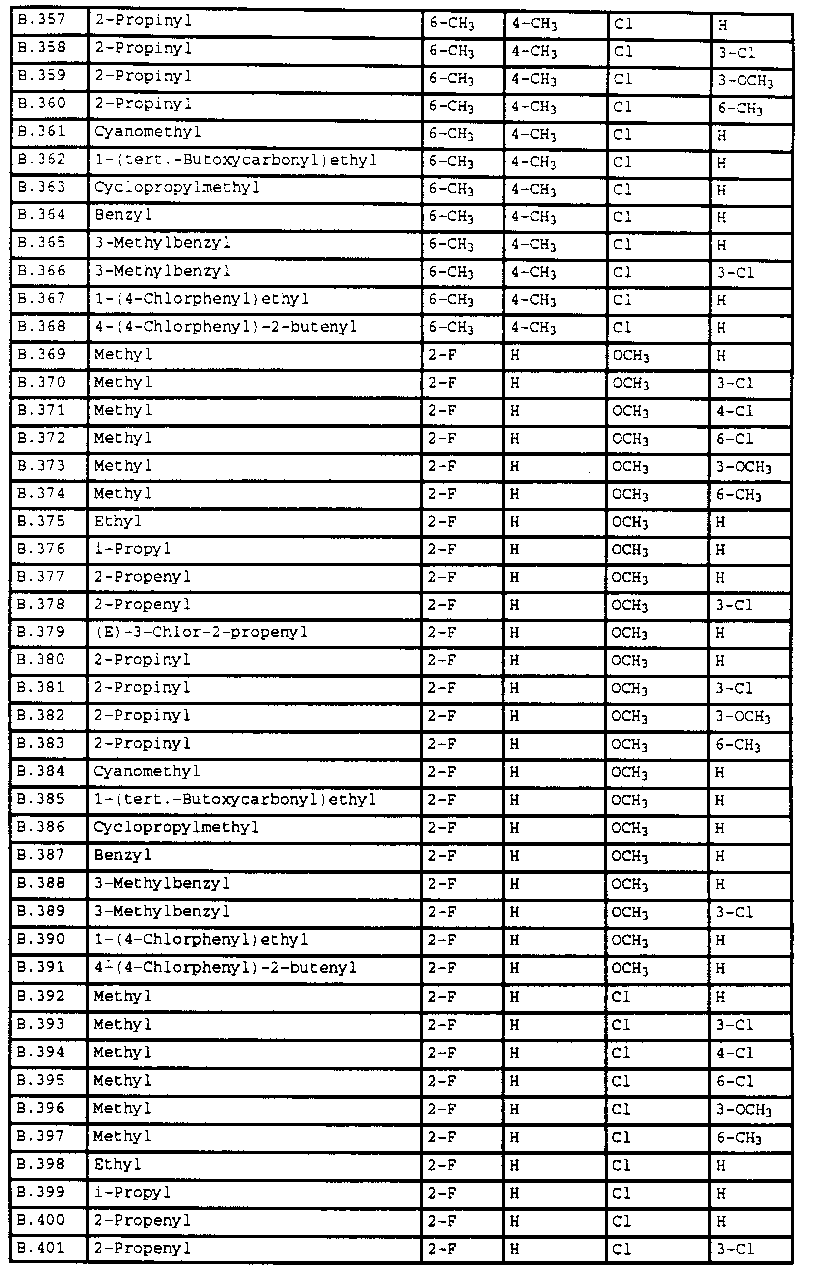

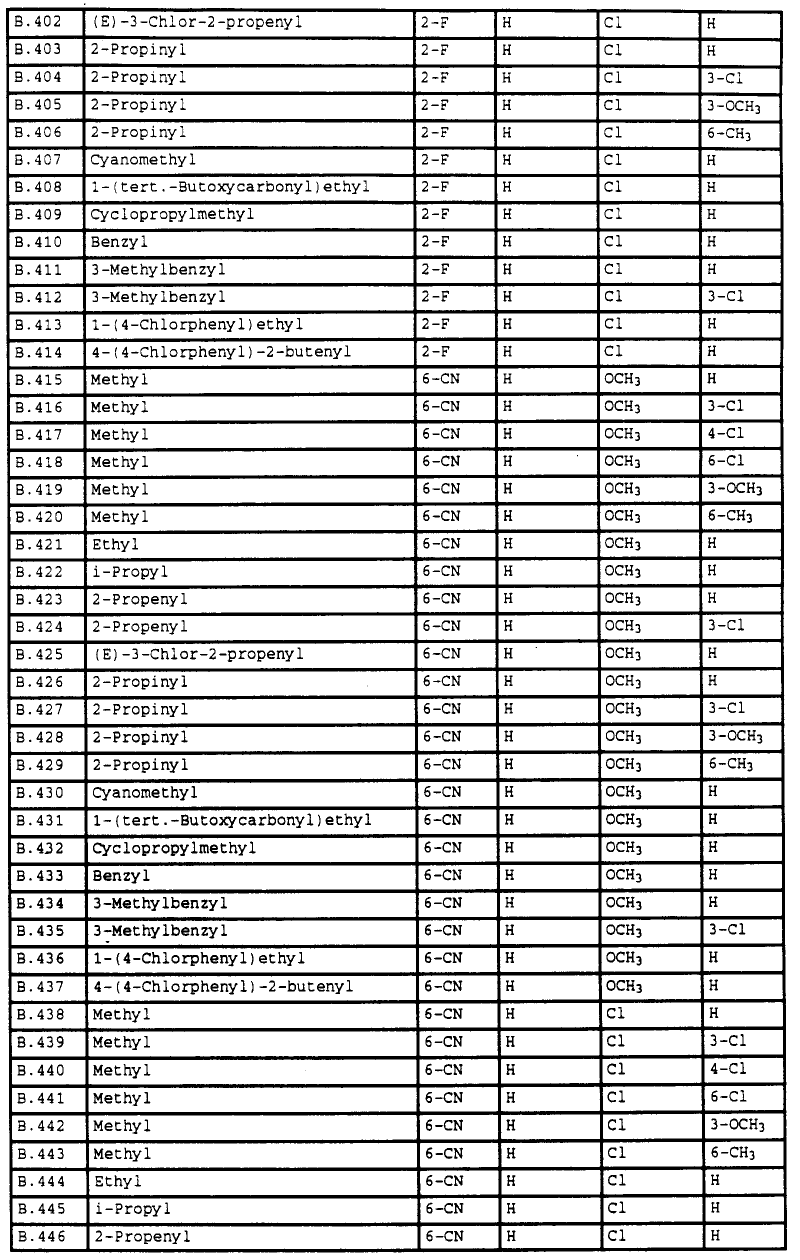

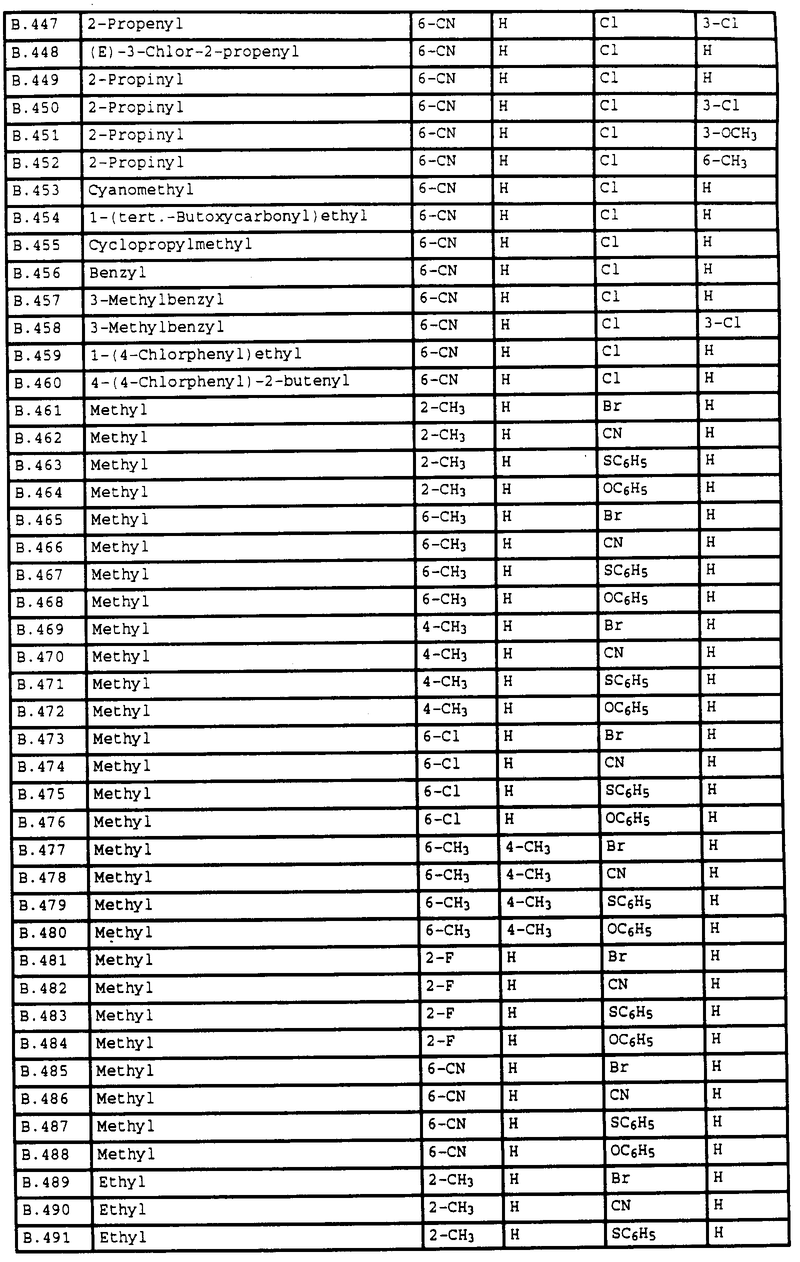

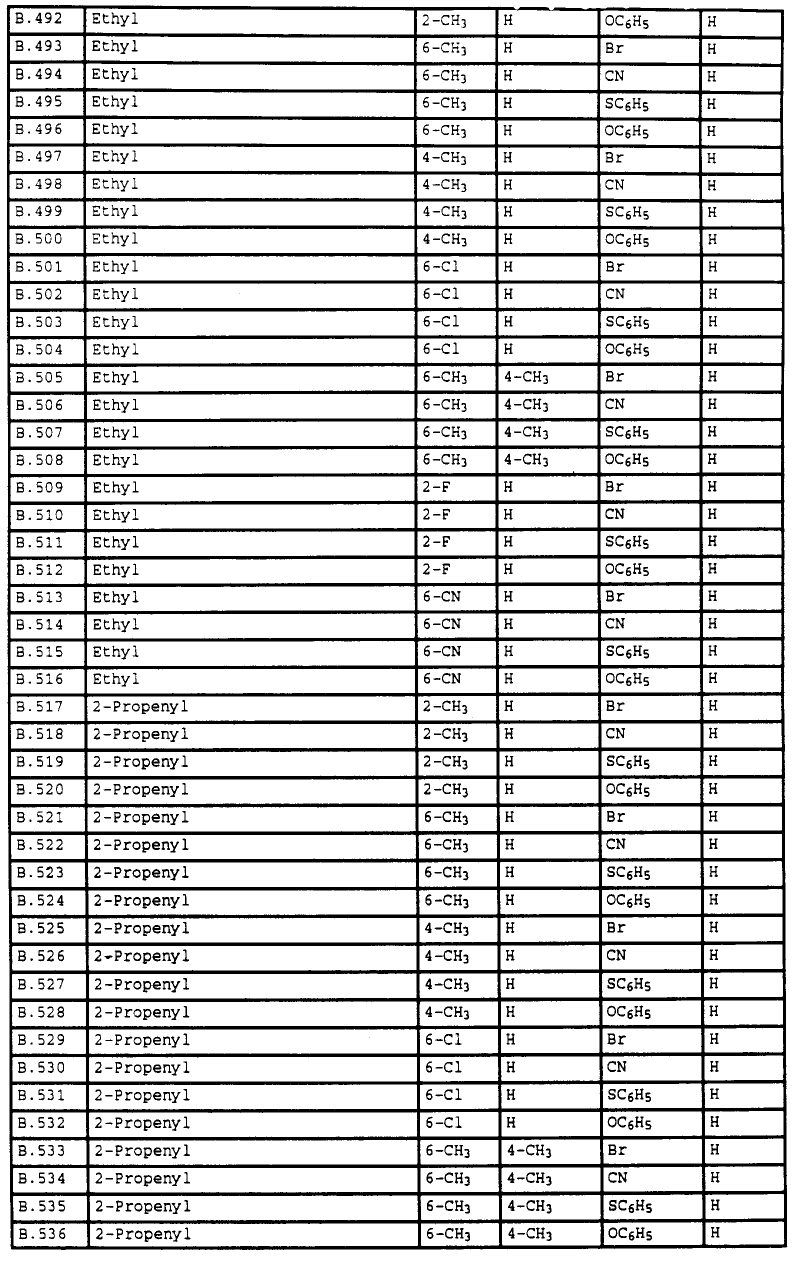

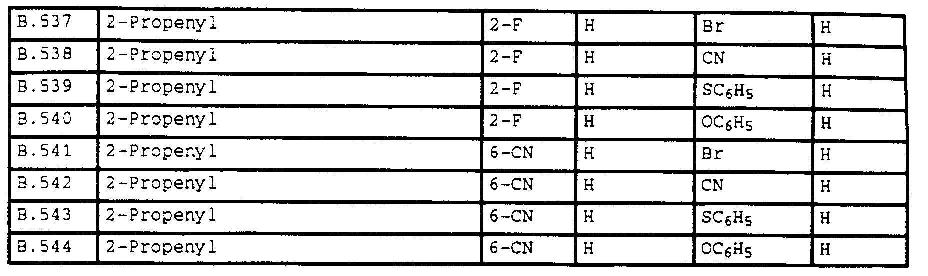

Die neuen substituierten Benzoxyphenyl-Derivate I sind auf verschiedene Weise erhältlich, und zwar vorzugsweise nach einem der folgenden Verfahren:
A) Umsetzung eines Phenols II oder dessen Alkalimetall-, Erdalkalimetall-, Silber- oder Ammoniumsalzes mit Phenylverbindungen III zu substituierten Phenoxymethylphenyl-Derivate I, wobei Y Sauerstoff bedeutet: 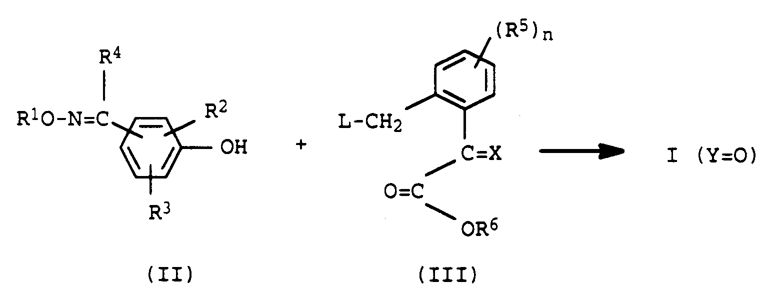
   L bedeutet eine übliche Abgangsgruppe wie Chlor, Brom, Iod, Mesylat, Trifluormethylsulfonat oder Toluolsulfonat.
   Üblicherweise arbeitet man in einerm inerten Lösungs- oder Verdünnungsmittel, z.B. in Aceton, Dimethylformamid oder Toluol, oder in einem Zweiphasensystem aus Wasser und z.B. Dichlormethan unter Verwendung eines Phasentransferkatalysators.
   Geeignete Phasentransferkatalysatoren sind z.B. Ammonium-, Sulfonium- oder Phosphoniumsalze, wobei die Art des Anions von untergeordneter Bedeutung ist. Als zweckmäßig haben sich z.B. Benzyltrimethylammonium-Salze wie Benzyltrimethylammoniumhydroxid und Tetrabutylammonium-Salze erwiesen.
   Normalerweise erfolgt die Umsetzung bei einer Reaktionstemperatur von 0 bis 100°C, insbesondere 20 bis 60°C.
   Die Edukte II und III werden normalerweise in etwa äquimolaren Mengen miteinander umgesetzt. Im Hinblick auf die Ausbeute kann es jedoch vorteilhaft sein, III in einem Überschuß von etwa 1 bis 20 mol-%, vorzugsweise 1 bis 10 mol-%, bezogen auf II einzusetzen.
   Im allgemeinen ist eine katalytische Menge an Phasentransferkatalysator, etwa zwischen 1 und 10 Mol%, bezogen auf II oder III, ausreichend.
   Die Phenylverbindungen III, in denen X für =CH-OCH₃ oder =N-OCH₃ steht, sind aus der EP-A 386 561, die Phenylverbindungen III, in denen X für =CH-CH₃ steht, aus der EP-A 513 580 bekannt. Die beiden Europäischen Anmeldungen offenbaren auch Verfahren, nach denen sich die Benzoxyphenyl-Derivate I mit Y = Sauerstoff herstellen lassen.
   Aus den Benzoxyphenyl-Derivaten I mit Y = Sauerstoff und R⁶ = C₁-C₄-Alkyl lassen sich die entprechenden Verbindungen I mit R⁶ = Wasserstoff nach an sich bekannten Verfahren der Esterspaltung herstellen (vgl. z.B. Houben Weyl, Methoden der Organischen Chemie, Bd. E 5, S, 223-254; Org. Reactions 24, (1976), S. 187-224).
B) Alkylierung von Hydroximsäure(thio-)estern IVa oder von Hydroxamsäureestern IVb 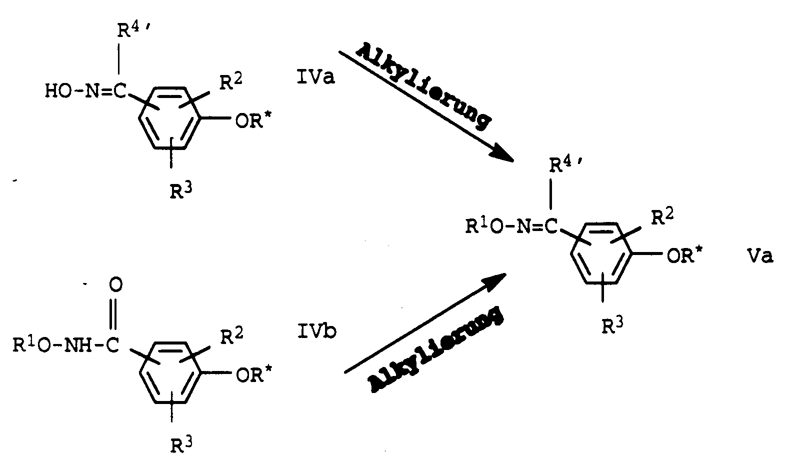 R⁴' bedeutet Alkoxy;
   R* steht für Wasserstoff, eine Phenolschutzgruppe wie Methoxymethyl, Allyl, Phenacyl, Acetyl, tert.-Butyl, Benzyl oder für den Rest: 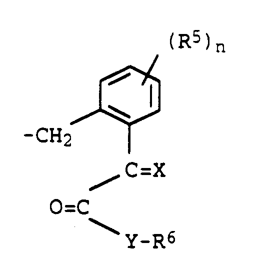
   Die Alkylierung erfolgt auf an sich bekannte Weise. Bezüglich der Alkylierung der Hydroximsäure(thio-)ester IVa sei beispielsweise auf Houben-Weyl, Methoden der Organischen Chemie, Band 10/1, S. 1190; J. Org. Chem. 46 (1981), S. 3623-3629 verwiesen.
   Als Alkylierungsmittel eignen sich insbesondere Alkylhalogenide, Alkylsulfonate, Dialkylsulfate oder Meerwein-Salze (vgl. hierzu z.B. J. Org. Chem. 36 (1971), S. 284-294), Alkyltriflate (vgl. z.B. J. Org. Chem. 54 (1989), S. 1736ff) sowie Diazomethan (vgl. z.B. Austr. J. Chem. 27 (1974), S. 1341-1349).
   Die Umsetzung erfolgt üblicherweise bei Temperaturen von 0 bis 60°C, vorzugsweise 0 bis 20°C.
   Geeignete Lösungsmittel sind beispielsweise Ether wie Diethylether, Dioxan, Ketone wie Aceton, Methylethylketon, Alkohole wie Methanol, Ethanol, sowie Acetonitril, Dimethylsulfoxid, Dimethylformamid und N,N-Dimethylpyrrolidinon. Besonders bevorzugt sind Aceton, Dimethylsulfoxid und Dimethylformamid.
   Verbindungen Va, bei denen R* für eine Phenolschutzgruppe steht, können durch Abspaltung der Schutzgruppe (vgl. z.B. Th. W. Green "Protective Groups in Organic Synthesis", Wiley + Sons, S. 87-108) in Verbindungen Va mit R* = Wasserstoff überführt werden.
   Die Verbindungen Va mit R* = Wasserstoff lassen sich analog Methode A) mit Phenylverbindungen III zu den substituierten Benzoxyphenyl-Derivaten I mit Y = Sauerstoff umsetzen.
C) Halogenierung von Hydroxamsäureestern IVb zu Hydroximsäureesterchloriden bzw. -bromiden Vb und anschließender nukleophiler Substitution des Halogens (vgl. hierzu z.B. J. Org. Chem. 50 (1985), S. 3348-3355) zu Verbindungen Vc: 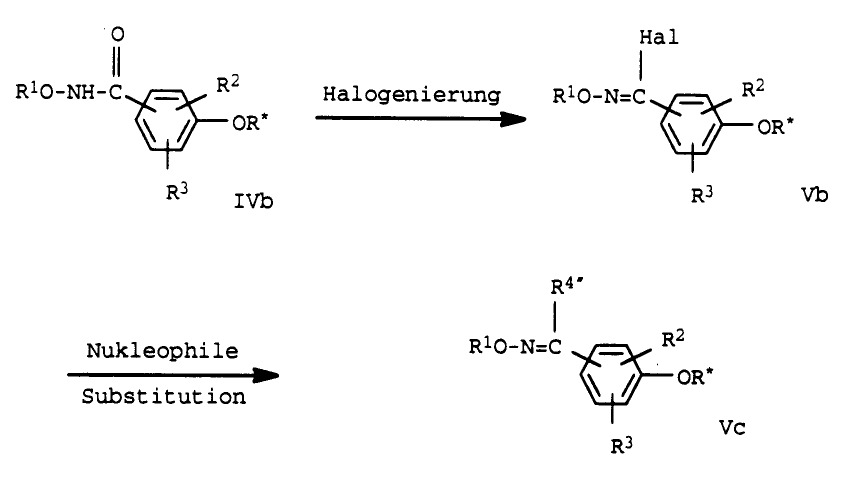 Hal bedeutet Chlor oder Brom und R⁴'' steht für R⁴, ausgenommen Chlor, Brom.
Als Halogenierungsmittel kommen beispielsweise elementares Chlor oder Brom, Hypohalogenide, Thionylchlorid, Sulfurylchlorid, N-Chlorsuccinimid, Phosphortribromid, Phosphorpentachlorid, Triphenylphosphin in Tetrachlormethan oder Triphenylphosphin in Tetrabrommethan in Betracht (vgl. z.B. EP-A 158 153; Houben-Weyl, Methoden der Organischen Chemie, Bd. VIII, S. 691; Synth. Commun. 16 (1986), 763-765; Gazz. Chim. Ital. 114 (1984), S. 131-132).
Die Halogenierung wird in einem inerten Lösungs- oder Verdünnungsmittel wie Tetrachlormethan, Benzol, Diethylether, Dimethylformamid, Dioxan, Dioxan/Wasser durchgeführt.
Die optimale Reaktionstemperatur hängt vom Halogenierungsmittel ab. In den meisten Fällen ist eine Temperatur zwischen etwa 10 und 30°C, insbesondere zwischen 0 und 10 °C ausreichend. Höhere Temperaturen, etwa von 20 bis 80 °C, insbesondere 40 bis 80 °C, haben sich bei der Halogenierung mit Triphenylphosphin in Tetrachlormethan oder Tetrabrommethan als vorteilhaft erwiesen.
   Die Menge an Halogenierungsmittel ist nicht besonders kritisch. Im allgemeinen sind 0,8 bis 2,0, vorzugsweise 0,95 bis 1,2 mol Halogenierungsmittel pro mol IVb ausreichend.
   Als Nukleophile kommen insbesondere Alkoxide (vgl. hierzu z.B. EP-A 158 153), Alkylamine (vgl. z.B. J. Org. Chem. 45 (1980), S. 4198 ff und Heterocycles 26 (1987), S. 188) Thiophenole und Alkylmercaptide (vgl. z.B. EP-A 426 460) in Betracht.
   Als Lösungsmittel für die nukleophile Substitution eignen sich die Alkylamine selbst sowie z.B. Ether wie Diethylether und Tetrahydrofuran, Alkohole wie Ethanol, Dimethylformamid, Dimethylsulfoxid und Acetonitril.
   Normalerweise sind 0,8 bis 2,0, insbesondere etwa 1,0 bis 1,2 mol Nukleophil pro mol Vb ausreichend. Ein größerer Überschuß an Nukleophil, der im Falle der Alkylamine sogar bis zur 20-fachen Menge pro mol Vb betragen kann, bringt im allgemeinen keine zusätzlichen Vorteile.
   Nach den bisherigen Erkenntnissen verläuft die Reaktion bei einer Temperatur von 0 bis 80 °C, vorzugsweise 0 bis 30 °C, mit ausreichender Geschwindigkeit.
   Verbindungen Vb und Vc, bei denen R* für eine Phenolschutzgruppe steht, können durch Abspaltung der Schutzgruppe (vgl. z.B. Th. W. Green "Protective Groups in Organic Synthesis", Wiley + Sons, S. 87-108) in Verbindungen Vb bzw. Vc mit R* = Wasserstoff überführt werden.
   Die Verbindungen Vb und Vc mit R* = Wasserstoff lassen sich analog Methode A) mit Phenylverbindungen III zu den substituierten Benzoxphenyl-Derivaten I mit Y = Sauerstoff umsetzen.
D)
   - Nukleophile Substitution von Chlor oder Brom an Hydroximsäurechloriden oder -bromiden IVc und Alkylierung der Verfahrensprodukte IVd
      oder
   - Alkylierung der Hydroximsäurechloride oder -bromide IVc und gewünschtenfalls nukleophile Substitution an den Verfahrensprodukten Vb 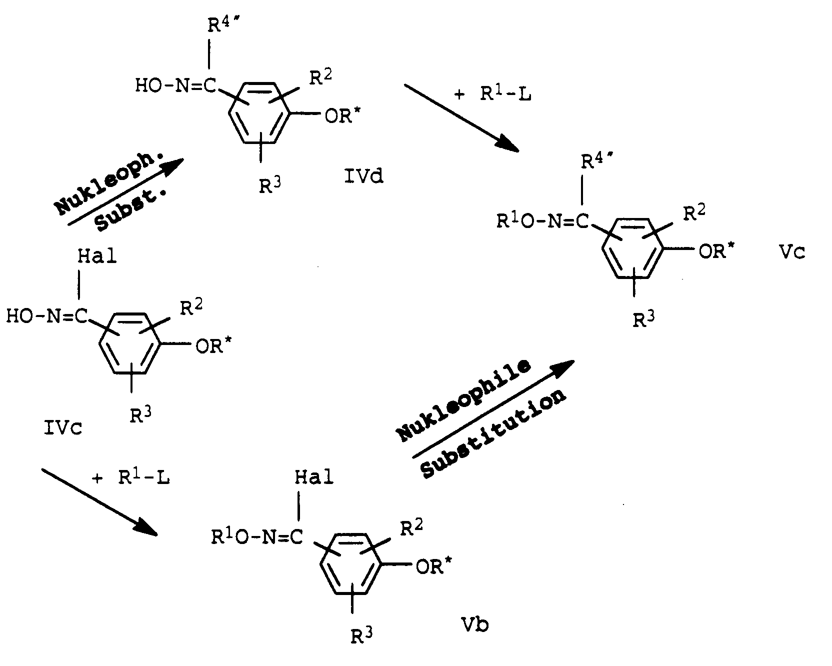
      Als Nukleophile kommen insbesondere Alkoxide (vgl. hierzu z.B. EP-A 158 153), Alkylamine (vgl. z.B. J. Org. Chem. 45 (1980), S. 4198 ff und Heterocycles 26 (1987), S. 188) und Alkylmercaptide (vgl. z.B. EP-A 426 460) in Betracht.
      Für die Alkylierungsreaktionen gelten hier die unter B) gemachten Ausführungen ebenfalls. Zur Definition von L sei auf A) hingewiesen.
      Bezüglich der nukleophilen Substitution sei auf die Ausführungen unter C) verwiesen. Dort ist auch die weitere Umsetzung der Verbindungen Vb und Vc zu Benzoxyphenyl-Derivaten der Formel I, in denen Y für Sauerstoff steht, beschrieben.
E) Alkylierung von Hydroximsäurecyaniden IVe {vgl. z.B. Liebigs Ann. Chem. 10, 1623-1629 (1989)}: 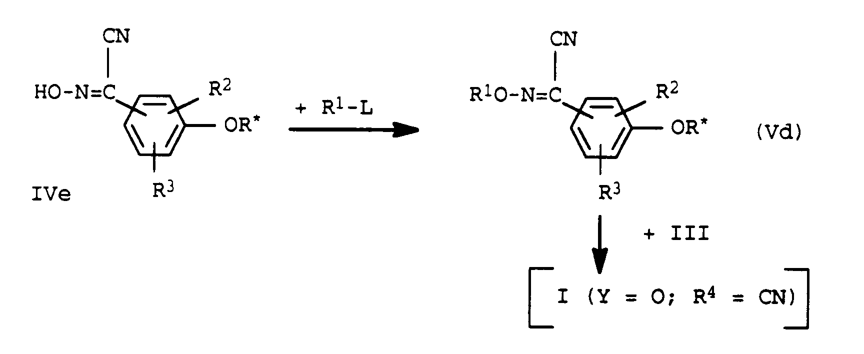 Die unter B) gemachten Ausführungen bezüglich der dortigen Alkylierungsreaktion gelten hier ebenfalls. L wurde unter A) definiert.
   Bezüglich der weiteren Umsetzung der Verbindungen Vd mit Phenylverbindungen III zu substituierten Phenoxymethylphenyl-Derivaten I, wobei Y für Sauerstoff und R⁴ für Cyano stehen, sei auf die Angaben unter C) verwiesen.
F) Durch Umsetzung der substituierten Phenoxymethylphenyl-Derivaten der Formel I, in denen Y für Sauerstoff steht, oder von aktivierten Carbonsäurederivaten VI mit primären (H₂N-R⁶) oder sekundären Aminen (HN(CH₃)-R⁶) sind die entsprechenden Verbindungen I mit Y = -NH- oder -N(CH₃)- herstellbar: 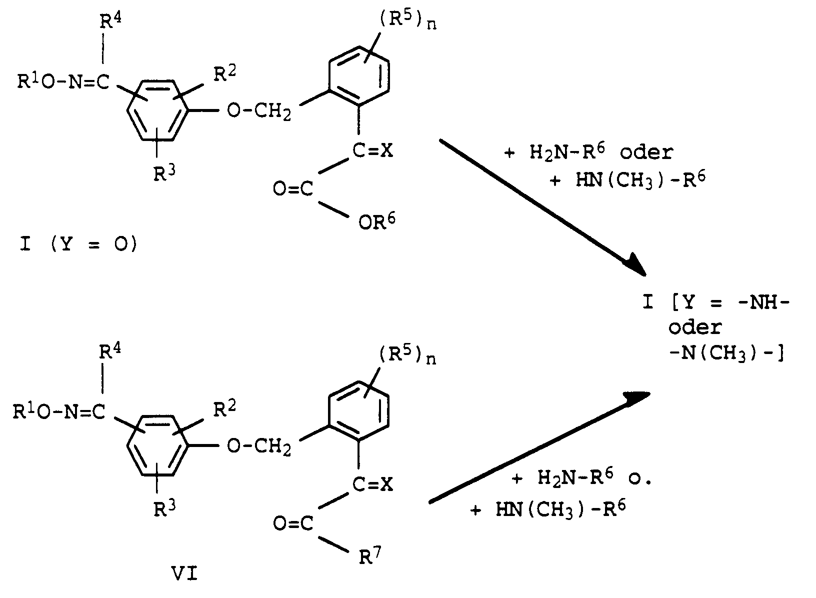
   R⁷ steht für einen Carbonyl-aktivierenden Rest, z.B. für Chlor, Brom und 1-Imidazolyl.
   Die Umsetzungen können in der Regel auf an sich bekannten Weise vorgenommen werden (vgl. z.B. Houben Weyl, Methoden der Organischen Chemie, Bd. E 5, S 941-977, S. 983-991; Houben Weyl, Methoden der Organischen Chemie, Bd. VIII, S. 654 ff).
   Die Reaktion wird normalerweise in einem inerten Lösungs- oder Verdünnungsmittel zwischen (- 10) und 60 °C durchgeführt.
   Geht man von den Verbindungen I mit Y = Sauerstoff aus, so hat es sich als besonders vorteilhaft erwiesen, bei einer Reaktionstemperatur von 10 bis 30 °C und mit einer etwa äquimolaren Menge an Amin oder mit einem Überschuß an Amin bis zur 10-fachen Menge, jeweils bezogen auf I (Y = O), zu arbeiten. Als Lösungsmittel kommen hierbei vor allem Ether wie Tetrahydrofuran und Dioxan, Alkohole wie Methanol und Ethanol, in Betracht. Gewünschtenfalls kann den genannten Lösungsmitteln noch Wasser zugesetzt werden.
   Bei der Variante, die von aktivierten Carbonsäurederivaten VI ausgeht, ist eine Reaktionstemperatur von (- 10) bis 5 °C besonders empfehlenswert, wobei eine Amin-Menge von 0,9 bis 5 mol, insbesondere 0,95 bis 2 mol, pro mol VI, üblicherweise für einen optimalen Umsatz ausreichend ist. Als Lösungsmittel kommen hierbei vor allem Ether wie Diethylether und Tetrahydrofuran, aromatische Kohlenwasserstoffe wie Toluol, chlorierte Kohlenwasserstoffe wie Dichlormethan oder Pyridin in Betracht.
   Die aktivierten Carbonsäurederivaten VI ihrerseits sind aus den Phenoxymethylphenyl-Derivaten I mit -YR⁶ = OH erhältlich (vgl. hierzu z.B. Houben Weyl, Methoden der Organischen Chemie, Bd. VIII, S. 463 ff).
   Die Vorprodukte der Formeln IVa, IVb und IVc sind beispielsweise nach folgenden Verfahren erhältlich:
G) Herstellung der Hydroximinsäureester IVa durch Umsetzung von Benzonitrilen VII mit Alkoholen oder Thiolen nach der Pinner-Reaktion und anschließender Umsetzung der gebildeten Imine mit Hydroxylamin (vgl. z.B. U.S. 4,743,701 und EP-A 158 153): 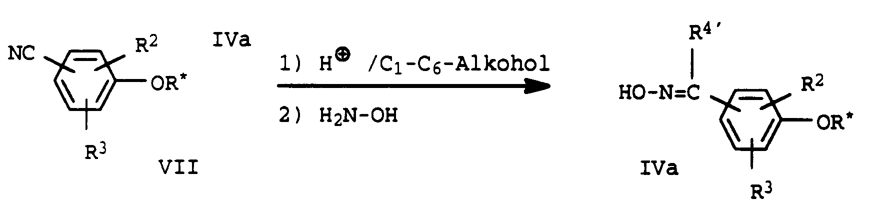
   Die Umsetzung erfolgt normalerweise in einem inerten Lösungs- oder Verdünnungsmittel. Als Lösungsmittel sind besonders Ether wie Dioxan und chlorierte Kohlenwasserstoffe wie Chloroform geeignet. Bei der Umsetzung von VII mit einem Alkohol kann auch ohne zusätzliches Lösungsmittel in einem überschuß an Alkohol gearbeitet werden.
   Die Menge an Alkohol ist nicht kritisch. Normalerweise sind 1 bis 10 mol Alkohol pro mol VII für eine optimale Umsetzung von VII ausreichend. Arbeitet man lösungsmittelfrei in dem betreffenden Alkohol, so kann dieser auch in einem größeren Überschuß vorliegen.
   Die Menge an Hydroxylamin beträgt vorzugsweise 0,9 bis 10 mol, insbesondere 1 bis 2 mol, pro mol VII.
   Die Pinner-Reaktion wird üblicherweise bei 20 bis 150 °C, Vorzugsweise 40 bis 80 °C, vorgenommen, wogegen für die anschließende Umsetzung mit Hydroxylamin 0 bis 60 °C, vorzugsweise 20 bis 40 °C, empfehlenswert sind.
H) Herstellung von Hydroximinsäurehalogeniden IVc durch Oxidative Halogenierung von Aldoximen IX: 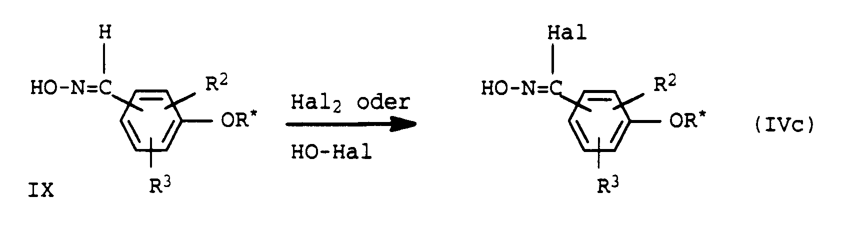
   Als Halogenierungsmittel kommen beispielsweise elementares Chlor oder Brom oder Hypohalogenide in Betracht (vgl. z.B. EP-A 158 153; Houben-Weyl, Methoden der Organischen Chemie, Bd. VIII, S. 691; Synth. Commun. 16 (1986), 763-765; Gazz. Chim. Ital. 114 (1984), S. 131-132).
   Geeignete Lösungs- oder Verdünnungsmittel sind z.B. Ether wie Diethylether und Dioxan, chlorierte Kohlenwasserstoffe wie Dichlormethan, sowie Dimethylformamid oder Essigsäure.
   Im allgemeinen sind 0,9 bis 5, insbesondere 1,0 bis 1,5 mol des Halogenierungsmittels pro mol IX für einen optimalen Umsatz ausreichend.
   Es empfiehlt sich, die Halogenierung bei einer Reaktionstemperatur von (-10) bis 60 °C, insbesondere (-5) bis 20 °C, vorzunehmen. 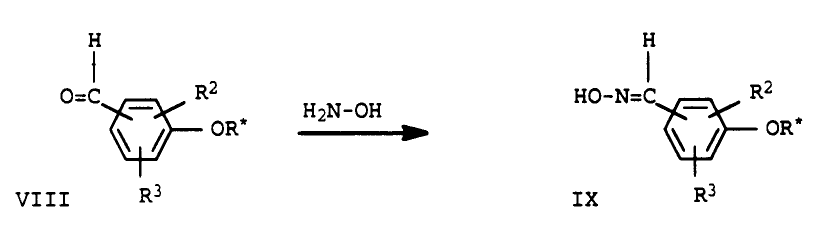
   Die Aldoxime IX sind ihrerseits durch Umsetzung von Benzaldehyden VIII mit Hydroxylamin erhältlich (vgl. z.B. Houben-Weyl, Methoden der Organischen Chemie, Band 10/4, 4. Auflage, 1968, Georg-Thieme Verlag, Stuttgart, Seiten 55-66):
   Geeignete Lösungs- oder Verdünnungsmittel sind Wasser, Alkohole wie Methanol und Ethanol oder Mischungen aus Wasser und Alkohol.
   Das Hydroxylamin wird vorzugseise als Hydroxylammonium-Salz, insbesondere als Hydrochlorid, Acetat oder Sulfat, eingesetzt, und zwar in einer Menge von etwa 0,8 bis 2,0, insbesondere 0,95 bis 1,2 mol, pro mol VIII.
   Zwecks Neutralisation der entstehenden Säure kann es zweckmäßig sein, in Gegenwart einer Base zu arbeiten. Geeignete Basen sind z.B. Alkalimetallhydrogencarbonate wie Natriumhydrogencarbonat, Alkalimetallacetate wie Natriumacetat, und organische Basen wie Ammoniak und Pyridin.
   Im allgemeinen sind 1,0 bis 1,7 mol an Base pro mol Hydroxylammoniumsalz ausreichend.
   Die Temperaturbereich liegt normalerweie bei 0 bis 80 °C, insbesondere 20 bis 60 °C.
J) Herstellung der Hydroxamsäureester IVb durch Umsetzung von Benzoesäurederivate XI mit Hydroxylaminen H₂N-OR¹ (vgl. hierzu Houben-Weyl, Methoden der Organischen Chemie, Bd. E5, S. 1144-1146): 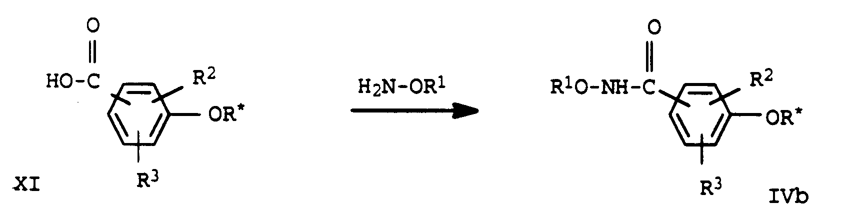
   Diejenigen Benzoesäurederivate XI, bei denen R* den Rest 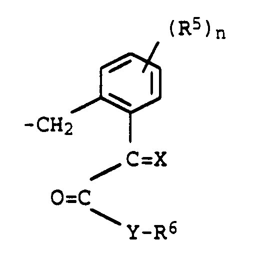 bedeutet, sind neu (= α-Methoxyimino-phenylessigsäuremethylamide XI').
   Ebenfalls neu sind diejenigen Verbindungen IVb, bei denen R* den Rest 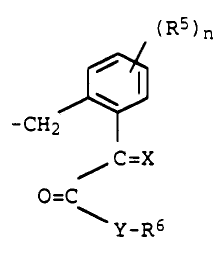 bedeutet (= 2-(Phenoxyethyl)-phenylessigsäure-Derivate IVb')
   Die Benzoesäurederivate XI bzw. die α-Methoxyimino-phenylessigsäuremethylamide XI' sind ihrerseits erhältlich, indem man beispielsweise
   - Acetophenonene X bzw. X' der Haloformreaktion unterwirft (vgl. hierzu z.B. J. Am. Chem. Soc. 68 (1946), S. 1648 ff und J. Org. Chem. 12 (1947), S. 603 ff; Helv. Chim. Acta 49 (1966), S. 561 ff): 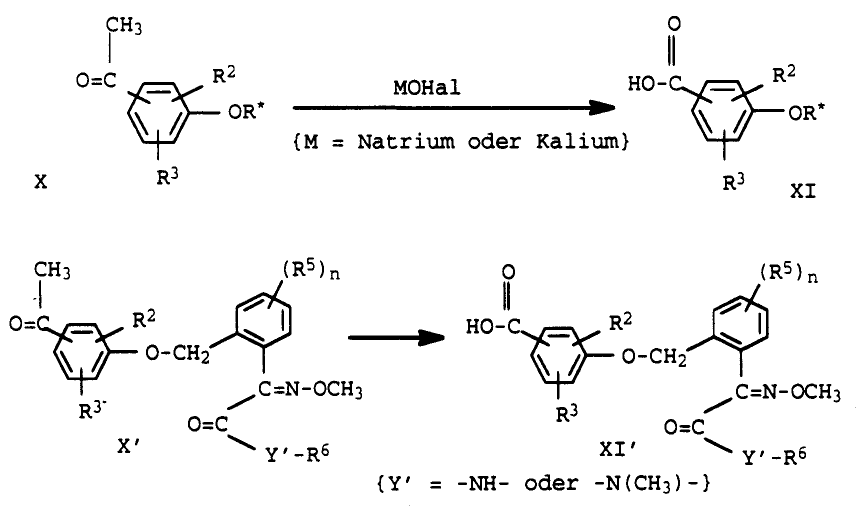 Als Reagenzien besonders geeignet haben sich die Erdalkalihypohalogenide wie Natriumhypochlorid, Kaliumhypochlorid und Natriumhypobromid erwiesen. Die Menge an Hypohalogenid ist nicht besonders kritisch; sie liegt zweckmäßigerweise bei 0,8 bis 20 mol, insbesondere von etwa 1,0 bis 10 mol, pro mol X oder X'.
      Vorteilhaft arbeitet man in Wasser oder Wasser-Dioxan-Gemischen bei einer Reaktionstemperatur von etwa 0 bis 100°C, besonders bevorzugt 5 bis 30°C.
   - oder indem man Halogenaromaten XII carbonyliert, vorteilhaft unter Pd-Katalyse (vgl. hierzu J. Org. Met. Chem. 358 (1988), S. 563-565; J. Org. Chem. 56 (1991), S. 4320-4322 und U.S. 4,990,657):

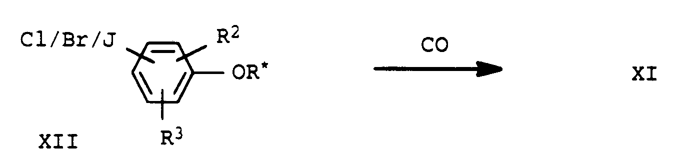

Die Carbonylierung wird vorzugsweise in einem Gemisch aus Wasser und z.B. Tetramethylharnstoff, Toluol oder Dimethylformamid vorgenommen.

Als Katalysatoren haben sich Palladium(II)-Verbindungen wie PdCl₂, Pd(O-COCH₃)₂ und Pd[P(C₆H₅)₃]₂Cl₂, gewünschtenfalls unter Zusatz von P(C₆H₅)₃, geeignet erwiesen, wobei normalerweise eine katalytische Menge an Katalysator, z.B. 0,01 bis 0,1 mol, vorzugsweise 0,01 bis 0,05 mol pro mol XII, für die Reaktion ausreichend ist.

Die Reaktionsführung erfolgt üblicherweise in Gegenwart einer Base, z.B. eines Erdalkalimetallcarbonates wie Kaliumcarbonat, oder einer organischen Base wie Triethylamin und Pyridin, wobei die Menge an Base vorzugsweise etwa 1 bis 10, insbesondere 2 bis 4 mol pro mol XII beträgt.

Die Reaktionstemperatur liegt allgemein bei 30 bis 200°C, insbesondere bei 100 bis 150°C.

Die Carbonylierung wird besonders vorteilhaft bei einem Kohlenmonoxid-Überdruck von 2 bis 200 bar, insbesondere von etwa 50 bar, vorgenommen.

Von der Carbonylierung der Halogenaromaten XII abgesehen werden alle vorstehend beschriebenen Reaktionen zweckmäßigerweise bei Atmosphärendruck oder unter dem Eigendruck des jeweiligen Verdünnungsmittels vorgenommen.

### Herstellungsbeispiele

### Beispiel 1

### E-2-Methoximino-2-[-2-(4-[ethoximino-methoxymethyl]-2,5-dimethyl-phenoxymethyl)-phenyl]-essigsäuremethylamid (Tab. 1, Nr. 1.004)

1,6 g Dimethylsulfat wurden bei 20-25 °C zu einer Suspension von 3,1 g E-2-Methoximino-2-[2-(4-ethoxyaminocarbonyl-2,5-dimethyl-phenoxymethyl)-phenyl]-essigsäuremethylamid (hergestellt nach Beispiel 5) und 1,74 g feingepulvertem Kaliumcarbonat in 60 ml wasserfreiem Aceton getropft. Nach 16 Stunden Rühren wurde die Reaktionsmischung in 100 ml Eiswasser, das mit 20 ml verd. Ammoniakwasser versetzt wurde, gegeben. Man extrahierte dreimal mit Methyl-tert.-butylether, wonach die die vereinigten organischen Phasen mit ges. Natriumchloridlösung gewaschen, dann über Natriumsulfat getrocknet und schließlich zur Trockene eingeengt wurden. Das Rohprodukt wurde durch Mitteldruckchromatographie über Kieselgel (Laufmittel: n-Heptan/Essigsäureethylester 1 : 1 v/v) gereinigt. Ausbeute: 0,45 g des gewünschten O-alkylierten Produkts als Öl.
¹H-NMR (in CDCl₃, TMS als interner Standard): δ [ppm] = 1,35(t,3H); 2,2(s,3H); 2,35(s,3H); 2,9(d,3H); 3,6(s,3H); 3,95(s,3H); 4,2(q,2H); 5,0(s,2H); 6,6(s,1H); 6,7(breit,NH); 7,1(s,1H); 7,2-7,6(m,4H)

### Beispiel 2

### E-2-Methoximino-2-[2-(4-[chlorethoximinomethyl]-2,5-dimethyl-phenoxymethyl)-phenyl]-essigsäuremethylamid (Tab. 1, Nr. 1.008)

3,3 g E-2-Methoximino-2-[2-(4-ethoxyaminocarbonyl-2,5-dimethyl-phenoxymethyl)-phenyl]-essigsäuremethylamid (hergestellt nach Beispiel 5) und 3,1 g Triphenylphosphin wurden unter wasserfreien Bedingungen in 30 ml Acetonitril gelöst. Nach Zugabe von 3,7 g Tetrachlormethan erhitzte man 2 Stunden auf Rückflußtemperatur. Anschließend kühlte man die Reaktionsmischung auf etwa 20°C ab und trennte sie chromatographisch über eine Kieselgelsäule (10 g Kieselgel) auf (Eluent: Toluol/Essigsäureethylester 9 : 1). Das Eluat wurde wie üblich auf das Produkt hin aufgearbeitet. Ausbeute: 1,9 g; Fp.: 87 - 90 °C;
¹H-NMR (in CDCl₃, TMS als interner Standard): δ [ppm] = 1,35(t,3H); 2,15(s,3H); 2,35(s,3H); 2,9(d,3H); 3,9(s,3H); 4,3(q,2H); 5,0(s,2H); 6,6(s,1H); 6,75(breit,NH); 7,2-7,6(m,5H).

### Beispiel 3

### E-2-Methoximino-2-[2-(4-[brom-ethoximinomethyl]-2,5-dimethylphenoxymethyl)-phenyl]-essigsäuremethylamid (Tab. 1, Nr. 1.009)

6,6 g E-2-Methoximino-2-[2-(4-ethoxyaminocarbonyl-2,5-dimethylphenoxymethyl)-phenyl]-essigsäuremethylamid (hergestellt nach Beispiel 5) und 6,2 g Triphenylphosphin wurden unter wasserfreien Bedingungen in 60 ml Acetonitril gelöst. Nach Zugabe von 8,0 g Tetrabrommethan erhitzte man 4 Stunden auf Rückflußtemperatur, wonach das Reaktionsgemisch nochmals mit 2 g Tetrabrommethan versetzt wurde. Anschließend erhitzte eine weitere Stunde auf Rückflußtemperatur und arbeitete dann analog Beispiel 6 auf das Produkt hin auf. Ausbeute: 4,8 g kristallines Produkt; Fp.: 74 - 77 °C;
¹H-NMR (in CDCl₃, TMS als interner Standard): δ [ppm] = 1,3(t,3H); 2,15(s,3H); 2,35(s,3H); 2,9(d,3H); 3,95(s,3H); 4,3(q,2H); 4,95(s,2H); 6,6(s,1H); 6,75(breit,NH); 7,2-7,6(m,5H).

### Beispiel 4

### E-2-Methoximino-2-[2-(4-[ethoximino-methylthiomethyl]-2,5-dimethylphenoxymethyl)-phenyl]-essigsäuremethylamid (Tab. 1, Nr. 1.010)

Zu einer Lösung von 1,9 g E-2-Methoximino-2-[2-(4-[brom-ethoximinomethyl]-2,5-dimethylphenoxymethyl)-phenyl]-essigsäuremethylamid (hergestellt nach Beispiel 3) in 20 ml wasserfreiem Dimethylformamid wurden 0,42 g festes Natriumthiomethylat gegeben. Nach 16 Stunden Rühren bei ca. 20 °C goß man die Reaktionsmischung auf ein Gemisch aus Eiswasser und Methyl-tert.-butylether. Man trennte die Phasen und extrahierte die wäßrige Phase noch dreimal mit Methyl-tert. butylether extrahiert. Danach wurden die vereinigten organischen Phasen mit 20 ml 2 N Natronlauge und dann mit 100 ml gesättigter Natriumchloridlösung gewaschen, über Natriumsulfat getrocknet und eingeengt. Durch Verreiben des Rückstandes mit n-Heptan/Diisopropylether erhielt man 1,4 g kristallines Produkt; Fp.: 146 - 148°C.
¹H-NMR (in CDCl₃, TMS als interner Standard): δ [ppm] = 1,35(t,3H); 1,85(s,3H); 2,2(s,3H); 2,3(s,3H); 2,9(d,3H); 3,95(s,3H); 4,3(q,2H); 5,0(s,2H); 6,65(s,1H); 6,75(breit,NH); 7,0(s,1H); 7,2-7,6(m,4H).

In der folgenen Tabelle 1 sind weitere substituierte Benzoxyphenyl-Derivate I enthalten, die auf die gleich Weise herstellt wurden oder herstellbar sind.

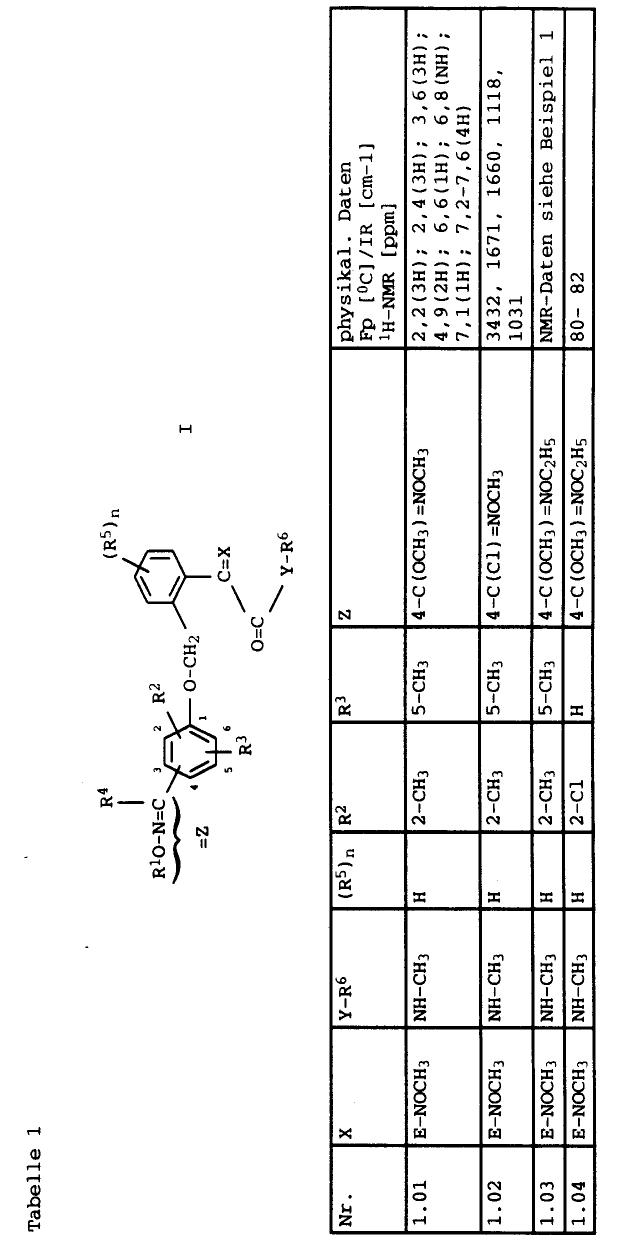

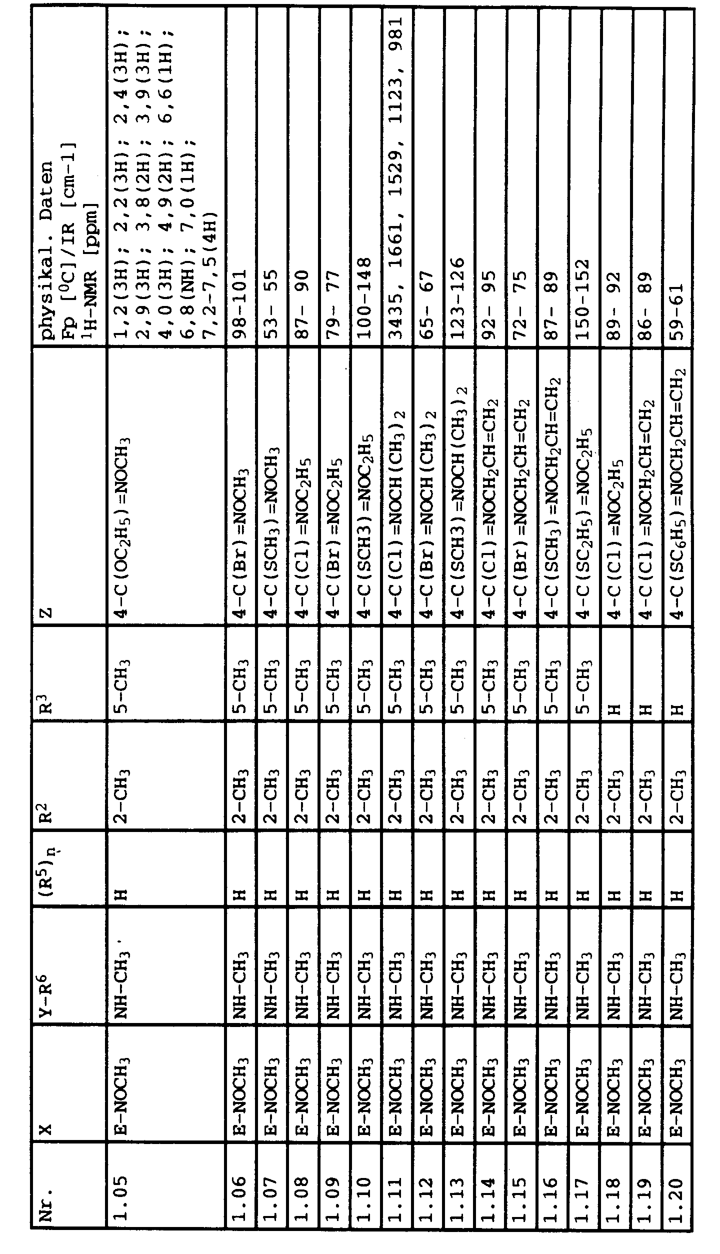

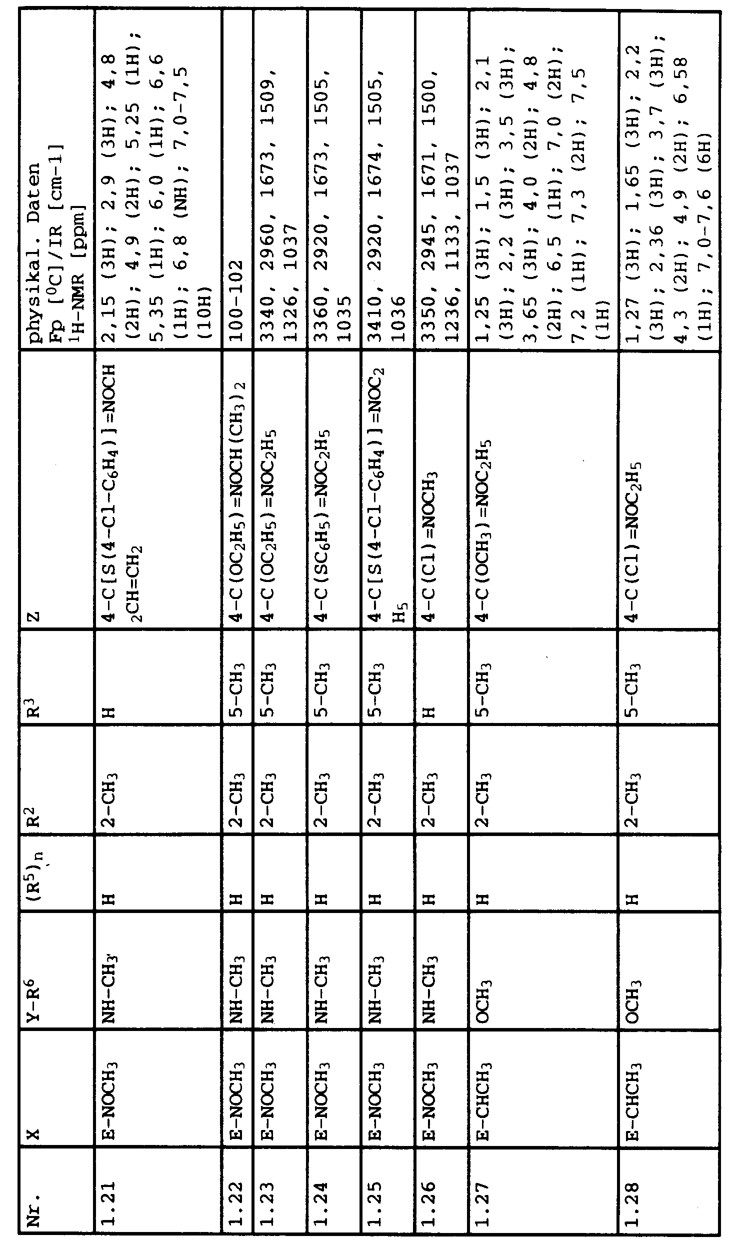

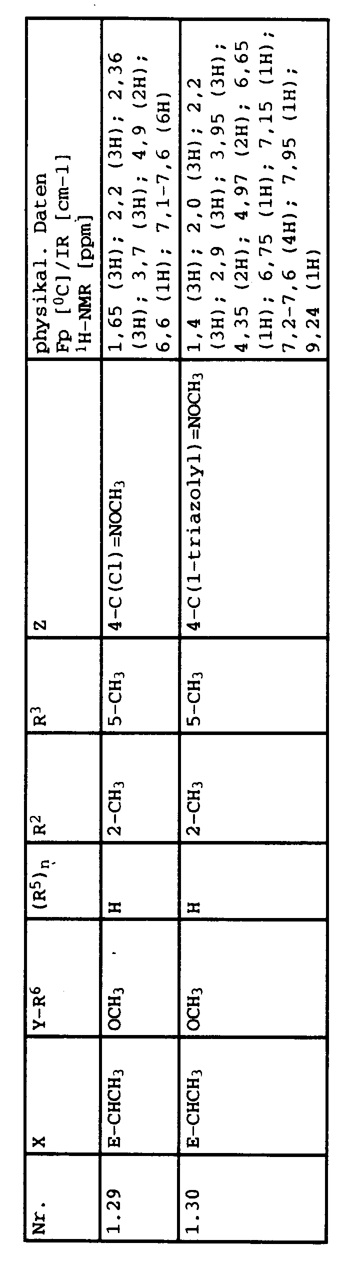

### Beispiel 5

### E-2-Methoximino-2-[2-(4-ethoxyaminocarbonyl-2,5-dimethyl-phenoxymethyl)-phenyl]-essigsäuremethylamid (Tab. 2, Nr. 2.003)

Eine Suspension aus 5,15 g o-Ethylhydroxylamin-hydrochlorid und 50 ml wasserfreiem Dichlormethan wurde bei 0°C mit 10,0 g N-Methylmorpholin versetzt. Zu dieser Mischung tropft man unter Eiskühlung eine Lösung von 18,6 g 2-Methoximino-2-[2-(4-chlorcarbonyl-2,5-dimethyl-phenoxymethyl)-phenyl]essigsäuremethylamid (hergestellt nach Beispiel 3) in 50 ml Dichlormethan. Nach beendeter Zugabe ließ man das Reaktionsgemisch auf 20-25 °C erwärmen und rührt weitere 12 Stunden bei dieser Temperatur. Anschließend trennte man den Feststoffanteil ab und engte die erhaltene Lösung bis zur Trockene ein. Der erhaltene Rückstand wurde mit Essigsäureethylester verrührt, wonach man den hierbei unlöslichen Anteil abtrennte und in Dichlormethan löste. Die Lösung wurde mit mit verd. Salzsäure und Wasser extrahiert, dann über Natriumsulfat getrocknet und eingeengt. Ausbeute: 17,4 g des Hydroxamids; Fp.: 177 - 180 °C;
¹H-NMR (in CDCl₃, TMS als interner Standard): δ [ppm] = 1,3 (t,3H); 2,15 (s,3H); 2,4 (s,3H); 2,9 (d,3H); 3,95 (s,3H); 4,05 (q,2H); 4,95 (s,2H); 6,6 (s,1H); 6,75 (breit,NH); 7,2 (s,1H); 7,2 - 7,6 (m,4H); 8,5 (s,1H).

### Beispiel 6

### E-2-Methoxyimino-2-[2-(4-ethoxyaminocarbonyl-2-methyl-phenoxy-methyl)-phenyl]-essigsäuremethylamid (Tab. 2, Nr. 2.06)

30 g 2-Methoximino-2[2-(4-chlorcarbonyl-2-methyl-phenoxymethylphenyl]-essigsäureamid wurden analog Beispiel 5 mit 8,6 g o-Ethylhydroxylamin-hydrochlorid umgesetzt. Nach 18 h rühren bei Raumtemperatur wurde wie oben beschrieben aufgearbeitet. Ausbeute 19,1 g des Hydroxamids; Fp.: 142-145°C
¹H-HMR (in CDCl₃, TMS als interner Standard): δ [ppm] = 1,2 (t,3H); 2,2(s,3H); 2,8 (d,3H); 3,9 (s,3H); 3,95 (q,2H); 4,9 (s,2H); 6,7 (d,1H); 6,9 (breit,NH); 7,1-7,6 (m,6H); 9,6 (breit,NH).

### Beispiel 7

### E-2-Methoximino-2-[2-(4-[ethoxyimino-(4-chlorphenylthio)methyl]-2,5-dimethylphenoxymethyl)-phenyl]-essigsäuremethylamid (Tab. 1, Nr. 1.21)

Zu einer Lösung von 2,4 g E-2-Methoximino-2-[2-[4-[brom-ethoximino-methyl)-2,5-dimethylphenoxymethyl)-phenyl]-essigsäuremethylamin (hergestellt nach Beispiel 3) in 20 ml wasserfreiem Dimethylformamid wurden 1,8 g Kaliumcarbonat und 1,44 g 4-Chlor-thiophenol gegeben. Nach 5 h Erwärmen auf 60°C wurden nochmals 1,4 g 3-Chlortherphenol zudosiert und der Ansatz noch 1 h bei 60°C belassen. Zur Aufarbeitung wurde der Ansatz in Eiswasser eingerührt und mit Methyl-tert.butylether extrahiert. Die vereinigten organischen Phasen wurden mit Natriumcarbonatlösung und Wasser gewaschen, über Natriumsulfat getrocknet und am Rotationsverdampfer eingeengt. Der Rückstand wurde über eine Kieselgelsäule chromatographiert (Laufmittel Toluol (Essigester 95/5): Man erhält 1,7 g der Titelverbindung als gelbes Öl.
¹H-NMR (in CDCl₃, TMS als interner Standard): δ [ppm] = 1,35 (t,3H); 2,0 (s,3H); 2,1 (s,3H); 2,9 (d,3H); 3,9 (s,3H); 4,3 (q,2H); 4,8 (s,2H); 6,4 (s,1H); 6,7 (breit,NH); 6,8 (s,1H); 7,0-7,5 (m,8H).

### Vorstufe α)

### E-2-Methoximino-2-[2-(4-acetyl-2,5-dimethyl-phenoxymethyl)-phenyl]-essigsäuremethylamid

Zu einer Mischung aus 18,45 g 2-Methoxyimino-2-[2-(4-acetyl-2,5-dimethylphenoxymethyl)-phenyl]-essigsäuremethylester und 180 ml Tetrahydrofuran wurden 8,5 ml einer 40 gew.-%igen wäßrigen Methylaminlösung gegeben. Nach 16 Stunden Rühren bei 20-25 °C wurde die Reaktionsmischung auf 200 ml Eiswasser/100 ml Methyl-tert. butylether gegossen. Hierzu gab man anschließend verdünnte Salzsäure, bis die wässrige Phase einen pH-Wert zwischen 2 und 3 hatte. Nach Trennung der Phasen extrahierte man die wäßrige Phase mehrmals mit Methyl-tert.-butylether. Die Vereinigten organischen Phasen wurden mit gesättigter Natriumchlorid-Lösung neutral gewaschen, getrocknet und eingeengt. Ausbeute: 17,6 g; Fp.: 146 - 148 °C;
¹H-NMR (in CDCl₃, TMS als interner Standard): δ [ppm] = 2,2 (s,3H); 2,5 (s,3H); 2,55 (s,3H); 2,9 (d,3H); 3,95 s,3H); 5,0(s,2H); 6,65(s,1H); 6,75(breit,NH); 7,2 - 7,6(m,5H).

### Vorstufe β)

### E-2-Methoximino-2-[2-(4-carboxy-2,5-dimethyl-phenoxymethyl)-phenyl]-essigsäuremethylamid

Zu einer auf 10 °C gekühlten Lösung von 20 g Natriumhydroxid in 120 ml Wasser tropfte man 24 g Brom so langsam zu, daß die Temperatur 10 °C nicht überstieg. Die erhaltene Bromoform-Lösung wurde unter Eiskühlung mit einer Lösung von 17 g des aus Vorstufe α) erhaltenen E-2-Methoximino-2-[2-(4-acetyl-2,5-dimethyl-phenoxymethyl)-phenyl]-essigsäuremethylamids in 150 ml Dioxan, versetzt. Nach einer weiteren Stunde Rühren bei 20-25 °C wurden 50 ml 20 gew.-%ige Natriumhydrogensulfit-Lösung zugegeben, um überschüssiges Bromoform zu entfernen. Das feste Produkt wurde durch Ansäuern der Reaktionsmischung mit konz. Salzsäure auf einen pH-Wert von 2 erhalten. Es wurde abgetrennt, zweimal mit Petrolether aufgerührt und bei 60 °C getrocknet. Ausbeute: 18,5 g; Fp.: 195 - 197 °C
¹H-NMR (in CDCl₃, TMS als interner Standard): δ [ppm] = 2,2 (s,3H); 2,6 (s,3H); 2,9 (d,3H); 3,95 (s,3H); 5,0 (s,2H); 6,6 (s,1H); 6,75 (breit,NH); 7,2 - 7,6 (m,4H); 7,85 (s,1H).

### Vorstufe γ)

### 2-Methoximino-2-[2-(4-chlorcarbonyl-2,5-dimethyl-phenoxymethyl)-phenyl]essigsäuremethylamid

Eine Lösung von 9,2 g des aus Vorstufe β) erhaltenen E-2-Methoximino-2-[2-(4-carboxy-2,5-dimethyl-phenoxymethyl)-phenyl]-essigsäuremethylamids in 100 ml wasserfreiem Dichlormethan wurde mit einigen Tropfen Dimethylformamid versetzt. Zu dieser Mischung tropfte man anschließend bei 20-25 °C 4,5 g Thionylchlorid wonach das Reaktionsgemisch 1,5 Std. auf Rückflußtemperatur erhitzt wurde. Anschließend ließ man abkühlen und entfernte die leichtflüchtigen Anteile bei reduziertem Druck. Der Rückstand wurde schließlich nochmals mit 100 ml Dichlormethan aufgenommen und durch einengen der Lösung erneut zurückgewonnen. Ausbeute: 9,3 g Säurechlorid als hellgelber Feststoff, der ohne weitere Reinigung für nachfolgende Umsetzungen verwendet werden konnte.
¹H-NMR (in CDCl₃, TMS als interner Standard): δ [ppm] = 2,2 (s,3H); 2,6 (s,3H); 2,95 (s,3H); 4,0 (s,3H); 4,95 (s,2H); 6,6 (s,1H); 7,2 - 7,6 (m,4H); 7,85 (s,1H); 8,1 (breit, 1H).

### Vorstufe δ

### E-2-Methoximino-2-[2-(4-carboxy-2-methyl-phenoxymethyl)-phenyl]-essigsäuremethylamid

Analog dem Verfahren zur Vorstufe β) wurden 145 g E-2-Methoximino-2-[2-(4-acetyl-2-methyl-phenoxymethyl)-phenyl)-essigsäuremethylamid in 1400 ml Dioxan zur Carbonsäure umgesetzt. Zur Aufarbeitung wurde der Ansatz mit konz. Schwefelsäure auf pH = 2 eingestellt und mit Dichlormethan extrahiert. Die vereinigten organischen Phasen wurden neutral gewaschen, über Natriumsulfat getrocknet und am Rotationsverdampfer eingeengt. Der verbleibende Feststoff wurde mit Diisopropylether verrieben, abgesaugt und getrocknet. Ausbeute: 110 g, Fp.: 185°C (Zers.)
¹H-NMR (in CDCl₃, TMS als interner Standard): δ [ppm] = 2,25 (s,3H); 2,9 (d,3H); 3,95 (s,3H); 5,0 (s,2H); 6,75 (breit,NH); 6,85 (d,1H); 7,1-7,5 (m,4H); 7,9 (m,2H); 11,0 (breit,OH).

### Vorstufe ε

### 2-[2-(4-Carboxy-2,5-dimethyl-phenoxymethyl)-phenyl]-E-but-2-ensäuremethylester

Analog dem Verfahren zur Vorstufe δ) wurden 9,6 g 2-[2-(Acetyl-2,5-dimethyl-phenoxymethyl)-phenyl]-E-but-2-ensäuremethylester in 60 ml Dioxan zur Carbonsäure umgesetzt. Nach dem Einengen am Rotationsverdampfer verblieb ein gelbes Öl, welches neben 70 % der Titelverbindung noch 30 % der durch Hydrolyse des Butansäuremethylester entstandenen "Disöure" enthielt.

Aus diesem Rohprodukt erhielt man analog den hier beschriebenen Verfahren und nach entsprechenden chromatographischer Abtrennung der Disäurederivate die erfindungsgemäßen Verbindungen (s. z.B. Tabelle 1, Nr. 1.29 bis 1.32)
¹H-NMR (in DMSO, TMS als interner Standard): δ [ppm] = 1,6 (d,3H); 2,1 (s,3H); 2,5 (s,3H); 3,6 (s,3H); 5,0 (s,2H); 6,8 (s,1H); 7,1 (q,1H); 7,4-7,5 (m,2H); 7,6 (m,1H); 7,7 (m,2H).

### Vorstufe χ

### E-2-Methoximino-2-[2-(4-carboxy-2-methyl-phenoxymethyl)-phenyl]-essigsäuremethylester

2,2 g E-2-Methoximino-2-[2-(4-jod-2-methyl-phenoxymethyl)-phenyl]-essigsäuremethylester, 0,35 g Bis-triphenylphosphinopalladium II-chlorid und 80 ml Tetramethylharnstoff wurden in 50 ml Wasser in einem 300 ml-HC-Autoklaven vorgelegt. Dann wurde Kohlenmonoxid bis zu 30 bar Druck aufgepreßt und auf 100°C erwärmt. Nach der Aufheizphase wurde der CO-Druck auf 100 bar eingestellt. Dieser Druck wurde stündlich nachreguliert. Nach 24 h wurde der Ansatz abfiltriert und vom Filtrat Tetramethylharnstoff bei 75°C im Ölpumpenvakuum (0,2 mbar) destilliert. Beim Aufnahmen des verbleibenden Rückstands im MTBE/wäßriger NaHCO₃-Lösung fiel das Produkt aus. Nach Absaugen und Trocknen erhielt man 0,9 g der Titelverbindung. Fp.: 192°C
¹H-NMR (in CDCl₃, TMS als interner Standard): δ [ppm] = 2,1 (s,3H); 3,7 (s,3H); 3,9 (s,3H); 5,0 (s,2H); 7,0 (d,1H); 7,25 (d,1H); 7,45 (m,2H); 7,55 (d,1H); 7,25 (m,2H).

In der folgenden Tabelle 2 sind weitere 2-(Phenoxymethyl)-phenylessigsäure-Derivate IVb' aufgeführt, die analog Beispiel 5 hergestellt wurden oder herstellbar sind.

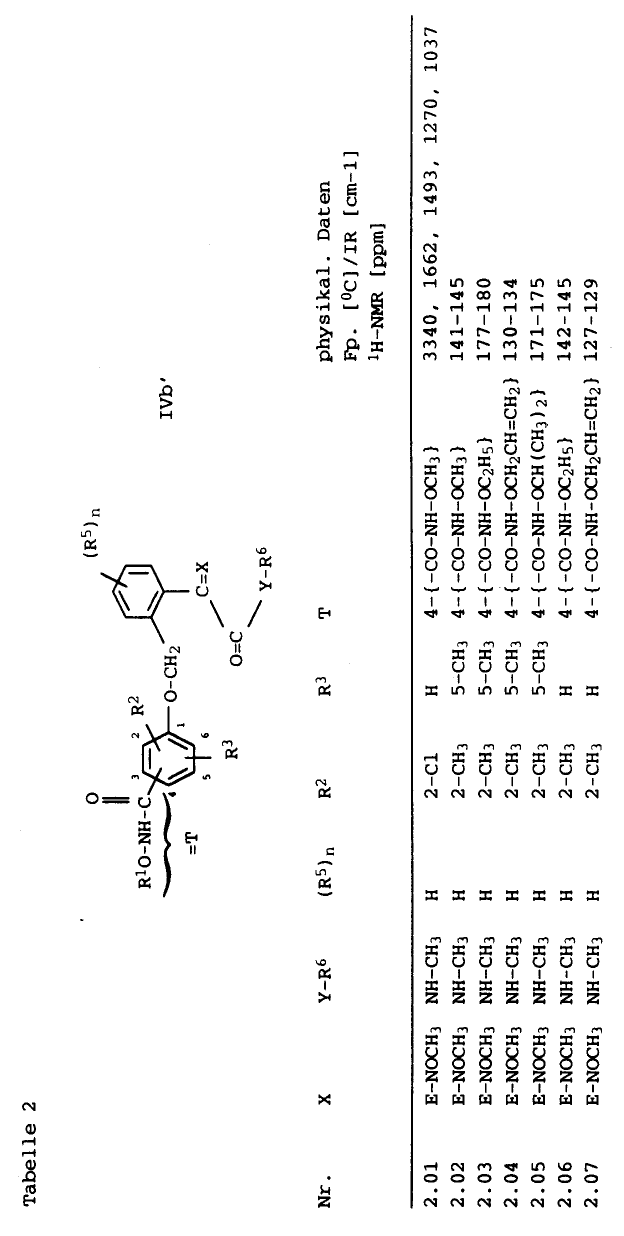

Die substituierten Benzoxyphenyl-Derivate I eignen sich zur Bekämpfung von Schadpilzen und von Schädlingen aus der Klasse der Insekten, Spinnentiere und Nematoden. Sie können im Pflanzenschutz sowie auf dem Hygiene-, Vorratsschutz- und Veterinärsektor als Fungizide und als Schädlingsbekämpfungsmittel eingesetzt werden.

Zu den schädlichen Insekten gehören:
- aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis;
- aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria;
- aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa;
- aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci;
- aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta;
- aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor;
- aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii;
- aus der Ordnung der Termiten (Isoptera) beispielsweise Calotermes flavicollis, Leucotermes flavipes, Reticulitermes lucifugus, Termes natalensis;
- aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Zu den schädlichen Spinnentieren (Acarina) gehören beispielsweise Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Beispiele für schädliche Nematoden sind Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Als Fungizide sind die substituierten Benzoxyphenyl-Derivate I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-VolumeVerfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen der Verbindung Nr. I.01 und 10 Gew.-Teilen N-Methyl-α-pyrrolidon, die zur Anwendung in Form kleinster Tropfen geeignet ist;
II. eine Lösung von 20 Gew.-Teilen der Verbindung Nr. I.02 in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen der Verbindung Nr. I.03 in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen der Verbindung Nr. I.04, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen der Verbindung Nr. I.01, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. I.02 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. I.03, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. I.04, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. I.01, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen der Verbindung Nr. 2.1, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthals$ure-di-isopropylester;
heterocyclische Substanzen, wie 2-Heptadecyl-2-imidazolin-acetat,
2,4-Dichlor-6-(o-chloranilino)-s-triazin,
O,O-Diethyl-phthalimidophosphonothioat,
5-Amino-1-β-[bis-(dimethylamino)-phosphinyl]-3-phenyl-1,2,4-triazol,
2,3-Dicyano-1,4-dithioanthrachinon, 2-Thio-1,3-dithiolo-β-[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
   sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

### Anwendungsbeispiele (fungizide Wirksamkeit)

Als Vergleichssubstanz diente 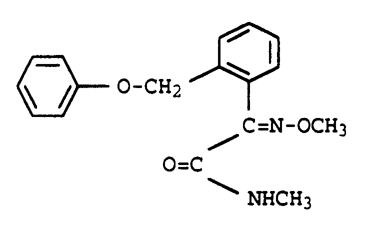 bekannt aus der EP-A 398 692 (Tabelle 3, Nr. 46).

### Anwendungsbeispiel 1

### Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach stellte man die Töpfe für 24 Stunden bei 20 bis 22°C in eine Kammer mit hoher Luftfeuchtigkeit (90 bis 95 %). Während dieser Zeit keimten die Sporen aus und die Keimschläuche drangen in das Blattgewebe ein. Die infizierten Pflanzen wurden anschließend mit wäßrigen Spritzbrühen, die 80 Gew.-% Wirkstoff und 20 Gew.-% Emulgiermittel in der Trockensubstanz enthielten, tropfnaß gespritzt. Nach dem Antrocknen des Spritzbelages stellte man die Versuchspflanzen im Gewächshaus bei Temperaturen zwischen 20 und 22°C und 65 bis 70 % relativer Luftfeuchte auf. Nach 8 Tagen wurde das Ausmaß der Rostpilzentwicklung auf den Blättern ermittelt.

Durch Behandlung mit wäßrigen Wirkstoffaufbereitungen, die 63 ppm der Verbindung Nr. 1.01, 1.02, 1.03, 1.04, 1.05, 1.07, 1.08, 1.09, 1.10, 1.11, 1.14, 1.15 oder 1.16 enthielten, konnte der Pilzbefall weitgehend verhindert werden (0-15 %).

Pflanzen, die mit einer wäßrigen Aufbereitung der Vergleichsverbindung (Konzentration: 63 ppm) behandelt wurden, zeigten wie die unbehandelten Kontrollpflanzen dagegen 65 % Pilzbefall auf den Blättern.

### Anwendungsbeispiel 2

### Wirksamkeit gegen Pyricularia oryzae (protektiv)

Blätter von in Töpfen gewachsenen Reiskeimlingen der Sorte "Tai-Nong 67" wurden mit wäßrigen Emulsionen, die 80 Gew.-% Wirkstoff und 20 Gew.-% Emulgiermittel in der Trockensubstanz enthielten, tropfnaß besprüht und 24 Stunden später mit einer wäßrigen Sporensuspension von Pyricularia oryzae inokuliert. Anschließend stellte man die Versuchspflanzen in Klimakammern bei 22-24°C und 95-99 % relativer Luftfeuchtigkeit auf. Nach 6 Tagen wurde das Ausmaß des Krankheitsbefalls ermittelt.

Während die Blätter von unbehandelten Kontrollpflanzen zu 70 % von Pilz befallen waren, wiesen die mit wäßrigen Aufbereitungen [Konzentration: 63 ppm] der Verbindungen Nr. 1.01, 1.03, 1.04, 1.05, 1.07, 1.08, 1.10, 1.11, 1.13, 1.14, 1.15 und 1.16 behandelten Pflanzen nur 0-25 % Pilzbefall an den Blättern auf.

Durch Applikation einer wäßrigen Aufbereitung der Vergleichsverbindung [Konzentration: 63 ppm] konnte der Pilzbefall nicht wesentlich verringert werden (60 % Befall der Blätter).

### Anwendungsbeispiele (pestizide Wirksamkeit)

Die Wirkstoffe wurden als 10 gew.-%ige Emulsion in einem Gemisch aus 70 Gew.-% Cyclohexanol, 20 Gew.-% Nekanil® LN^{*)} und 10 Gew.-% Emulphor® EL^{**)} aufbereitet und entsprechend der gewünschten Konzentration mit Wasser verdünnt. Nach Abschluß der Versuche wurde die jeweils niedrigste Konzentration ermittelt, bei der die Verbindungen I im Vergleich zu unbehandelten Kontrollversuchen noch eine 80-100 %ige Mortalität hervorriefen (Wirkschwelle bzw. Minimalkonzentration).
^{*)} Lutensol®AP6 = Netzmittel mit Emulgier- und Dispergierwirkung auf der Basis ethoxylierter Alkylphenole
^{**)} Emulan®EL = Emulgator auf der Basis ethoxylierter Fettalkohole

### Anwendungsbeispiel 3 (insektizide Wirkung)

### Wirksamkeit gegen Nephotettix cincticeps (Grüne Reiszikade); Kontaktwirkung

Mit wäßrigen Wirkstoffaufbereitungen behandelte Rundfilter (⌀ 9 cm) wurden mit 5 adulten Zikaden belegt. 24 Std. später beurteilte man die Mortilität wie folgt:
- 100 %: = keine überlebenden Zikaden;
- 80 %: = 1 überlebende Zikade;
- 60 %: = 2 überlebende Zikaden;
- <60 %: = mindestens 3 überlebende Zikaden (= ohne Wirkung).

Die Wirkschwelle der Verbindungen Nr. 1.01, 1.02, 1.03, 1.04, 1.08, 1.09, 1.10, 1.11, 1.14, 1.15 und 1.16 lag bei maximal 0,4 mg pro Rundfilter.

### Anwendungsbeispiel 4 (insektizide Kontaktwirkung)

### Wirksamkeit gegen Prodenia litura (Ägypt. Baumwollwurm)

Mit wäßrigen Wirkstoffaufbereitungen behandelte Filter (⌀ ca. 9 cm) wurden mit 5 Raupen belegt. Nach 4 Std. erfolgte die erste Beurteilung. Sofern noch mindestens eine Raupte lebte, wurde eine Futtermischung folgender Zusammensetzung zugegeben.
- 20 l: Wasser;
- 800 g: Agar-Agar;
- 13 l: Wasser;
- 1370 g: Hefepulver (Hefe "Engevita");
- 5150 g: Maismehl;
- 1300 g: Weizenkeime;
- 200 g: Wessonsalz;
- 100 g: Cellulosepulver;
- 50 g: Sorbinsäure;
- 50 g: Nipagin;
- 10 g: Vitaminmischung aus
- 30 g: Vitamin B1,
- 60 g: Vitamin B2,
- 30 g: Vitamin B6,
- 120 g: Nikotinsäure/Niacin,
- 120 g: D-Pantothenat-Calcium,
- 30 g: Folsäure und
- 1,2 g: Biotin;
- 50 ml: Antibiotica (1 % in Alkohol);
- 180 g: Ascorbinsäure;
- 1,5 l: Wasser zum Einspülen der Vitamine und Ausspülen der Gefäße.

Nach 24 Stunden bestimmte man schließlich die Mortalität.

Die Wirkschwelle (= 4 Raupen tot) der Verbindungen Nr. 1.02, 1.04 und 1.05 lag bei maximal 0,4 mg pro Rundfilter.

### Anwendungsbeispiel 5 (akarizide Kontaktwirkung)

### Wirksamkeit gegen Tetranychus telarius (Bohnenspinnmilbe)

In Töpfen befindliche Buschbohnen, die das zweite Folgeblattpaar gebildet hatten und die stark mit den Spinnmilben befallen waren, wurden mit den wäßrigen Wirkstoffaufbereitungen tropfnaß gespritzt. Dazu besprühte man die Pflanzen auf einem Drehteller von allen Seiten mit ca. 30 ml der Spritzbrühe. Nach 5 Tagen im Gewächshaus wurde mittels Mikroskop (Binokular) der Bekämpfungserfolg in % festgestellt.

Bei diesem Test zeigten die Verbindungen Nr. 1.01 bis 1.16 Wirkschwellen von maximal 400 ppm.

## Patentansprüche

1. Substituierte Phenoxymethylphenyl-Derivate der Formel I 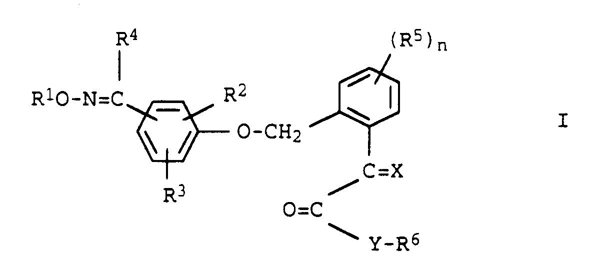 in der die Variablen die folgende Bedeutung haben:
X =CH-OCH₃, =CH-CH₃ oder =N-OCH₃;
R¹ C₁-C₆-Alkyl, C₂-C₆-Alkenyl, C₃-C₆-Alkinyl, C₁-C₆-Halogenalkyl, C₃-C₆-Halogenalkenyl, C₁-C₄-Alkoxy-C₁-C₆-alkyl, Cyano-C₁-C₆-alkyl, C₁-C₆-Alkoxycarbonyl-C₁-C₆-alkyl, eine C₃-C₆-Cycloalkyl- oder C₃-C₆-Cycloalkyl-C₁-C₄-alkylgruppe, eine ArylC₁-C₆-alkyl- oder Aryl-C₃-C₆-alkenyl- oder Aryloxy-C₁-C₆-alkylgruppe, eine gesättigte oder ungesättigte 4- bis 6-gliedrige Heterocyclyl- oder Heterocyclyl-C₁-C₄-alkylgruppe oder eine Heteroaryl-C₁-C₆-alkylgruppe, wobei die heterocyclischen Ringe neben C-Atomen jeweils ein oder zwei Ringglieder enthalten, die ausgewählt sind aus der Gruppe bestehend aus einem Sauerstoff- oder Schwefelatom und ein oder zwei Stickstoffatomen und ein oder zwei Gruppen -N(CH₃)-,
wobei die carbocyclischen und heterocyclischen Ringe jeweils ihrerseits einen oder mehrere Reste tragen können, ausgewählt aus der Gruppe bestehend aus: Halogen, C₁-C₄-Alkyl, C₁-C₂-Halogenalkyl, C₃-C₆-Cycloalkyl, C₁-C₄-Alkoxy, C₁-C₂-Halogenalkoxy, Halogen, Aryl und Aryloxy;
R², R³ Wasserstoff, Halogen, Cyano, Nitro, C₁-C₄-Alkyl, C₁-C₂-Halogenalkyl, C₁-C₄-Alkoxy, C₁-C₂-Halogenalkoxy, oder, sofern R² und R³ benachbart sind, zusammen einen Oxymethylidenoxy- oder Oxyethylidenoxy-Brücke, wobei jedes C-Atom dieser Brücken gewünschtenfalls ein oder zwei Halogenatome und/oder Methylreste tragen kann;
R⁴ Cyano, Chlor, Brom, C₁-C₆-Alkoxy, C₁-C₆-Alkylthio, C₁-C₄-Halogenalkoxy, C₁-C₄-Alkoxy-C₁-C₄-alkoxy, C₃-C₆-Cycloalkyl-C₁-C₂-alkoxy, C₁-C₆-Alkylamino, Di-(C₁-C₆-alkyl)amino, C₃-C₆-Alkenyloxy, C₃-C₆-Alkinyloxy, Aryloxy, Arylthio wobei die aromatischen Ringe ein bis drei Reste tragen können, ausgewählt aus der Gruppe bestehend aus C₁-C₄-Alkyl, C₁-C₄-Halogenalkyl, C₁-C₄-Alkoxy, C₁-C₄-Halogenalkoxy und C₁-C₄-Alkylthio, und wobei die aromatischen Ringe zusätzlich so viele Halogenatome tragen können, daß die Gesamtzahl der Reste 4 oder 5 beträgt;
R⁵ Nitro, Cyano, Halogen, C₁-C₄-Alkyl, C₁-C₄-Halogenalkyl, C₁-C₄-Alkoxy, C₁-C₄-Halogenalkoxy, C₁-C₄-Alkylthio, Phenyl oder Phenoxy, wobei die aromatischen Ringe ein bis drei Reste tragen können, ausgewählt aus der Gruppe bestehend aus C₁-C₄-Alkyl, C₁-C₄-Halogenalkyl, C₁-C₄-Alkoxy, C₁-C₄-Halogenalkoxy und C₁-C₄-Alkylthio, und wobei die aromatischen Ringe zusätzlich so viele Halogenatome tragen können, daß die Gesamtzahl der Reste 4 oder 5 beträgt;
oder, sofern n für 2, 3, oder 4 steht, eine an zwei benachbarte C-Atome des Grundkörpers annellierte 1,3-Butadien-1,4-diylgruppe oder eine mono- oder dihalogenierte 1,3-Butadien-1,4-diylgruppe, wobei diese annellierten Ringe ihrerseits ein oder zwei Reste tragen können, ausgewählt aus der Gruppe Nitro, Cyano, Halogen, C₁-C₄-Halogenalkyl, C₁-C₄-Alkoxy, C₁-C₄-Halogenalkoxy und C₁-C₄-Alkylthio;
n 0, 1, 2, 3 oder 4, wobei die Reste R⁵ gleich oder verschieden sein können, wenn n für 2, 3 oder 4 steht;
Y Sauerstoff, -NH- oder -N(CH₃)-;
R⁶ Wasserstoff oder C₁-C₄-Alkyl.

2. Verfahren zur Herstellung von substituierten Phenoxymethylphenyl-Derivaten der Formel I gemäß Anspruch I, wobei Y für Sauerstoff steht, dadurch gekennzeichnet, daß man Phenole der Formel II 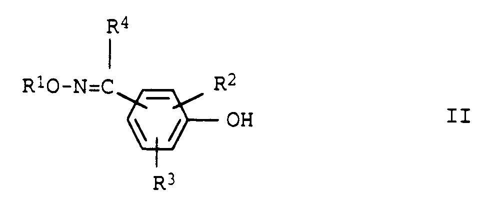 mit Phenylverbindungen der Formel III, 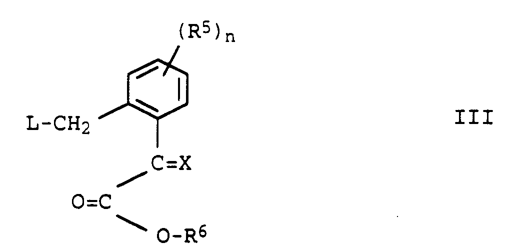 in denen L für eine Abgangsgruppe steht, umsetzt.

3. Verfahren zur Herstellung von substituierten Phenoxymethylphenyl-Derivaten der Formel I gemäß Anspruch I, wobei Y für -NH- oder -N(CH₃)- steht, dadurch gekennzeichnet, daß man Phenoxymethylphenyl-Derivate der Formel I, in denen Y für Sauerstoff steht, oder Phenylbenzylether der Formel VI 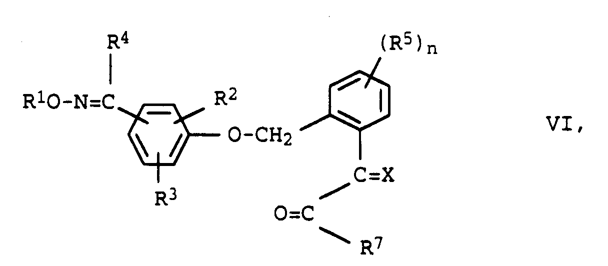 wobei R⁷ für einen Carbonyl-aktivierenden Rest steht, mit einem Amin der Formel H₂N-R⁶ oder HN(CH₃)-R⁶ umsetzt.

4. Verfahren zur Herstellung von Phenylglyoxylsäure-Derivaten der allgemeinen Formel XI' 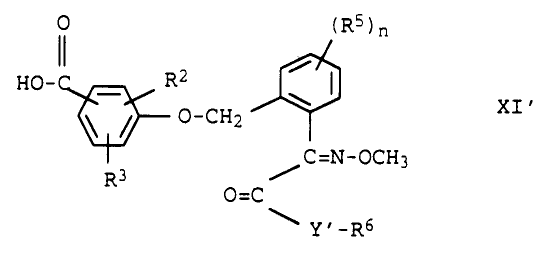 wobei Y' für -NH- oder -N(CH₃)-steht,
dadurch gekennzeichnet, daß man Acetophenone der allgemeinen Formel X' 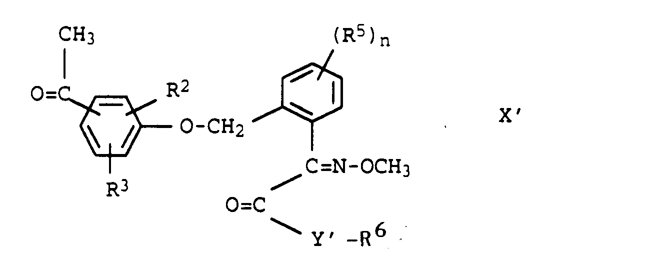 der Haloformreaktion unterwirft.

5. α-Methoxyimino-phenylessigsäuremethylamide der Formel XI' 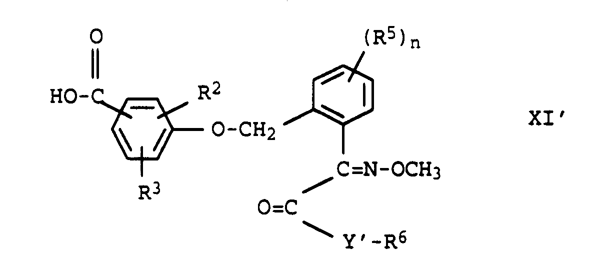 in der die Variablen die folgende Bedeutung haben:
Y' -NH- oder -N(CH₃)-;
R² und R³ unabhängig voneinander Wasserstoff, Halogen, Cyano, Nitro, C₁-C₄-Alkyl, C₁-C₂-Halogenalkyl, C₁-C₄-Alkoxy, C₁-C₂-Halogenalkoxy;
R⁵ Nitro, Cyano, Halogen, C₁-C₄-Alkyl, C₁-C₄-Halogenalkyl, C₁-C₄-Alkoxy, C₁-C₄-Halogenalkoxy, C₁-C₄-Alkylthio, Phenyl oder Phenoxy, wobei die aromatischen Ringe ein bis drei Reste tragen können, ausgewählt aus der Gruppe bestehend aus C₁-C₄-Alkyl, C₁-C₄-Halogenalkyl, C₁-C₄-Alkoxy, C₁-C₄-Halogenalkoxy und C₁-C₄-Alkylthio, und wobei die aromatischen Ringe zusätzlich so viele Halogenatome tragen können, daß die Gesamtzahl der Reste 4 oder 5 beträgt;
oder, sofern n für 2, 3, oder 4 steht, eine an zwei benachbarte C-Atome des Grundkörpers annellierte 1,3-Butadien-1,4-diylgruppe oder eine mono- oder dichlorierte 1,3-Butadien-1,4-diylgruppe, wobei diese annellierten Ringe ihrerseits ein oder zwei Reste tragen kann, ausgewählt aus der Gruppe Nitro, Cyano, C₁-C₄-Halogenalkyl, C₁-C₄-Alkoxy, C₁-C₄-Halogenalkoxy und C₁-C₄-Alkylthio;
n 0, 1, 2, 3 oder 4, wobei die Reste R⁵ gleich oder verschieden sein können, wenn n für 1, 2, 3 oder 4 steht;
R⁶ Wasserstoff oder C₁-C₄-Alkyl.

6. 2-(Phenoxymethyl)-phenylessigsäure-Derivate der Formel IVb' 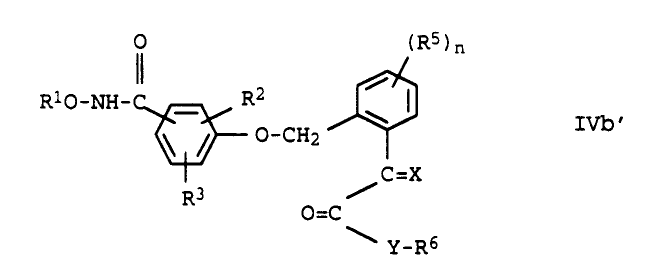 in der die Variablen die folgende Bedeutung haben:
X =CH-OCH₃, =CH-CH₃ oder =N-OCH₃;
Y Sauerstoff, -NH- oder -N(CH₃)-;
R¹ C₁-C₆-Alkyl, C₂-C₆-Alkenyl, C₃-C₆-Alkinyl, C₁-C₆-Halogenalkyl, C₃-C₆-Halogenalkenyl, C₁-C₄-Alkoxy-C₁-C₆-alkyl, Cyano-C₁-C₆-alkyl, C₁-C₆-Alkoxycarbonyl-C₁-C₆-alkyl, eine C₃-C₆-Cycloalkyl- oder C₃-C₆-Cycloalkyl-C₁-C₄-alkylgruppe, eine Aryl-C₁-C₆-alkyl- oder Aryl-C₃-C₆-alkeny1- oder Aryloxy-C₁-C₆-alkylgruppe, eine gesättigte oder ungesättigte 4- bis 6-gliedrige Heterocyclyl- oder Heterocyclyl-C₁-C₄-alkylgruppe oder eine Heteroaryl-C₁-C₆-alkylgruppe, wobei die heterocyclischen Ringe neben C-Atomen jeweils ein oder zwei Ringglieder enthalten, die ausgewählt sind aus der Gruppe bestehend aus einem Sauerstoff- oder Schwefelatom und ein oder zwei Stickstoffatomen und ein oder zwei Gruppen -N(CH₃)-,
wobei die carbocyclischen und heterocyclischen Ringe jeweils ihrerseits einen oder mehrere Reste tragen können, ausgewählt aus der Gruppe bestehend aus: Halogen, C₁-C₄-Alkyl, C₁-C₂-Halogenalkyl, C₃-C₆-Cycloalkyl, C₁-C₄-Alkoxy, C₁-C₂-Halogenalkoxy, Halogen, Aryl und Aryloxy;
R², R³ Wasserstoff, Halogen, Cyano, Nitro, C₁-C₄-Alkyl, C₁-C₂-Halogenalkyl, C₁-C₄-Alkoxy, C₁-C₂-Halogenalk-oxy, oder, sofern R² und R³ benachbart sind, zusammen einen Oxymethylidenoxy- oder Oxy-ethylidenoxy-Brücke, wobei jedes C-Atom dieser Brücken gewünschtenfalls ein oder zwei Halogenatome und/oder Methylreste tragen kann;
R⁵ Nitro, Cyano, Halogen, C₁-C₄-Alkyl, C₁-C₄-Halogenalkyl, C₁-C₄-Alkoxy, C₁-C₄-Halogenalkoxy, C₁-C₄-Alkylthio, Phenyl oder Phenoxy, wobei die aromatischen Ringe ein bis drei Reste tragen können, ausgewählt aus der Gruppe bestehend aus C₁-C₄-Alkyl, C₁-C₄-Halogenalkyl, C₁-C₄-Alkoxy, C₁-C₄-Halogenalkoxy und C₁-C₄-Alkylthio, und wobei die aromatischen Ringe zusätzlich so viele Halogenatome tragen können, daß die Gesamtzahl der Reste 4 oder 5 beträgt;
oder, sofern n für 2, 3, oder 4 steht, eine an zwei benachbarte C-Atome des Grundkörpers annellierte 1,3-Butadien-1,4-diylgruppe oder eine mono- oder dihalogenierte 1,3-Butadien-1,4-diylgruppe, wobei diese annellierten Ringe ihrerseits ein oder zwei Reste tragen können, ausgewählt aus der Gruppe Nitro, Cyano, C₁-C₄-Halogenalkyl, C₁-C₄-Alkoxy, C₁-C₄-Halogenalkoxy und C₁-C₄-Alkylthio;
n 0, 1, 2, 3 oder 4, wobei die Reste R⁵ gleich oder verschieden sein können, wenn n für 1, 2, 3 oder 4 steht;
R⁶ Wasserstoff oder C₁-C₄-Alkyl.

7. Phenoxymethylphenyl-Derivate der Formel I nach Anspruch 1, wobei
n für 0 oder 1;
X für =CH-OCH₃, =CH-CH₃ oder =N-OCH₃;
Y für Sauerstoff oder -NH-;
R¹ für C₁-C₆-Alkyl, C₂-C₆-Alkenyl, C₃-C₆-Alkinyl, C₁-C₆-Halogenalkyl oder C₃-C₆-Halogenalkenyl;
R² für Cyano, Chlor, Fluor, Methyl, Trifluormethyl oder Methoxy;
R³ für Wasserstoff, Cyano, Chlor, Fluor, Methyl oder Methoxy;
R⁴ für Cyano, Chlor, Brom, C₁-C₆-Alkoxy, C₁-C₆-Alkylthio, C₃-C₆-Alkenyloxy, Aryloxy oder Arylthio und
R⁵ für Cyano, Halogen, C₁-C₆-Alkyl, C₁-C₆-Halogenalkyl, C₁-C₆-Alkoxy oder Phenyl
stehen.

8. Phenoxymethylphenyl-Derivate der Formel I nach Anspruch 1, wobei
n für 0 oder 1;
X für =NO-CH₃;
Y für -NH-;
R¹ für C₁-C₆-Alkyl, C₂-C₆-Alkenyl, C₃-C₆-Alkinyl, C₁-C₆-Halogenalkyl oder C₃-C₆-Halogenalkenyl;
R² für Cyano, Chlor, Fluor, Methyl, Trifluormethyl oder Methoxy;
R³ für Wasserstoff, Cyano, Chlor, Fluor, Methyl oder Methoxy;
R⁴ für Cyano, Chlor, Brom, C₁-C₆-Alkoxy, C₁-C₆-Alkylthio, C₃-C₆-Alkenyloxy, Phenyloxy oder Phenylthio; und
R⁵ für Cyano, Chlor, Fluor, Methyl, Trifluormethyl, Methoxy oder Phenyl
stehen.

9. Verwendung der Phenoxymethylphenyl-Derivate der Formel I gemäß Anspruch 1 als Fungizide.

10. Verwendung der Phenoxymethylphenyl-Derivate der Formel I gemäß Anspruch 1 zur Bekämpfung von Schädlingen.

11. Fungizides Mittel, enthaltend feste und/oder flüssige Trägerstoffe und eine fungizid wirksame Menge mindestens eines substituierten Phenoxymethylphenyl-Derivats der Formel I gemäß Anspruch 1.

12. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge mindestens eines substituierten Phenoxymethylphenyl-Derivats der Formel I gemäß Anspruch 1 behandelt.

13. Mittel zur Bekämpfung von Schädlingen, enthaltend inerte Zusatzstoffe und eine pestizid wirksame Menge mindestens eines substituierten Phenoxymethylphenyl-Derivats der Formel I gemäß Anspruch I.

14. Verfahren zur Bekämpfung von Schädlingen, dadurch gekennzeichnet, daß man die Schädlinge und/oder deren Lebensraum mit einer pestizid wirksamen Menge mindestens eines substituierten Phenoxymethylphenyl-Derivats der Formel I gemäß Anspruch I behandelt.

## Claims

1. A substituted phenoxymethylphenyl derivative of the formula I 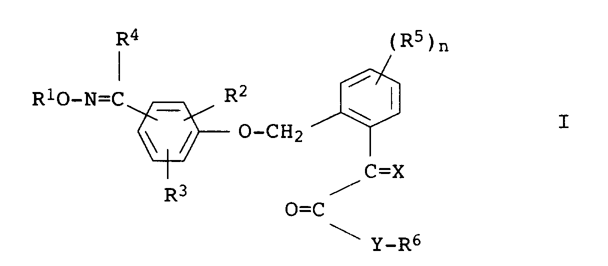 where the variables have the following meanings:
X is =CH-OCH₃, =CH-CH₃ or =N-OCH₃;
R¹ is C₁-C₆-alkyl, C₂-C₆-alkenyl, C₃-C₆-alkynyl, C₁-C₆-haloalkyl, C₃-C₆-haloalkenyl, C₁-C₄-alkoxy-C₁-C₆-alkyl, cyan-C₁-C₆-alkyl, C₁-C₆-alkoxycarbonyl-C₁-C₆-alkyl, a C₃-C₆-cycloalkyl or C₃-C₆-cycloalkyl-C₁-C₄-alkyl group, an aryl-C₁-C₆-alkyl or aryl-C₃-C₆-alkenyl or aryloxy-C₁-C₆-alkyl group, a saturated or unsaturated 4- to 6-membered heterocyclyl or heterocyclyl-C₁-C₄-alkyl group or a heteroaryl-C₁-C₆-alkyl group, the heterocyclic rings in addition to C atoms in each case containing one or two ring members which are selected from the group consisting of an oxygen or sulfur atom and one or two nitrogen atoms and one or two groups -N(CH₃)-, it being possible for the carbocyclic and heterocyclic rings in each case in turn to carry one or more radicals selected from the group consisting of: halogen, C₁-C₄-alkyl, C₁-C₂-haloalkyl, C₃-C₆-cycloalkyl, C₁-C₄-alkoxy, C₁-C₂-haloalkoxy, halogen, aryl and aryloxy;
R² and R³ are hydrogen, halogen, cyano, nitro, C₁-C₄-alkyl, C₁-C₂-haloalkyl, C₁-C₄-alkoxy, C₁-C₂-haloalkoxy, or, if R² and R³ are adjacent, together are an oxymethylidenoxy or oxyethylidenoxy bridge, it being possible for each C atom of these bridges if desired to carry one or two halogen atoms and/or methyl radicals;
R⁴ is cyano, chlorine, bromine, C₁-C₆-alkoxy, C₁-C₆-alkylthio, C₁-C₄-haloalkoxy, C₁-C₄-alkoxy-C₁-C₄-alkoxy, C₃-C₆-cycloalkyl-C₁-C₂-alkoxy, C₁-C₆-alkylamino, di-(C₁-C₆-alkyl)amino, C₃-C₆-alkenyloxy, C₃-C₆-alkynyloxy, aryloxy, arylthio, it being possible for the aromatic rings to carry one to three radicals selected from the group consisting of C₁-C₄-alkyl, C₁-C₄-haloalkyl, C₁-C₄-alkoxy, C₁-C₄-haloalkoxy and C₁-C₄-alkylthio, and it being possible for the aromatic rings additionally to carry sufficient halogen atoms such that the total number of radicals is 4 or 5;
R⁵ is nitro, cyano, halogen, C₁-C₄-alkyl, C₁-C₄-haloalkyl, C₁-C₄-alkoxy, C₁-C₄-haloalkoxy, C₁-C₄-alkylthio, phenyl or phenoxy, it being possible for the aromatic rings to carry one to three radicals selected from the group consisting of C₁-C₄-alkyl, C₁-C₄-haloalkyl, C₁-C₄-alkoxy, C₁-C₄-haloalkoxy and C₁-C₄-alkylthio, and it being possible for the aromatic rings additionally to carry sufficient halogen atoms such that the total number of radicals is 4 or 5;
or, if n is 2, 3, or 4, is a 1,3-butadiene-1,4-diyl group or a mono- or dihalogenated 1,3-butadiene-1,4-diyl group fused to two adjacent C atoms of the parent substance, it being possible for these fused rings in turn to carry one or two radicals selected from the group consisting of nitro, cyano, halogen, C₁-C₄-haloalkyl, C₁-C₄-alkoxy, C₁-C₄-haloalkoxy and C₁-C₄-alkylthio;
n is 0, 1, 2, 3 or 4, it being possible for the radicals R⁵ to be identical or different if n is 2, 3 or 4;
Y is oxygen, -NH- or -N(CH₃)-;
R⁶ is hydrogen or C₁-C₄-alkyl.

2. A process for preparing substituted phenoxymethylphenyl derivatives of the formula I as claimed in claim 1, where Y is oxygen, which comprises reacting phenols of the formula II 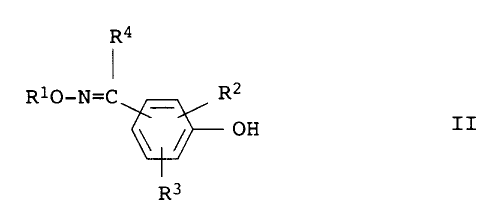 with phenyl compounds of the formula III 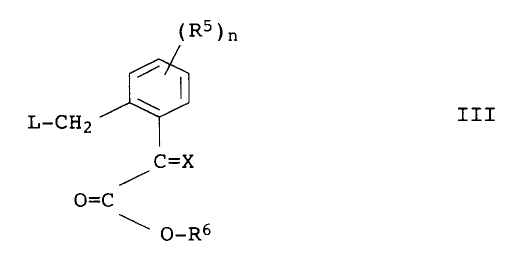 where L is a leaving group.

3. A process for preparing substituted phenoxymethylphenyl derivatives of the formula I as claimed in claim 1, where Y is -NH- or -N(CH₃)-, which comprises reacting phenoxymethylphenyl derivatives of the formula I in which Y is oxygen, or phenyl benzyl ethers of the formula VI 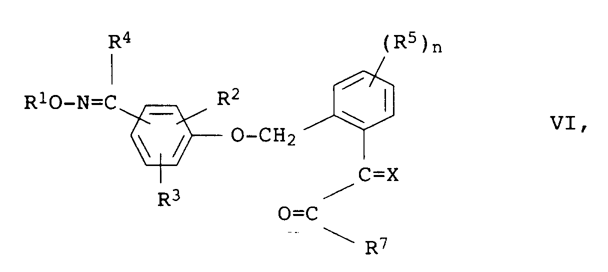 where R⁷ is a carbonyl-activating radical, with an amine of the formula H₂N-R⁶ or HN(CH₃)-R⁶.

4. A process for preparing phenylglyoxylic acid derivatives of the general formula XI' 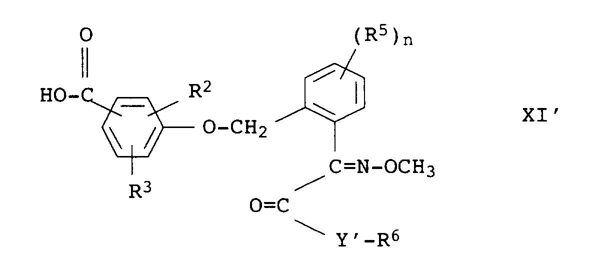 where Y' is -NH- or -N(CH₃)-,
which comprises subjecting acetophenones of the general formula X' 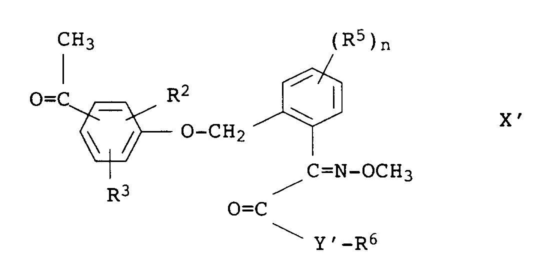 to the haloform reaction.

5. An α-methoxyiminophenylacetic acid methylamide of the formula XI' 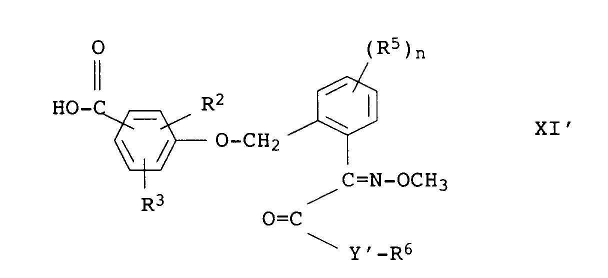 where the variables have the following meanings:
Y' is -NH- or -N(CH₃)-;
R² and R³ independently of one another are hydrogen, halogen, cyano, nitro, C₁-C₄-alkyl, C₁-C₂-haloalkyl, C₁-C₄-alkoxy, C₁-C₂-haloalkoxy;
R⁵ is nitro, cyano, halogen, C₁-C₄-alkyl, C₁-C₄-haloalkyl, C₁-C₄-alkoxy, C₁-C₄-haloalkoxy, C₁-C₄-alkylthio, phenyl or phenoxy, it being possible for the aromatic rings to carry one to three radicals selected from the group consisting of C₁-C₄-alkyl, C₁-C₄-haloalkyl, C₁-C₄-alkoxy, C₁-C₄-haloalkoxy and C₁-C₄-alkylthio, and it being possible for the aromatic rings additionally to carry sufficient halogen atoms such that the total number of radicals is 4 or 5;
or, if n is 2, 3, or 4, is a 1,3-butadiene-1,4-diyl group or a mono- or dichlorinated 1,3-butadiene-1,4-diyl group fused to two adjacent C atoms of the parent substance, it being possible for these fused rings in turn to carry one or two radicals selected from the group consisting of nitro, cyano, C₁-C₄-haloalkyl, C₁-C₄-alkoxy, C₁-C₄-haloalkoxy and C₁-C₄-alkylthio;
n is 0, 1, 2, 3 or 4, it being possible for the radicals R⁵ to be identical or different if n is 1, 2, 3 or 4;
R⁶ is hydrogen or C₁-C₄-alkyl.

6. A 2-(phenoxymethyl)phenylacetic acid derivative of the formula IVb' 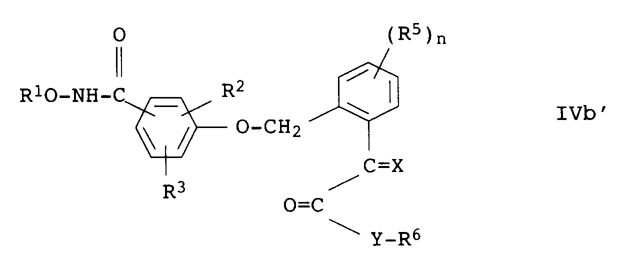 where the variables have the following meanings:
X is =CH-OCH₃, =CH-CH₃ or =N-OCH₃;
Y is oxygen, -NH- or -N(CH₃)-;
R¹ is C₁-C₆-alkyl, C₂-C₆-alkenyl, C₃-C₆-alkynyl, C₁-C₆-haloalkyl, C₃-C₆-haloalkenyl, C₁-C₄-alkoxy-C₁-C₆-alkyl, cyan-C₁-C₆-alkyl, C₁-C₆-alkoxycarbonyl-C₁-C₆-alkyl, a C₃-C₆-cycloalkyl or C₃-C₆-cycloalkyl-C₁-C₄-alkyl group, an aryl-C₁-C₆-alkyl or aryl-C₃-C₆-alkenyl or aryloxy-C₁-C₆-alkyl group, a saturated or unsaturated 4- to 6-membered heterocyclyl or heterocyclyl-C₁-C₄-alkyl group or a heteroaryl-C₁-C₆-alkyl group, the heterocyclic rings in addition to C atoms in each case containing one or two ring members which are selected from the group consisting of an oxygen or sulfur atom and one or two nitrogen atoms and one or two groups -N(CH₃)-,
it being possible for the carbocyclic and heterocyclic rings in each case in turn to carry one or more radicals selected from the group consisting of: halogen, C₁-C₄-alkyl, C₁-C₂-haloalkyl, C₃-C₆-cycloalkyl, C₁-C₄-alkoxy, C₁-C₂-haloalkoxy, halogen, aryl and aryloxy;
R² and R³ are hydrogen, halogen, cyano, nitro, C₁-C₄-alkyl, C₁-C₂-haloalkyl, C₁-C₄-alkoxy, C₁-C₂-haloalkoxy, or, if R² and R³ are adjacent, together are an oxymethylidenoxy or oxyethylidenoxy bridge, it being possible for each C atom of these bridges if desired to carry one or two halogen atoms and/or methyl radicals;
R⁵ is nitro, cyano, halogen, C₁-C₄-alkyl, C₁-C₄-haloalkyl, C₁-C₄-alkoxy, C₁-C₄-haloalkoxy, C₁-C₄-alkylthio, phenyl or phenoxy, it being possible for the aromatic rings to carry one to three radicals selected from the group consisting of C₁-C₄-alkyl, C₁-C₄-haloalkyl, C₁-C₄-alkoxy, C₁-C₄-haloalkoxy and C₁-C₄-alkylthio, and it being possible for the aromatic rings additionally to carry sufficient halogen atoms such that the total number of radicals is 4 or 5;
or, if n is 2, 3, or 4, is a 1,3-butadiene-1,4-diyl group or a mono- or dihalogenated 1,3-butadiene-1,4-diyl group fused to two adjacent C atoms of the parent substance, it being possible for these fused rings in turn to carry one or two radicals selected from the group consisting of nitro, cyano, C₁-C₄-haloalkyl, C₁-C₄-alkoxy, C₁-C₄-haloalkoxy and C₁-C₄-alkylthio;
n is 0, 1, 2, 3 or 4, it being possible for the radicals R⁵ to be identical or different if n is 1, 2, 3 or 4;
R⁶ is hydrogen or C₁-C₄-alkyl.

7. A phenoxymethylphenyl derivative of the formula I as claimed in claim 1, where
n is 0 or 1;
X is =CH-OCH₃, =CH-CH₃ or =N-OCH₃;
Y is oxygen or -NH-;
R¹ is C₁-C₆-alkyl, C₂-C₆-alkenyl, C₃-C₆-alkynyl, C₁-C₆-haloalkyl or C₃-C₆-haloalkenyl;
R² is cyano, chlorine, fluorine, methyl, trifluoromethyl or methoxy;
R³ is hydrogen, cyano, chlorine, fluorine, methyl or methoxy;
R⁴ is cyano, chlorine, bromine, C₁-C₆-alkoxy, C₁-C₆-alkylthio, C₃-C₆-alkenyloxy, aryloxy or arylthio, and
R⁵ is cyano, halogen, C₁-C₆-alkyl, C₁-C₆-haloalkyl, C₁-C₆-alkoxy or phenyl.

8. A phenoxymethylphenyl derivative of the formula I as claimed in claim 1, where
n is 0 or 1;
X is =NO-CH₃;
Y is -NH-;
R¹ is C₁-C₆-alkyl, C₂-C₆-alkenyl, C₃-C₆-alkynyl, C₁-C₆-haloalkyl or C₃-C₆-haloalkenyl;
R² is cyano, chlorine, fluorine, methyl, trifluoromethyl or methoxy;
R³ is hydrogen, cyano, chlorine, fluorine, methyl or methoxy;
R⁴ is cyano, chlorine, bromine, C₁-C₆-alkoxy, C₁-C₆-alkylthio, C₃-C₆-alkenyloxy, phenyloxy or phenylthio; and
R⁵ is cyano, chlorine, fluorine, methyl, trifluoromethyl, methoxy or phenyl.

9. The use of the phenoxymethylphenyl derivatives of the formula I as claimed in claim 1 as fungicides.

10. The use of the phenoxymethylphenyl derivatives of the formula I as claimed in claim 1 for controlling pests.

11. A fungicidal composition containing solid and/or liquid carriers and a fungicidally active amount of at least one substituted phenoxymethylphenyl derivative of the formula I as claimed in claim 1.

12. A method for controlling fungi, which comprises treating the fungi or the materials, plants, seeds or the soil threatened by fungal attack with a fungicidally active amount of at least one substituted phenoxymethylphenyl derivative of the formula I as claimed in claim 1.

13. A composition for controlling pests, containing inert additives and a pesticidally active amount of at least one substituted phenoxymethylphenyl derivative of the formula I as claimed in claim 1.

14. A method for controlling pests, which comprises treating the pests and/or their habitat with a pesticidally active amount of at least one substituted phenoxymethylphenyl derivative of the formula I as claimed in claim 1.

## Revendications

1. Dérivés phénoxyméthylphényliques substitués de formule I 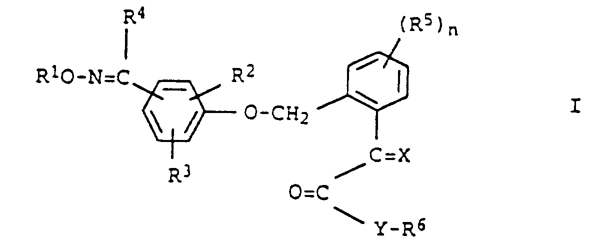 dans laquelle les symboles ont les significations suivantes :
X =CH-OCH₃, =CH-CH₃ ou =N-OCH₃ ;
R¹ un groupe alkyle en C1-C6, alcényle en C2-C6, alcynyle en C3-C6, halogénoalkyle en C1-C6, halogénoalcényle en C3-C6, (alcoxy en C1-C4)alkyle en C1-C6, cyano-alkyle en C1-C6, (alcoxy en C1-C6)carbonyle-alkyle en C1-C6, un groupe cycloalkyle en C3-C6 ou (cycloalkyle en C3-C6)alkyle en C1-C4, un groupe aryl-alkyle en C1-C6 ou aryl-alcényle en C3-C6 ou aryloxy-alkyle en C1-C6, un groupe hétérocycle ou hétérocyclyl-alkyle en C1-C4, saturé ou insaturé, de quatre à six chaînons, ou un groupe hétéroaryl-alkyle en C1-C6, les hétérocycles pouvant contenir chacun, en plus des atomes de carbone, un ou deux chaînons cycliques choisis dans le groupe consistant en un atome d'oxygène ou de soufre et un ou deux atomes d'azote et un ou deux groupes -N(CH₃)-,
les noyaux carbocycliques et hétérocycliques pouvant porter chacun un ou plusieurs substituants choisis parmi les suivants : halogéno, alkyle en C1-C4, halogénoalkyle en C1-C2, cycloalkyle en C3-C6, alcoxy en C1-C4, halogénoalcoxy en C1-C2, halogéno, aryle et aryloxy ;
R², R³ l'hydrogène, un halogène, un groupe cyano, nitro, alkyle en C1-C4, halogénoalkyle en C1-C2, alcoxy en C1-C4, halogénoalcoxy en C1-C2 ou bien, lorsque R² et R³ sont voisins, ils représentent ensemble un pont oxyméthylidène-oxy ou oxyéthylidène-oxy, chaque atome de carbone de ces ponts pouvant porter, si on le désire, un ou deux atomes d'halogènes et/ou un ou deux groupes méthyle ;
R⁴ un groupe cyano, le chlore, le brome, un groupe alcoxy en C1-C6, alkylthio en C1-C6, halogénoalcoxy en C1-C4, (alcoxy en C1-C4)-alcoxy en C1-C4, (cycloalkyle en C3-C6)alcoxy en C1-C2, alkylamino en C1-C6, di-(alkyle en C1-C6)amino, alcényloxy en C3-C6, alcynyloxy en C3-C6, aryloxy, arylthio, les noyaux aromatiques pouvant porter un à trois substituants choisis parmi les groupes alkyle en C1-C4, halogénoalkyle en C1-C4, alcoxy en C1-C4, halogénoalcoxy en C1-C4 et alkylthio en C1-C4, et les noyaux aromatiques pouvant encore porter des atomes d'halogènes en quantité telle que le nombre total des substituants soit de 4 ou 5 ;
R⁵ un groupe nitro, cyano, un halogène, un groupe alkyle en C1-C4, halogénoalkyle en C1-C4, alcoxy en C1-C4, halogénoalcoxy en C1-C4, alkylthio en C1-C4, phényle ou phénoxy, les noyaux aromatiques pouvant porter un à trois substituants choisis parmi les groupes alkyle en C1-C4, halogénoalkyle en C1-C4, alcoxy en C1-C4, halogénoalcoxy en C1-C4 et alkylthio en C1-C4, et les noyaux aromatiques pouvant encore porter des atomes d'halogènes en quantité telle que le nombre total des substituants soit de 4 ou 5 ; ou bien, lorsque n est égal à 2, 3 ou 4, un groupe 1,3-butandièn-1,4-diyle ou un groupe 1,3-butandièn-1,4-diyle mono- ou di-halogéné, condensé sur deux atomes de carbone voisins du corps fondamental, ces cycles condensés pouvant eux-mêmes porter un ou deux substituants choisis parmi les groupes nitro, cyano, les halogènes, les groupes halogénoalkyle en C1-C4, alcoxy en C1-C4, halogénoalcoxy en C1-C4 et alkylthio en C1-C4 ;
n est égal à 0, 1, 2, 3 ou 4, les symboles R⁵ pouvant avoir des significations identiques ou différentes lorsque n est égal à 2, 3 ou 4 ;
Y représente l'oxygène, un groupe -NH- ou -N(CH₃)- ;
R⁶ représente l'hydrogène ou un groupe alkyle en C1-C4.

2. Procédé de préparation des dérivés phénoxyméthylphényliques substitués de formule I selon la revendication 1 dans laquelle Y représente l'oxygène, caractérisé par le fait que l'on fait réagir des phénols de formule II 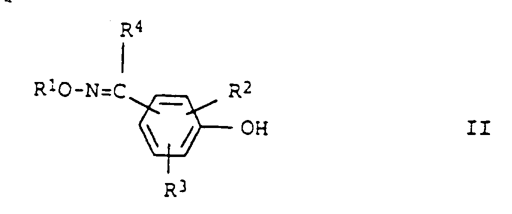 avec des dérivés phényliques de formule III 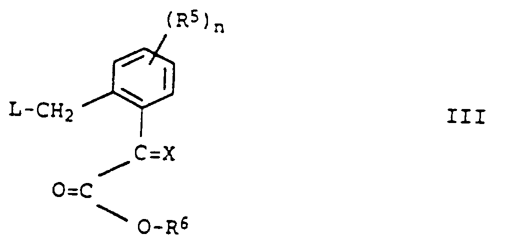 dans laquelle L représente un groupe éliminable.

3. Procédé de préparation des dérivés phénoxyméthylphényliques substitués de formule I selon la revendication 1 dans laquelle Y représente -NH- ou -N(CH₃)-, caractérisé par le fait que l'on fait réagir des dérivés phénoxyméthylphényliques de formule I dans laquelle Y représente l'oxygène, ou des éthers phénylbenzyliques de formule VI 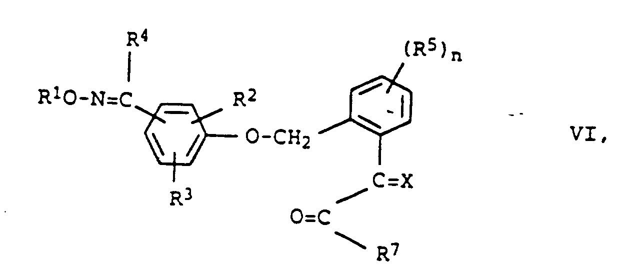 dans laquelle R⁷ représente un substituant activant le groupe carbonyle, avec une amine de formule H₂N-R⁶ ou HN(CH₃)-R⁶.

4. Procédé de préparation des dérivés de l'acide phénylglyoxylique de formule générale XI' 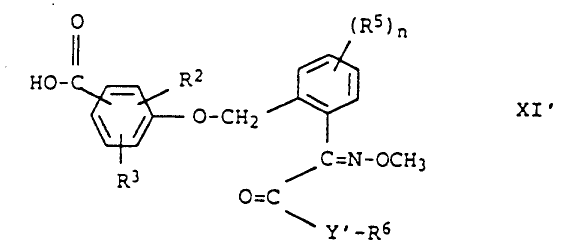 dans laquelle Y' représente -HN- ou -N(CH₃)-,
caractérisé par le fait que l'on soumet des acétophénones de formule générale X' 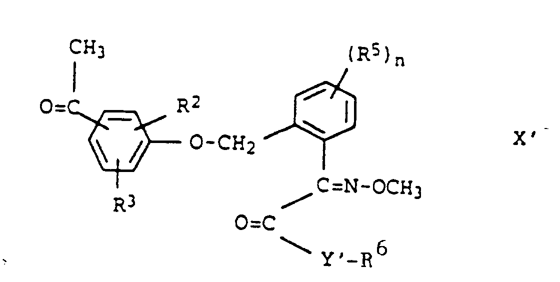 à la réaction d'halogénoformation.

5. Alpha-méthoxyimino-phénylacétométhylamides de formule XI' 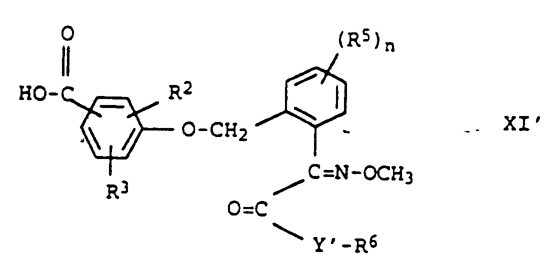 dans laquelle les symboles ont les significations suivantes :
Y' représente -HN- ou -N(CH₃)- ;
R² et R³ représentent chacun, indépendamment l'un de l'autre,
l'hydrogène, un halogène, un groupe cyano, nitro, alkyle en C1-C4, halogénoalkyle en C1-C2, alcoxy en C1-C4, halogénoalcoxy en C1-C2 ;
R⁵ représente un groupe nitro, cyano, un halogène, un groupe alkyle en C1-C4, halogénoalkyle en C1-C4, alcoxy en C1-C4, halogénoalcoxy en C1-C4, alkylthio en C1-C4, phényle ou phénoxy dont les cycles aromatiques peuvent porter un à trois substituants choisis parmi les groupes alkyle en C1-C4, halogénoalkyle en C1-C4, alcoxy en C1-C4, halogénoalcoxy en C1-C4 et alkylthio en C1-C4, les noyaux aromatiques pouvant en outre porter des atomes d'halogènes en quantité telle que le nombre total des substituants soit de 4 ou 5 ; ou bien, lorsque n est égal à 2, 3 ou 4, un groupe 1,3-butadièn-1,4-diyle ou un groupe 1,3-butadièn-1,4-diyle mono- ou di-chloré, condensé sur deux atomes de carbone voisins du corps fondamental, ces cycles condensés pouvant eux-mêmes porter un ou deux substituants choisis parmi les groupes nitro, halogénoalkyle en C1-C4, alcoxy en C1-C4, halogénoalcoxy en C1-C4 et alkylthio en C1-C4 ;
n est égal à 0, 1, 2, 3, 4, les symboles R⁵ pouvant avoir des significations identiques ou différentes lorsque n est égal à 1, 2, 3 ou 4 ;
R⁶ représente l'hydrogène ou un groupe alkyle en C1-C4.

6. Dérivés de l'acide 2-(phénoxyméthyl)phénylacétique de formule IV'b 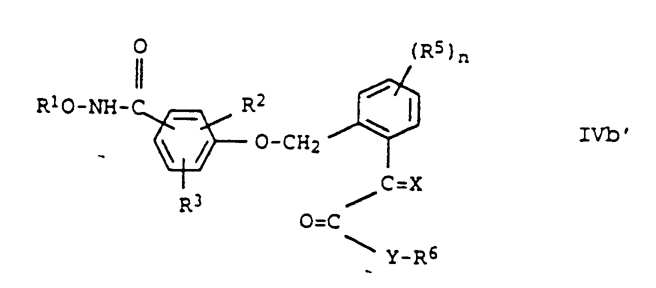 dans laquelle les symboles ont les significations suivantes :
X représente =CH-OCH₃, =CH-CH₃ ou =N-OCH₃ ;
Y représente l'oxygène, un groupe -NH- ou -N(CH₃)- ;
R¹ représente un groupe alkyle en C1-C6, alcényle en C2-C6, alcynyle en C3-C6, halogénoalkyle en C1-C6, halogénoalcényle en C3-C6, (alcoxy en C1-C4)alkyle en C1-C6, cyano-alkyle en C1-C6, (alcoxy en C1-C6)-carbonyl-alkyle en C1-C6, un groupe cycloalkyle en C3-C6 ou (cycloalkyle en C3-C6)alkyle en C1-C4, un groupe aryl-alkyle en C1-C6 ou aryl-alcényle en C3-C6 ou aryloxy-alkyle en C1-C6, un groupe hétérocyclyle ou hétérocyclyl-alkyle en C1-C4 saturé ou insaturé de quatre à six chaînons ou un groupe hétéroaryl-alkyle en C1-C6, les noyaux hétérocycliques pouvant contenir chacun, en plus des atomes de carbone, un ou deux chaînons cycliques choisis dans le groupe consistant en un atome d'oxygène ou de soufre et un ou deux atomes d'azote et un ou deux groupes -N(CH₃)-, les noyaux carbocycliques et hétérocycliques pouvant eux-mêmes porter un ou plusieurs substituants choisis parmi les halogènes, les groupes alkyle en C1-C4, halogénoalkyle en C1-C2, cycloalkyle en C3-C6, alcoxy en C1-C4, halogénoalcoxy en C1-C2, les halogènes, les groupes aryle et aryloxy ;
R², R³ représentent l'hydrogène, un halogène, un groupe cyano, nitro, alkyle en C1-C4, halogénoalkyle en C1-C2, alcoxy en C1-C4, halogénoalcoxy en C1-C2, ou bien, lorsque R² et R³ sont voisins, ils représentent ensemble un pont oxyméthylidène-oxy ou oxyéthylidène-oxy, chaque atome de carbone de ces ponts pouvant porter, si on le désire, un ou deux atomes d'halogènes et/ou groupes méthyle ;
R⁵ représente un groupe nitro, cyano, un halogène, un groupe alkyle en C1-C4, halogénoalkyle en C1-C4, alcoxy en C1-C4, halogénoalcoxy en C1-C4, alkylthio en C1-C4, phényle ou phénoxy, les noyaux aromatiques pouvant porter un à trois substituants choisis parmi les groupes alkyle en C1-C4, halogénoalkyle en C1-C4, alcoxy en C1-C4, halogénoalcoxy en C1-C4 et alkylthio en C1-C4, les noyaux aromatiques pouvant en outre porter des atomes d'halogènes en nombre tel que le nombre total des substituants soit de 4 ou 5 ;
ou bien, lorsque n est égal à 2, 3 ou 4, un groupe 1,3-butadièn-1,4-diyle ou un groupe 1,3-butadièn-1,4-diyle mono- ou di-halogéné, condensé sur deux atomes de carbone voisins du corps fondamental, ces noyaux condensés pouvant eux-mêmes porter un ou deux substituants choisis parmi les groupes nitro, cyano, halogénoalkyle en C1-C4, alcoxy en C1-C4, halogénoalcoxy en C1-C4 et alkylthio en C1-C4 ;
n est égal à 0, 1, 2, 3 ou 4, les symboles R⁵ pouvant avoir des significations identiques ou différentes lorsque n est égal à 1, 2, 3 ou 4 ;
R⁶ représente l'hydrogène ou un groupe alkyle en C1-C4.

7. Dérivés phénoxyméthylphényliques de formule I selon la revendication 1 dans laquelle
n est égal à 0 ou 1
X représente =CH-OCH₃, =CH-CH₃ ou =N-OCH₃ ;
Y représente l'oxygène ou -NH- ;
R¹ représente un groupe alkyle en C1-C6, alcényle en C2-C6, alcynyle en C3-C6, halogénoalkyle en C1-C6 ou halogénoalcényle en C3-C6 ;
R² représente un groupe cyano, le chlore, le fluor, un groupe méthyle, trifluorométhyle ou méthoxy ;
R³ représente l'hydrogène, un groupe cyano, le chlore, le fluor, un groupe méthyle ou méthoxy ;
R⁴ représente un groupe cyano, le chlore, le brome, un groupe alcoxy en C1-C6, alkylthio en C1-C6, alcényloxy en C3-C6, aryloxy ou arylthio et
R⁵ représente un groupe cyano, un halogène, un groupe alkyle en C1-C6, halogénoalkyle en C1-C6, alcoxy en C1-C6 ou phényle.

8. Dérivés phénoxyméthylphényliques de formule I selon la revendication 1, dans laquelle
n est égal à 0 ou 1 ;
X représente =NO-CH₃ ;
Y représente -HN- ;
R¹ représente un groupe alkyle en C1-C6, alcényle en C2-C6, alcynyle en C3-C6, halogénoalkyle en C1-C6 ou halogénoalcényle en C3-C6 ;
R² représente un groupe cyano, le chlore, le fluor, un groupe méthyle, trifluorométhyle ou méthoxy ;
R³ représente l'hydrogène, un groupe cyano, le chlore, le fluor, un groupe méthyle ou méthoxy ;
R⁴ représente un groupe cyano, le chlore, le brome, un groupe alcoxy en C1-C6, alkylthio en C1-C6, alcényloxy en C3-C6, phényloxy ou phénylthio ; et
R⁵ représente un groupe cyano, le chlore, le fluor, un groupe méthyle, trifluorométhyle, méthoxy ou phényle.

9. Utilisation des dérivés phénoxyméthylphényliques de formule I selon la revendication 1 en tant que fongicides.

10. Utilisation des dérivés phénoxyméthylphényliques de formule I selon la revendication 1 pour la lutte contre les parasites.

11. Produit fongicide contenant des véhicules solides et/ou liquides et une quantité fongicide efficace d'au moins un dérivé phénoxyméthylphénylique substitué de formule I selon la revendication 1.

12. Procédé pour combattre les mycètes, caractérisé par le fait que l'on traite les mycètes ou les matériaux, végétaux, semences ou sols menacés d'une attaque par les mycètes par une quantité fongicide efficace d'au moins un dérivé phénoxyméthylphénylique substitué de formule I selon la revendication 1.

13. Produit pour combattre les parasites, contenant des additifs inertes et une quantité parasiticide efficace d'au moins un dérivé phénoxyméthylphénylique substitué de formule I selon la revendication 1.

14. Procédé pour combattre les parasites, caractérisé par le fait que l'on traite les parasites et/ou leur habitat par une quantité parasiticide efficace d'au moins un dérivé phénoxyméthylphénylique substitué de formule I selon la revendication 1.
